(19) **Europäisches Patentamt**
**European Patent Office**
**Office européen des brevets**

(11) **EP 1 981 582 B1**

(12) **EUROPÄISCHE PATENTSCHRIFT**

(45) Veröffentlichungstag und Bekanntmachung des Hinweises auf die Patenterteilung:
**01.07.2009 Patentblatt 2009/27**

(21) Anmeldenummer: **07703207.6**

(22) Anmeldetag: **01.02.2007**

(51) Int Cl.:
*A61N 1/36* (2006.01)   *A61B 5/04* (2006.01)
*A61B 5/12* (2006.01)   *G10L 11/00* (2006.01)
*G10L 15/02* (2006.01)

(86) Internationale Anmeldenummer:
**PCT/EP2007/000878**

(87) Internationale Veröffentlichungsnummer:
**WO 2007/090563 (16.08.2007 Gazette 2007/33)**

(54) **VORRICHTUNG UND COMPUTERPROGRAMM ZUM ERZEUGEN EINES ANSTEUERSIGNALS FÜR EIN COCHLEA-IMPLANTAT BASIEREND AUF EINEM AUDIOSIGNAL**

DEVICE AND COMPUTER PROGRAM FOR GENERATING A CONTROL SIGNAL FOR A COCHLEA-IMPLANT BASED ON AN AUDIO SIGNAL

DISPOSITIF ET PROGRAMME INFORMATIQUE UTILISÉS POUR PRODUIRE UN SIGNAL DE COMMANDE POUR UN IMPLANT COCHLÉAIRE SUR LA BASE D'UN SIGNAL AUDIO

(84) Benannte Vertragsstaaten:
**AT FR GB**

(30) Priorität: **10.02.2006 DE 102006006296**

(43) Veröffentlichungstag der Anmeldung:
**22.10.2008 Patentblatt 2008/43**

(73) Patentinhaber: **Fraunhofer-Gesellschaft zur Förderung der angewandten Forschung e.V.
80686 München (DE)**

(72) Erfinder: **KLEFENZ, Frank
68159 Mannheim (DE)**

(74) Vertreter: **Burger, Markus et al
Schoppe, Zimmermann,
Stöckeler & Zinkler
Patentanwälte
Postfach 246
D-82043 Pullach bei München (DE)**

(56) Entgegenhaltungen:
WO-A-01/99470          US-A- 4 980 918
US-A- 5 381 512          US-A- 5 388 182
US-A1- 2005 069 162          US-A1- 2005 234 366

EP 1 981 582 B1

**Beschreibung**

Die vorliegende Erfindung bezieht sich im Allgemeinen auf eine Vorrichtung und ein Computerprogramm zum Erzeugen eines Ansteuersignals für ein Cochlea-Implantat basierend auf einem Audiosignal, im Speziellen auf ein Konzept zur selektiven Generierung von elektrischen Stimuli für Cochela-Implantate mittels zweistufiger Rückfilterung einer Neurotransmitter-Vesikel-Freisetzungs(release)-Verteilung.

Schon seit langer Zeit ist es ein Ziel der Medizin und insbesondere der Medizintechnik, Menschen mit einem Gehörschaden eine Teilnahme am normalen gesellschaftlichen Leben mit möglichst geringen Einschränkungen zu ermöglichen. Bereits seit vielen Jahren existieren herkömmliche Hörgeräte, die ein empfangenes akustisches Signal verstärken und somit auch schwerhörigen Patienten ermöglichen, akustische Signale geringer Lautstärke wahrzunehmen.

Seit einigen Jahren ist es sogar möglich, Patienten mit einer schweren und irreparablen Schädigung des Innenohrs zumindest zu einem eingeschränkten Hörempfinden zu verhelfen. Zu diesem Zweck werden mittels Elektroden eines Cochlea-Implantats intakte Hörnerven des Patienten angeregt. Die entsprechenden Anregungssignale werden dabei von einem Audiosignal abgeleitet, das an den entsprechenden Patienten wiedergegeben werden soll.

Ferner ist ein neurophysiologisch parametrisiertes auditorisches Computersimulationsmodell bekannt, das beispielsweise in der Veröffentlichung "Neuronale Repräsentation des Hörvorgangs als Basis" von G. Szepannek, F. Klefenz und C. Weihs (Informatik-Spektrum, Bd. 28, Nr. 5, Seiten 389 - 395, Oktober 2005, Springer-Verlag) beschrieben ist.

Eine hydromechanische Flüssigkeitssäulenanregung in der Cochlea und eine Wanderwellenbewegung auf der Basilarmembran werden durch einen Satz von gekoppelten elektromechanischen Differenzialgleichungen modelliert. Nähere Ausführungen diesbezüglich finden sich beispielsweise in der Doktorarbeit "Ein psychophysiologisches Gehörmodell zur Nachbildung von Wahrnehmungsschwellen für die Audiocodierung" von F. Baumgarte (Doktorarbeit an der Universität Hannover, 2000).

Die Bewegung bzw. Wanderwellenbewegung der Basilarmembran führt zu einer Koppelbewegung der Stereozilien der inneren Haarzellen. Eine Auslenkung der Stereozilien aus ihrer Ruhelage depolarisiert eine Ruhe-Membranspannung der inneren Haarzelle, wodurch eine Wahrscheinlichkeit eines Austritts eines Neurotransmitter-Vesikels aus der Haarzelle in den synaptischen Spalt erhöht wird. Austrittszeitpunkte der Neurotransmitter-Vesikel werden nach einem Modell von Meddis-Poveda modelliert. Eine Modellierung des Spannungsverlaufs in Nervenzellen geht auf eine Arbeit von Hodgkin und Huxley zurück. Eine Membranspannung wird dabei durch ein Austausch von Ionen sowie einen externen Strom durch die freigesetzten Neurotransmitter beeinflusst. Diffundiert ein Vesikel aus der präsynaptischen inneren Haarzelle (ICH) in einen synaptischen Spalt, so bindet es an einem Rezeptorprotein der postsynaptischen Membran und setzt Ladung frei. Durch die Moleküle eines Vesikels erhöht das postsynaptische Potential um etwa 0,5 bis 1 mV. überschreitet die Polarisation der postsynaptischen Nervenzelle einen bestimmten Schwellenwert v, so kommt es zur Freisetzung eines Aktionspotentials.

Aktionspotentiale kennzeichnen sich durch ihren stets nahezu identischen Verlauf. Die Membranspannung depolarisiert zunächst für eine sehr kurze Dauer von weniger als 1 ms extrem stark, danach hyperpolarisiert sie und ist für einen Zeitraum blockiert, in dem keine weiteren Aktionspotentiale auftreten können.

Die WO 01/99470 A1 beschreibt einen Audioprozessor für ein Cochlea-Implantat. Der Audioprozessor verwendet ein Modell einer Basilarmembranbewegung, um Stimuli basierend auf einer vorhergesagten Bewegung, die das Audiosignal in einer akustisch angeregten Normal-hörenden Cochlea erzeugen würde, auszuwählen. Die Filter überdecken mehrere Kanäle und überlappen sich. Daher erzeugen die Stimuli ein neurales Anregungsmuster, das eine räumlich-zeitliche Wanderwelle annähert, die auf einer Basilarmembran einer akustisch angeregten Normal-hörenden Cochlea beobachtet wird.

Die US 5 381 512 beschreibt ein Verfahren und eine Vorrichtung zur Sprachmerkmalserkennung basierend auf Modellen der auditorischen Signalverarbeitung. Eine Stimulus-Wellenform wird unter Verwendung eines Modells des menschlichen Gehörsystems verarbeitet, um eine Mehrzahl von Ausgangswellenformen bereitzustellen. Jede Ausgangswellenform entspricht einer Anregung an unterschiedlichen Orten entlang der Basilarmembran in der Cochlea, und entspricht der schmalen Frequenzbandbreite, der kurzen Zeitantwort und den Wellenausbreitungscharakteristika der menschlichen Cochlea. Eine primäre Merkmalsdetektion wird erreicht, indem Antwort-Wellenformen und deren räumliche und zeitliche Ableitungen mit vorbestimmten Vorlagen verglichen werden. Eine sekundäre Merkmalsdetektion wird erreicht, indem räumliche und zeitliche Muster von primären Merkmalen mit Mustern, die zu menschlichen Sprachelementen gehören, verglichen werden.

Die US 5 388 182 beschreibt ein nichtlineares Verfahren und eine Vorrichtung zur Codierung und Decodierung von akustischen Signalen mit einer Datenkompression und einer Rauschunterdrückung unter Verwendung von Cochlea-Filtern, einer Wavelet-Analyse und einer unregelmäßigen Abtastwert-Rekonstruktion. Das Verfahren umfasst das Erstellen einer Filterbank unter Verwendung von Wavelet-Transformationen von grundlegenden Filter-Impulsantworten, um die Antwort der Säugetier-Cochlea darzustellen. Eine Datenkompression wird erhalten, indem eine diskrete Darstellung erzeugt wird. Eine Rekonstruktion basiert auf einer Rahmen-Theorie. Ein Rekonstruktionsverfahren basiert auf einer unregelmäßigen Abtastung, die Ergebnisse mit guter Qualität in sehr wenigen Stufen erzeugt.

Die US 4 980 918 beschreibt ein Spracherkennungssystem mit einer effizienten Speicherung und einer schnellen Erzeugung von phonologischen Graphen. Ein Spracherkennungssystem umfasst einen Sprachprozessor und ein Wort-Erkennungs-Computer-Teilsystem. Das Wort-Erkennungs-Computer-Teilsystem umfasst eine Einrichtung zur Entwicklung eines Graphen für Verbindungen zwischen verschiedenen Knoten. Das Teilsystem umfasst ferner eine Einrichtung zur Bestimmung eines Graphen von Grenz-Verbindungen zwischen benachbarten Worten, sowie eine Einrichtung zur Speicherung eines Verzeichnisses von Verbindungen. Das Teilsystem umfasst ferner eine Einrichtung zur Konvertierung eines unbekannten Lauts in eine codierte Sequenz von Verbindungen.

Die US 20005/069162 A1 beschreibt eine zweiohrige adaptive Hörhilfe. Das Hörhilfesystem umfasst einen ersten und einen zweiten Kanal, wobei einer der Kanäle eine einstellbare Verzögerung aufweist. Der erste Kanal umfasst eine Richtungs-Einheit zum Empfangen des akustischen Signals und zum Bereitstellen eines gerichteten Signals, eine Korrelations-Einheit zum Empfangen des gerichteten Signals und zum Bereitstellen eines Rausch-verringerten Signals unter Verwendung von Korrelationsmaßen zum Identifizieren eines Sprachsignals in dem gerichteten Signal, und einen Kompensierer zum Empfangen des Rausch-verringerten Signals und zum Bereitstellen eines kompensierten Signals zur Kompensation eines Hör-Verlustes des Benutzers.

Es ist die Aufgabe der vorliegenden Erfindung, ein Konzept zum Erzeugen eines Ansteuersignals für ein Cochlea-Implantat basierend auf einem Audiosignal zu schaffen, das es ermöglicht, einem Patienten mit einem Cochlea-Implantat einen gegenüber herkömmlichen Konzepten verbesserten Höreindruck zu geben.

Diese Aufgabe wird durch ein Computerprogramm zur Durchführung eines Verfahrens zum Erzeugen eines Ansteuersignals für ein Cochlea-Implantat basierend auf einem Audiosignal gemäß Anspruch 1, und durch eine Vorrichtung zum Erzeugen eines Ansteuersignals für ein Cochlea-Implantat gemäß Anspruch 12 gelöst.

Die vorliegende Offenbarung schafft ein Verfahren zum Erzeugen eines Ansteuersignals für ein Cochlea-Implantat, basierend auf einem Audiosignal. Das Verfahren umfasst ein Berechnen einer ersten Information über ein Aktivitätsmuster über der Zeit an einer Mehrzahl von inneren Haarzellen eines Gehörmodells, das sich aufgrund des Audiosignals ergibt, sowie ein Herausfiltern von durch die erste Information beschriebenen Aktivitätsereignissen, basierend auf einer Erkennung eines charakteristischen Musters in dem Aktivitätsmuster. Durch das Herausfiltern wird eine bereinigte Information erhalten. Die bereinigte Information wird weiterhin als Ansteuersignal für das Cochlea-Implantat verwendet, oder es wird das Ansteuersignal von der bereinigten Information abgeleitet.

Die vorliegende Erfindung beruht auf der Erkenntnis, dass in einem Aktivitätsmuster mit der Zeit an einer Mehrzahl von inneren Haarzellen eines Gehörmodells eine Vielzahl von Aktivitätsimpulsen anliegen, die für einen Höreindruck bei einem Patienten nicht relevant sind. Somit besteht der Kerngedanke der vorliegenden Erfindung darin, ein charakteristisches Muster in dem Nervenaktivitätsmuster zu erkennen und basierend auf der Erkennung des charakteristischen Musters zu entscheiden, welche Aktivitätsereig-nisse herauszufiltern sind, weil sie beispielsweise für eine Wahrnehmung eines Patienten lediglich von untergeordneter Bedeutung sind. Es wurde ferner erkannt, dass es eine Mehrzahl von charakteristischen Mustern gibt, die einen Hinweis darauf geben, ob bestimmte Aktivitätsereignisse herauszufiltern sind oder nicht. Treten beispielsweise zwei Aktivitätsereignisse an der gleichen inneren Haarzelle zeitlich kurz nacheinander auf, so trägt nur das erste Auftreten zu einem Höreindruck bei. Ferner treten in dem Aktivitätsmuster charakteristische Trajektorien (linienförmige Muster) auf. Je nach Anwendungsfall kann es dabei beispielsweise vorteilhaft sein, entweder Aktivitätsereignisse, die zu einer Trajektorie gehören, oder Aktivitätsereignisse, die nicht zu einer Trajektorie gehören, herauszufiltern bzw. zu entfernen. Zusammenfassend lässt sich jedoch festhalten, dass gemäß dem Kerngedanken der vorliegenden Erfindung herauszufilternde Aktivitätsereignisse jeweils durch Identifizieren charakteristischer Muster erkannt werden können.

Durch das Herausfiltern entsteht eine bereinigte Information. Es hat sich nämlich gezeigt, dass ein verbesserter Höreindruck bei einem Patienten erzielt werden kann, wenn eine bereinigte Information, in der überflüssige Aktivitätsereignisse entfernt sind, als Ansteuersignal für das Cochlea-Implantant oder aus Ausgangsbasis für das Ableiten des Ansteuersignals des Cochlea-Implantats herangezogen werden.

Ferner wurde im Rahmen der vorliegenden Erfindung erkannt, dass sich gerade das Aktivitätsmuster über der Zeit an einer Mehrzahl von inneren Haarzellen eines Gehörmodells besonders gut für eine Filterung eignet, um basierend auf einer Erkennung eines charakteristischen Musters störende Informationen, die beispielsweise für eine Sprachverständigkeit nicht relevant sind, herauszufiltern.

Es wurde nämlich herausgefunden, dass gerade in dem Aktivitätsmuster an einer Mehrzahl von inneren Haarzellen Sprachsignale in charakteristischen Signalverläufen bzw. Mustern resultieren, die eine Unterscheidung zwischen Sprachsignalen und störenden Neben-Geräuschen ermöglicht. Bei einer Festlegung, welche Aktivitätsereignisse aus dem Aktivitätsmuster über der Zeit an der Mehrzahl von inneren Haarzellen herauszufiltern sind, können beispielsweise neurophysiologische Erkenntnisse herangezogen werden, die eine Aussage darüber geben, welche Aktivitätsmuster in einem neuronalen Netz, das beispielsweise dem menschlichen Gehirn nachempfunden ist, eine besonders starke Antwort ergeben.

Aus der ersten Information werden dann beispielsweise Aktivitätsereignisse herausgefiltert, von denen aufgrund eines in dem Aktivitätsmuster auftretenden charakteristischen Musters erkannt wird, dass sie in dem menschlichen Hirn nur zu einer schwachen Reaktion führen würden. Es wurde nämlich erkannt, dass das menschliche Gehirn auf bestimmte charakteristische Muster besonders stark anspricht, während es bei Auftreten anderer charakteristischer Muster in dem Aktivitätsmuster nur eine vernachlässigbare Reaktion zeigt. Eine Bestimmung, auf welche charakteristischen Muster das menschliche Gehirn sensibel reagiert, kann beispielsweise durch eine Beobachtung von Gehirnströmen bzw. eine Beobachtung einer Gehirnaktivität bestimmt werden.

Im übrigen wird darauf hingewiesen, dass sich beispielsweise gezeigt hat, dass Aktivitätsereignisse in dem Aktivitätsmuster, die im Anschluss an die Freisetzung eines Aktionspotentials an einer Synapse bzw. einer Nervenfaser innerhalb einer Refraktärzeit (Totzeit) der Synapse bzw. der Nervenfaser auftreten, für eine Sprachverständlichkeit nicht relevant sind. Ferner sind für eine Sprachverständigkeit solche Aktivitätsereignisse nicht relevant, die nicht zu einer Trajektorie in dem Aktivitätsmuster über der Zeit an der Mehrzahl von inneren Haarzellen gehören.

Die vorliegende Erfindung ermöglicht somit, aus dem Aktivitätsmuster über der Zeit an der Mehrzahl von inneren Haarzellen die Information zu eliminieren, die für einen Patienten mit einem Cochlea-Implantat nicht von wesentlicher Bedeutung ist bzw. für einen solchen Patienten sogar eine störende Wirkung hat. Somit kann der Höreindruck des entsprechenden Patienten verbessert werden.

Ferner bringt die vorliegende Erfindung den Vorteil mit sich, dass in dem Ansteuersignal für das Cochlea-Implantat weniger Aktivitätsereignisse auftreten als bei der Verwendung von herkömmlichen Konzepten. Dies erleichtert die Bereitstellung der durch das Cochlea-Implantant basierend auf dem Ansteuersignal erzeugten Nerven-Stimulationssignale. Typische Cochlea-Implantate sind nämlich nur in der Lage, eine bestimmte Anzahl von Nerven-Stimulationsimpulsen pro Zeiteinheit abzugeben, wobei sich der Schaltungsaufwand erhöht, je mehr Nerven-Stimulationsimpulse pro Zeiteinheit abgegeben werden müssen. Im übrigen erhöht sich auch ein Energieverbrauch nahezu linear mit einer Anzahl an abgegeben Nerven-Stimulationsimpulsen. Durch das erfindungsgemäße Konzept, "unnötige" bzw. für eine Sprachverständlichkeit nicht relevante Aktivitätsimpulse aus dem Aktivitätsmuster herauszufiltern, verringert sich somit die Komplexität des Cochlea-Implantats ebenso wie der Stromverbrauch desselben.

Bei einem weiteren bevorzugten Ausführungsbeispiel beschreibt die erste Information über das Aktivitätsmuster über der Zeit zeitliche Verläufe einer Anzahl an freigesetzten Neurotransmitter-Vesikeln für eine Mehrzahl von inneren Haarzellen. Das Herausfiltern umfasst bei diesem Ausführungsbeispiel ein Entfernen von Neurotransmitter-Vesikel-Auftreten, die nicht zu einer Erzeugung eines Aktionspotentials beitragen oder ein Aktionspotential generieren, aus der ersten Information. Es hat sich nämlich gezeigt, dass Neurotransmitter-Vesikel-Auftreten nur dann für das Cochlea-Implantat bzw. einen menschlichen Patienten von Bedeutung sind, wenn die entsprechenden Neurotransmitter-Vesikel-Auftreten tatsächlich in einem Aktionspotential resultieren. Eine entsprechende bereinigte Information, die nur noch Neurotransmitter-Vesikel-Auftreten beschreibt, die auch in einem Aktionspotential resultieren, weist ein deutlich niedrigeres Datenaufkommen auf als die zugehörige unbereinigte Information, die alle Neurotransmitter-Vesikel-Auftreten beschreibt. Das Erzeugen der bereinigten Informationen über ein Neurotransmitter-Vesikel-Auftreten stellt daher eine Möglichkeit dar, um das Datenaufkommen in einem System zum Betrieb eines Cochlea-Implantats zu verringern. Die genannte bereinigte Information kann beispielsweise wesentlich Ressourcen-effizienter ausgetauscht werden als eine ungereinigte Information über das Neurotransmitter-Vesikel-Auftreten. Dies ist von besonderer Bedeutung, wenn die bereinigte Information über das Neurotransmitter-Vesikel-Auftreten zwischen mehreren Komponenten eines Systems zum Betrieb eines Cochlea-Implantats ausgetauscht wird. In vielen Fällen beispielsweise wird die (herkömmlicherweise unbereinigte) Information über einen Neurotransmitter-Vesikel-Auftreten zwischen einer externen Verarbeitung und dem Cochlea-Implantat drahtlos oder drahtgebunden übertragen, wobei die Kapazität der entsprechenden Übertragungsstrecke an das Datenaufkommen angepasst sein muss. Eine Verringerung des Datenaufkommens aufgrund der erfindungsgemäßen Bereinigung der Information (Herausfiltern) bewirkt dabei direkt, dass weniger Ressourcen (z. B. Frequenzressourcen oder Energie) benötigt werden, um die entsprechende Übertragung zu ermöglichen.

Ferner muss bei Verwendung der vorliegenden Erfindung jede weitere Verarbeitungseinheit nur noch eine geringere Datenmenge (nur die bereinigte Information über das Neurotransmitter-Vesikel-Auftreten) verarbeiten als herkömmlich üblich (unbereinigte Information über das Neurotransmitter-Vesikel-Auftreten). Durch die erfindungsgemäße Reduktion des Datenaufkommens aufgrund des Herausfilterns bzw. Entfernens von Neurotransmitter-Vesikel-Auftreten, die nicht zu einer Erzeugung eines Aktionspotentials beitragen oder ein Aktionspotential generieren, geht im Übrigen keine für einen Patienten wesentliche Information verloren. Somit kann auf quasiverlustfreiem Wege das Datenaufkommen und der für die Verarbeitung notwendige Ressourcenbedarf deutlich verringert werden.

Das Aktivitätsmuster umfasst im Übrigen bevorzugt zeitliche Verläufe einer Anzahl an pro Zeiteinheit freigesetzten Neurotransmitter-Vesikeln oder einer Anzahl an während eines bestimmten Zeitintervalls oder zu einem bestimmten Zeitpunkt in dem synaptischen Spalt vorhandenen Neurotransmitter-Vesikeln. Das Aktivitätsmuster kann aber auch lediglich eine Information darüber umfassen, ob während eines bestimmten Zeitintervalls Neurotransmitter-Vesikel in den synaptischen Spalt freigesetzt werden, oder ob während eines bestimmten Zeitintervalls oder zu einem bestimmten Zeitpunkt Neurotransmitter-Vesikel in dem synaptischen Spalt vorhanden sind.

Die Tatsache, ob ein Neurotransmitter-Vesikel-Auftreten zu einer Erzeugung eines Aktionspotentials beiträgt oder nicht, oder ob ein Neurotransmitter-Vesikel-Auftreten ein Aktionspotential generiert oder nicht, kann beispielsweise mit Hilfe eines Modells für eine Erzeugung von Aktionspotentialen bestimmt werden. Das entsprechende Modell kann beispielsweise eine Abschätzung darüber liefern, ob ein Neurotransmitter-Vesikel-Auftreten zu einer Erzeugung eines Aktionspotentials beiträgt oder ein Aktionspotential generiert. Sind gemäß dem entsprechenden Modell beispielsweise mehrere Neurotransmitter-Vesikel notwendig, um ein Aktionspotential zu generieren, so ist es beispielsweise möglich, all diejenigen Neurotransmitter-Vesikel-Auftreten zu entfernen, die nur vorbereitend für eine Auslösung eines Aktionspotentials erforderlich sind, so dass die bereinigte Information nur noch eine Information über das Auftreten des letzten Neurotransmitter-Vesikels, das schließlich das Aktionspotential auslöst, enthält.

Bei einem weiteren Ausführungsbeispiel wird es bevorzugt, bei dem Herausfiltern Neurotransmitter-Vesikel-Auftreten für Haarzellen zu entfernen, deren Synapse sich aufgrund einer zeitlich zurückliegenden Freisetzung eines Aktionspotentials in einem Refraktär-Zeitraum bzw. in einer Totzeit befindet. Speziell innerhalb einer absoluten Totzeit bzw. absoluten Refraktärzeit kann nämlich davon ausgegangen werden, dass ein Neurotransmitter-Vesikel-Auftreten in keinem Fall zu einer Freisetzung eines Aktionspotentials beiträgt. Somit stellt die Berücksichtigung einer Refraktärzeit von Synapsen ein besonders leicht auswertbares Kriterium dar, anhand dessen nicht-relevante Neurotransmitter-Vesikel-Auftreten aus der ersten Information über das Aktivitätsmuster entfernt werden können.

Das Auftreten von Aktivitätsereignissen innerhalb der Refraktärzeit ist im übrigen im Rahmen einer Mustererkennung durch das Vorhandensein zumindest zweier zeitlich kurz beabstandeter Aktivitätsereignisse an der gleichen inneren Haarzelle erkennbar.

Bei einem weiteren bevorzugten Ausführungsbeispiel der vorliegenden Erfindung umfasst das Herausfiltern der Aktivitätsereignisse basierend auf einem charakteristischen Muster in dem Aktivitätsmuster ein Bestimmen, welche der durch die erste Information beschriebenen Aktivitätsereignisse zu einer Trajektorie gehören bzw. nicht zu einer Trajektorie gehören. Das Herausfiltern umfasst in diesem Fall ein Ableiten der bereinigten Information über das Aktivitätsmuster, wobei die bereinigte Information aus der ersten Information durch Eliminieren von Aktivitätsereignissen, die nicht zu einer Trajektorie gehören, gebildet wird.

Das entsprechende Ausführungsbeispiel basiert auf der wesentlichen Erkenntnis, dass Aktivitätsereignisse dann besonders wichtig für eine Sprachverständigkeit sind, wenn die Aktivitätsereignisse auf verschiedenen Hörnerven in einem zeitlichen Zusammenhang stehen, der durch eine Trajektorie beschrieben werden kann. Es hat sich nämlich gezeigt, dass insbesondere bei Sprachsignalen Wanderwellen auf der Basilarmembran auftreten, die verschiedene Haarzellen zeitlich nacheinander und in einer durch eine Ausbreitungsgeschwindigkeit auf der Basilarmembran bestimmten charakteristischen zeitlichen Abfolge anregen. Es hat sich ferner gezeigt, dass das menschliche Gehirn durch seinen Aufbau als ein neuronales Netz besonders stark auf eine entsprechende Trajektorie bzw. vorbestimmte charakteristische Sequenz von Aktivitätsereignissen anspricht. Daher sind die Aktivitätsereignisse, die zu einer Trajektorie gehören, besonders relevant für eine Sprachverständlichkeit. Die Aktivitätsereignisse hingegen, die nicht auf einer Trajektorie liegen, sind für eine Sprachverständigkeit weniger relevant bzw. sogar störend. Daher wird durch das erfindungsgemäße Eliminieren von Aktivitätsereignissen, die nicht zu einer Trajektorie gehören, nicht nur die Datenmenge, die zur Beschreibung der Aktivitätsereignisse nötig ist, verringert, sondern es wird gleichzeitig eine Sprachverständlichkeit der in dem Audiosignal enthaltenen Sprachinformationen für einen Patienten mit dem Cochlea-Implantat verbessert.

Bei einem alternativen Ausführungsbeispiel ist es allerdings auch möglich, Aktivitätsereignisse aus der ersten Information herauszufiltern bzw. zu eliminieren, die auf einer Trajektorie liegen. So existieren nämlich Störgeräusche, die eine charakteristische Trajektorie aufweisen. Beispielsweise resultierten Knack-Impulse, die häufig durch Übertragungsstörungen auftreten, in einer Wanderwelle auf der Basilarmembran, die als charakteristische Trajektorie in dem Aktivitätsmuster erkennbar ist. Somit ermöglicht das herausfiltern von Aktivitätsereignissen, die auf einer Trajektorie liegen, eine Verminderung bzw. Eliminierung von Störungen und resultiert somit in einem verbesserten Höreindruck.

Bei einem bevorzugten Ausführungsbeispiel beschreibt die erste Information zeitliche Verläufe einer Anzahl von freigesetzten Neurotransmitter-Vesikeln für eine Mehrzahl von inneren Haarzellen, wobei jeder Haarzelle aus der Mehrzahl von inneren Haarzellen ein zeitlicher Verlauf zugeordnet ist. Die Anzahl an freigesetzten Neurotransmitter-Vesikeln kann beispielsweise eine Anzahl an neu freigesetzten Neurotransmitter-Vesikeln für einzelne Zeitabschnitte umfassen. Die zeitlichen Verläufe können aber auch eine Gesamtzahl an Neurotransmitter-Vesikeln, die an dem synaptischen Spalt vorhanden sind, beschreiben. Es hat sich nämlich gezeigt, dass in der Anzahl an freigesetzten Neurotransmitter-Vesikeln Trajektorien erkennbar sind, die beispielsweise in dem Fall, dass ein Sprachsignal in dem Audiosignal enthalten ist, eine charakteristische Form aufweisen. Mit anderen Worten, in dem zeitlichen Verlauf der Neurotransmitter-Vesikel-Freisetzung über einer Mehrzahl von Nervenzellen sind charakteristische linienförmige Muster (Trajektorien) erkennbar, wobei zu den Trajektorien gehörige Aktivitätsereignisse einen besonders großen Beitrag zu einer Sprachverständlichkeit des durch das Neurotransmitter-Vesikel-Auftreten beschriebenen Sprachsignals liefern.

Bei einem weiteren bevorzugten Ausführungsbeispiel beschreibt die erste Information eine Mehrzahl von zeitlichen Spannungsverläufen auf einer Mehrzahl von Nervenfasern, die mit verschiedenen inneren Haarzellen gekoppelt sind. Dabei ist jeder Haarzelle ein zeitlicher Spannungsverlauf auf einer zugehörigen Nervenfaser zugeordnet. Es wurde nämlich erkannt, dass ähnlich wie bei dem Neurotransmitter-Vesikel-Auftreten auch in den Spannungsverläufen auf einer Mehrzahl von Nervenfasern, die mit verschiedenen Haarzellen gekoppelt sind (kurz als Nervenaktivitätsmuster bezeichnet) linienförmige charakteristische Nervenfaser-übergreifende Strukturen (Trajektorien) erkennbar sind, die eine Information über ein in dem Audiosignal enthaltenes Sprachsignal umfassen. Es wurde ferner erkannt, dass durch ein Entfernen von Aktionspotentialen bzw. Aktivitätsereignissen, die nicht zu einer der Trajektorien gehören, aus den zeitlichen Spannungsverläufen auf der Mehrzahl von Nervenfasern eine Sprachverständlichkeit verbessert werden kann. Ferner können Störungen vermindert werden, wenn Aktivitätsereignisse entfernt werden, die zu bestimmten (Stör-) Trajektorien gehören.

Bei einem weiteren bevorzugten Ausführungsbeispiel umfasst das Bestimmen, welche der durch die erste Information beschriebenen Aktivitätsereignisse nicht zu einer Trajektorie gehören, ein Durchführen einer Mustererkennung. Das Durchführen der Mustererkennung umfasst ein Empfangen der Information über das Aktivitätsmuster über der Zeit sowie ein Interpretieren des Aktivitätsmusters über der Zeit für die Mehrzahl von Haarzellen als eine zweidimensionale Darstellung. Eine erste Richtung der zweidimensionalen Darstellung beschreibt die Zeit, während hingegen eine zweite Richtung der zweidimensionalen Darstellung einen Index der inneren Haarzellen beschreibt. Eine in der zweidimensionalen Darstellung enthaltene Information beschreibt das Auftreten von Aktivitätsimpulsen in Abhängigkeit von der Zeit und von dem Index der inneren Haarzellen. Das Durchführen der Mustererkennung umfasst ferner eine Identifizierung eines Kurvenverlaufs in der zweidimensionalen Darstellung als eine Trajektorie, falls der Kurvenverlauf hinsichtlich eines vorgegebenen Ähnlichkeitsmaßes mindestens eine vorgegebene Ähnlichkeit mit einem Vergleichs-Kurvenverlauf aufweist.

Es wurde nämlich erkannt, dass Trajektorien in einer vorteilhaften Weise durch eine Durchführung einer Mustererkennung basierend auf der ersten Information identifiziert werden können. Wird die erste Information in der beschriebenen Weise zweidimensional dargestellt, so treten in der zweidimensionalen Darstellung nämlich linienförmige Verläufe (Trajektorien) auf, wobei eine Trajektorie somit ein linienförmiger Verlauf ist, der Aktivitätsereignisse in verschiedenen inneren Haarzellen verbindet, wenn die Aktivitätsereignisse zweidimensional über der Zeit und über der einem Index der inneren Haarzellen dargestellt sind.

In anderen Worten, eine Mustererkennung ist vorteilhaft einsetzbar, um in einer zweidimensionalen Darstellung der Aktivitätsereignisse über der Zeit und über einem Index der inneren Haarzellen diejenige Information zu erkennen, die beispielsweise für eine Sprachverständlichkeit relevant bzw. nicht-relevant ist.

Bei einem weiteren bevorzugten Ausführungsbeispiel umfasst das Bestimmen, welche Aktivitätsimpulse nicht zu einer Trajektorie gehören, ein Bestimmen von Aktivitätsimpulsen, die zu einer Trajektorie gehören. Eine Trajektorie wird bei einem Ausführungsbeispiel nur dann erkannt, wenn eine Anzahl von Aktivitätsimpulsen, die zu einer Trajektorie gehören, größer als eine vorgegebene Mindestanzahl ist. In anderen Worten, nur Trajektorien mit einer vorgegebenen minimalen Länge werden erkannt. Kürzere Trajektorien hingegen werden nicht berücksichtigt. Entsprechende Verfahren bringen eine weitere Reduzierung einer Datenmenge mit sich, da nur lange Trajektorien (länger als die vorgegebene Mindestanzahl) erkannt werden. Kurze Trajektorien hingegen werden nicht als Trajektorien identifiziert, wodurch beispielsweise Aktivitätsereignisse, die zu einer kurzen Trajektorie gehören, aus der ersten Information entfernt werden. Damit wird nicht nur die Informationsmenge reduziert, sondern wiederum die Sprachverständigkeit deutlich erhöht. Ferner können umgekehrt gerade Aktivitätsereignisse entfernt werden, die zu einer besonders langen Trajektorie (länger als eine vorgegebene Mindestlänge) gehören, das besonders lange Trajektorien beispielsweise eine breitbandige Störung (z.B. ein Knakken) beschreiben können.

Bei einem bevorzugten Ausführungsbeispiel umfasst das Identifizieren eines Kurvenverlaufs in der zweidimensionalen Darstellung ein Vergleichen der zweidimensionalen Darstellung oder eines Ausschnitts aus der zweidimensionalen Darstellung mit einem Vergleichs-Kurvenverlauf. Es wurde nämlich erkannt, dass Trajektorien durch eine begrenzte Anzahl an Vergleichs-Kurvenverläufen beschrieben werden können. Ein Vergleichen mit einem Vergleichskurvenverlauf kann im Übrigen in Ressourceneffizienter Weise realisiert werden. Bevorzugt werden mehrere Vergleichs-Kurvenverläufe verwendet, die mehrere mögliche Trajektorien beschreiben. Eine Verwendung von Vergleichs-Kurvenverläufen ist besonders einfach möglich, da Trajektorien typischerweise gerade oder gekrümmte Linien darstellen, wobei die zu erkennenden Trajektorien typischerweise die Krümmungsrichtung nicht wechseln. Mit anderen Worten, Trajektorien sind typischerweise über einen gesamten Verlauf entweder rechtsgekrümmt oder linksgekrümmt oder gerade. Linienförmige Verläufe mit einer einzigen Krümmungsrichtung können allerdings durch eine sehr begrenzte Anzahl an Vergleichsmustern zumindest näherungsweise beschrieben werden. Daher ist ein Mustervergleich eine effiziente Vorgehensweise zum Identifizieren einer Trajektorie in der zweidimensionalen Darstellung.

Bevorzugt werden als Vergleichsmuster gerade oder hyperbelförmig gekrümmte Kurven verwendet. Es wurde nämlich erkannt, dass aufgrund der Beschaffenheit der Cochlea Trajektorien typischerweise eine entsprechende Form aufweisen. Verantwortlich für die Krümmung der Trajektorien ist im Wesentlichen die Ausbreitungsgeschwindigkeit von Wanderwellen auf der Basilarmembran der Cochlea.

Bei einem weiteren bevorzugten Ausführungsbeispiel umfasst das Identifizieren eines Kurvenverlaufs in der zweidimensionalen Darstellung als eine Trajektorie ein schrittweises Verzerren der zweidimensionalen Darstellung, um eine verzerrte zweidimensionale Darstellung des Aktivitätsmusters über der Zeit zu erhalten. Das Identifizieren eines Kurvenverlaufs als eine Trajektorie umfasst ferner ein Bestimmen, ob in der verzerrten zweidimensionalen Darstellung eine näherungsweise gerade Linie enthalten ist, wobei eine näherungsweise gerade Linie in der verzerrten zweidimensionalen Darstellung als eine Trajektorie identifiziert wird. Das entsprechende Konzept basiert auf der Erkenntnis, dass Trajektorien durch eine geeignete Verzerrung der zweidimensionalen Darstellung zu einer geraden bzw. einer näherungsweise geraden Linie verbogen werden können. Eine Erkennung einer näherungsweise geraden Linie ist allerdings in besonders einfacher Weise möglich, da eine gerade bzw. näherungsweise gerade Linie durch das gleichzeitige Auftreten einer Mehrzahl von Aktivitätsereignissen in einer einzigen Zeile der verzerrten zweidimensionalen Darstellung erkennbar ist. Eine Erkennung des gleichzeitigen Auftretens einer Mehrzahl von Aktivitätsereignissen in einer Zeile der zweidimensionalen Darstellung kann aber leicht durch eine Summation der in der entsprechenden Zeile auftretenden Aktivitätsereignisse und durch Vergleichen des Summenwerts mit einem Schwellenwert erkannt werden.

Somit stellt ein schrittweises Verzerren ein besonders effizientes Verfahren einer Mustererkennung für eine gebogene bzw. gekrümmte Linie dar, wobei die eigentliche Mustererkennung lediglich ausgelegt sein muss, um ein Auftreten einer geraden bzw. näherungsweise geraden Linie zu detektieren.

Bei einem weiteren bevorzugten Ausführungsbeispiel umfasst das Ableiten der bereinigten Information ein Erkennen einer Trajektorie in der ersten Information, ein Markieren von Aktivitätsereignissen, die zu der Trajektorie gehören, sowie ein Eliminieren von nicht-markierten Aktivitätsereignissen. Somit werden aus der ersten Information alle diejenigen Aktivitätsereignisse entfernt, die nicht als zu einer Trajektorie gehörig markiert sind. Dabei kann zunächst eine Erkennung von Trajektorien über einen Ausschnitt der ersten Information durchgeführt werden, woraufhin nicht-markierte Ereignisse gelöscht werden. Die entsprechende Vorgehensweise ist ressourceneffizienter implementierbar als eine Erzeugung einer neun bereinigten Information, was beispielsweise durch Übernehmen der zu den Trajektorien gehörigen Aktivitätsereignissen erfolgen könnte. Werden nämlich aus der ersten Information die nicht zu einer Trajektorie gehörigen Aktivitätsereignisse entfernt, so ist es nicht erforderlich, eine Kopie der ersten Information zu erstellen.

Bei einem weiteren bevorzugten Ausführungsbeispiel umfasst das Berechnen der ersten Information über das Aktivitätsmuster über der Zeit ein Berechnen des Aktivitätsmusters über der Zeit an einer Mehrzahl von inneren Haarzellen eines Gehörmodells, wobei die inneren Haarzellen an verschiedenen Orten entlang der Cochlea angeordnet sind. Damit weisen die verschiedenen Haarzellen Empfindlichkeitsmaxima bei verschiedenen Frequenzen auf. Es ergibt sich somit eine Frequenzselektivität der Aktivitätsereignisse an den verschiedenen Haarzellen. Somit ist das Aktivitätsmuster über der Zeit besonders charakteristisch für einen Bereich von Frequenzen, die beispielsweise für eine Sprachverständigkeit relevant sind. Ereignisse in dem Audiosignal mit einem breitbandigen Frequenzspektrum, beispielsweise Ansätze von Vokalen oder Konsonanten, spiegeln sich somit in besonders charakteristischer Weise in den Trajektorien wider.

In anderen Worten, die verschiedenen inneren Haarzellen sind bevorzugt so gewählt, dass sie eine maximale Empfindlichkeit bei verschiedenen in dem Audiosignal enthaltenen Frequenzen aufweisen.

Die vorliegende Erfindung umfasst ferner ein Computerprogramm zur Durchführung des beschriebenen Verfahrens.

Ferner umfasst die vorliegende Erfindung eine Vorrichtung zum Erzeugen eines Ansteuersignals für ein Cochlea-Implantat basierend auf einem Audiosignal. Die erfindungsgemäße Vorrichtung umfasst Einrichtungen, die ausgelegt sind, um die im Hinblick auf das Verfahren beschriebenen Schritte durchzuführen.

Bevorzugte Ausführungsbeispiele der vorliegenden Erfindung werden nachfolgend bezugnehmend auf die beiliegenden Zeichnungen näher erläutert. Es zeigen:

Fig. 1    ein Flussdiagramm eines Verfahrens zum Erzeugen eines Ansteuersignals für ein Cochlea-Implantat basierend auf einem Audiosignal, gemäß einem ersten Ausführungsbeispiel der vorliegenden Offenbarung;

Fig. 2    eine schematische Darstellung eines Ablaufs bei einer Simulation eines menschlichen Gehörs sowie der bei der Simulation auftretenden Zwischen- und Endergebnisse;

Fig. 3 ein Flussdiagramm eines Verfahrens zum Bestimmen eines ersten bereinigten Neurotransmitter-Vesikel-Auftretens aus einer Information über ein Neurotransmitter-Vesikel-Auftreten;

Fig. 4 eine schematische Darstellung der Abläufe bei einem Ableiten eines ersten bereinigten Neurotransmitter-Vesikel-Auftretens aus einer Information über ein Neurotransmitter-Vesikel-Auftreten;

Fig. 5 ein Flussdiagramm eines Verfahrens zum Ableiten eines zweiten bereinigten Neurotransmitter-Vesikel-Auftretens aus einem ersten bereinigten Neurotransmitter-Vesikel-Auftreten;

Fig. 6 eine graphische Darstellung einer Geometrie der Basilarmembran und einer Reaktion der Basilarmembran auf eine Anregung;

Fig. 7 eine graphische Darstellung einer Verzögerung bei einer Ausbreitung von Audiosignalen verschiedener Frequenz aus einer Basilarmembran;

Fig. 8 eine graphische Darstellung eines Cochleograms für einen Vokal "i";

Fig. 9 eine graphische Darstellung eines Cochleograms, einer Transmitter-Freisetzungs-Wahrscheinlichkeit und einer Transmitter-Vesikel-Freisetzung für einen Vokal "A";

Fig. 10a eine graphische Darstellung eines Aktivitätsmusters, das ein Neurotransmitter-Vesikel-Auftreten an einer Mehrzahl von inneren Haarzellen über der Zeit beschreibt;

Fig. 10b eine graphische Darstellung eines bereinigten Aktivitätsmusters, das ein bereinigtes Neurotransmitter-Vesikel-Auftreten an einer Mehrzahl von inneren Haarzellen über der Zeit beschreibt;

Fig. 11 ein Flussdiagramm eines Verfahrens zum Ableiten eines bereinigten Nervenaktivitätsmusters aus einem Audiosignal;

Fig. 12 eine schematische Darstellung einer Vorgehensweise bei einer Erzeugung einer zweidimensionalen Darstellung aus einem Aktivitätsmuster über der Zeit an einer Mehrzahl von inneren Haarzellen eines Gehörmodells;

Fig. 13 eine zweite zweidimensionale graphische Darstellung eines Aktivitätsmusters über der Zeit an einer Mehrzahl von inneren Haarzellen eines Gehörmodells;

Fig. 14 eine dritte zweidimensionale graphische Darstellung eines Aktivitätsmusters über der Zeit an einer Mehrzahl von inneren Haarzellen eines Gehörmodells;

Fig. 15 ein Blockschaltbild einer Vorrichtung zur erfindungsgemäßen Durchführung einer Mustererkennung basierend auf dem Aktivitätsmuster;

Fig. 16 eine graphische Darstellung von Signalen in einer Vorrichtung zur erfindungsgemäßen Durchführung einer Mustererkennung;

Fig. 17 ein Schaltbild eines Hubel-Wiesel-Netzes zur erfindungsgemäßen Durchführung einer Mustererkennung;

Fig. 18 ein Schaltbild eines Hubel-Wiesel-Netzes zur erfindungsgemäßen Durchführung einer Mustererkennung, dargestellt als ein neuronales Netz;

Fig. 19 eine graphische Darstellung von Trainingsmustern zum Trainieren eines Hubel-Wiesel-Netzes; und

Fig. 20 eine schematische Darstellung eines Cochlea-Implantats zur Verwendung in Verbindung mit der vorliegenden Erfindung.

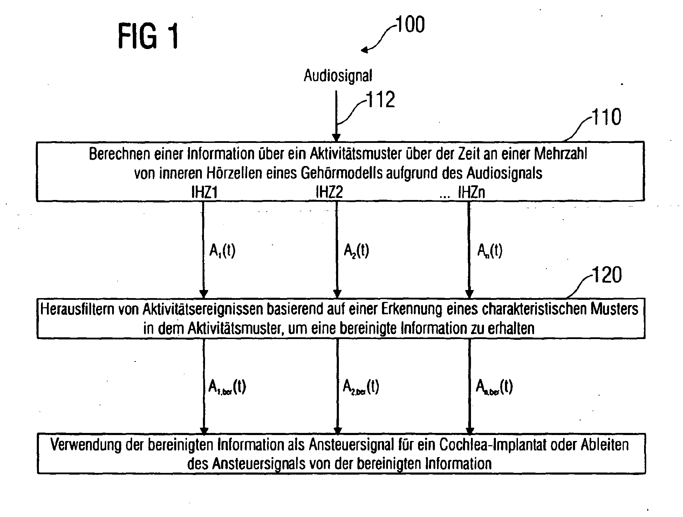

Fig. 1 zeigt ein Flussdiagramm eines Verfahrens zum Erzeugen eines Ansteuersignals für ein Cochlea-Implantat gemäß einem ersten Ausführungsbeispiel der vorliegenden Erfindung. Das Flussdiagramm der Fig. 1 ist in seiner Gesamtheit mit 100 bezeichnet.

Das erfindungsgemäße Verfahren umfasst in einem ersten Schritt 110 ein Berechnen einer Information über ein Aktivitätsmuster über der Zeit an einer Mehrzahl von inneren Haarzellen eines Gehörmodells aufgrund des Audiosignals 112. Damit ist ein Aktivitätsmuster an einer Mehrzahl von inneren Haarzellen bekannt, wobei die inneren Haarzellen mit IHZ1, IHZ2 bis IHZn bezeichnet sind. In anderen Worten, für die Mehrzahl von inneren Haarzellen IHZ1 bis IHZn sind jeweils zugehörige zeitliche Verläufe bekannt. Ein zu einer bestimmten inneren Haarzelle IHZi gehöriger zeitlicher Verlauf umfasst dabei typischerweise ein oder mehrere Aktivitätsereignisse, wobei die Aktivitätsereignisse eine Aktivität an der zugehörigen inneren Haarzelle IHZi beschreiben.

Ein Aktivitätsereignis kann beispielsweise ein Auftreten einer Anregung der entsprechenden inneren Haarzelle IHZi beschreiben, beispielsweise eine Auslenkung eines zu der inneren Haarzelle IHZi gehörigen Stereoziliums, eine Veränderung bzw. ein Maximum oder Minimum einer Calciumkonzentration in der inneren Haarzelle IHZi, eine Veränderung, ein Minimum oder ein Maximum in einer Transmitter-Freisetzungs-Rate in der inneren Haarzelle IHZi, ein Neurotransmitter-Vesikel-Auftreten in der inneren Haarzelle oder ein durch die innere Haarzelle IHZi erzeugtes Aktionspotential.

In anderen Worten, ein zu einer inneren Haarzelle IHZi gehöriger zeitlicher Verlauf zeigt ein Aktivitätsereignis beispielsweise dadurch an, dass in dem zeitlichen Verlauf ein lokales Minimum oder ein lokales Maximum auftritt, bzw. dadurch dass ein vorgegebener Schwellwert durch eine der genannten Größen überschritten bzw. unterschritten wird.

Ferner sei darauf hingewiesen, dass ein Aktivitätsereignis typischerweise auf der Anregung der entsprechenden inneren Haarzelle durch eine Bewegung der Basilarmembran basiert.

Die Information über ein Aktivitätsmuster über der Zeit an der Mehrzahl von inneren Haarzellen des Gehörmodells umfasst somit eine Beschreibung einer Mehrzahl von zeitlichen Verläufen $A_1(t)$, $A_2(t)$, $A_n(t)$, wobei die einzelnen zeitlichen Verläufe separat die Anregung der zugeordneten inneren Haarzellen beschreiben.

Ein zweiter Schritt 120 des Verfahrens 100 umfasst, basierend auf der Information über das Aktivitätsmuster bzw. basierend auf den einzelnen zeitlichen Verläufen $A_1(t)$, $A_2(t)$ bis $A_n(t)$, ein Herausfiltern von Aktivitätsereignissen basierend auf einer Erkennung eines charakteristischen Musters in dem Aktivitätsmuster, um eine bereinigte Information zu erhalten. Dabei werden bevorzugt Aktivitätsereignisse aus dem Aktivitätsmuster herausgefiltert, die für eine Sprachverständlichkeit eines durch das Aktivitätsmuster beschriebenen Sprachsignals nur eine untergeordnete Bedeutung haben.

Das Herausfiltern der Aktivitätsereignisse kann entweder separat für jeden einzelnen der zeitlichen Verläufe $A_1(t)$, $A_2(t)$ bis $A_n(t)$ erfolgen, oder es kann ein gemeinsames Herausfiltern durchgeführt werden, wobei für das Herausfiltern jeweils mindestens zwei der zeitlichen Verläufe A1(t), A2(t) bis An(t) herangezogen werden. Durch das Herausfiltern entstehen somit bereinigte zeitliche Verläufe $A_{1,ber}(t)$, $A_{2,ber}(t)$ bis $A_{n,ber}(t)$, wobei jeder bereinigte zeitliche Verlauf $A_{i,ber}(t)$ einem entsprechenden zugehörigen zeitlichen Verlauf $A_i(t)$ zugeordnet ist. Ein bereinigter zeitlicher Verlauf $A_{i,ber}(t)$ enthält dabei die Aktivitätsereignisse, die in dem zugehörigen nicht-bereinigten zeitlichen Verlauf $A_1(t)$ enthalten sind, und die beispielsweise für eine Sprachverständlichkeit relevant sind.

In anderen Worten, es wird untersucht, ob in dem Aktivitätsmuster ein charakteristisches Muster enthalten wird. Ausgehend von einem erkannten charakteristischen Muster, das durch eine Mehrzahl von Aktivitätsereignissen gebildet wird, werden sodann Aktivitätsereignisse identifiziert, nicht zu dem erkannten Muster gehören, und aus der unbereinigten Information über das Aktivitätsmuster herausgefiltert (bzw. eliminiert). Alternativ können aber auch Aktivitätsereignisse, die Teil eines erkannten charakteristischen Musters sind, aus der unbereinigten Information herausgefiltert werden. Dabei müssen nicht notwendigerweise alle zu dem charakteristischen Muster gehörigen Aktivitätsereignisse herausgefiltert werden, wenngleich ein Herausfiltern von sämtlichen zu dem charakteristischen Muster gehörigen Aktivitätsereignissen eine Option darstellt. Vielmehr kann es beispielsweise ausreichend sein, in dem unbereinigten Aktivitätsmuster ein bestimmtes charakteristisches Muster zu erkennen, und dann Aktivitätsimpulse herauszufiltern, die einen echten Teil (im Sinne einer echten Teilmenge) des charakteristischen Musters bilden.

In dem bereinigten zeitlichen Verlauf von $A_{i,ber}$ (t) sind somit beispielsweise Aktivitätsereignisse, die in dem nicht-bereinigten zeitlichen Verlauf $A_i(t)$ enthalten sind, die aber nicht für eine Sprachverständlichkeit relevant sind, herausgefiltert bzw. entfernt.

Die bereinigten zeitlichen Verläufe $A_{1,ber}(t)$, $A_{2,ber}(t)$ bis $A_{n,ber}(t)$ bilden somit eine bereinigte Information, aus der im Vergleich zu der unbereinigten Information (die durch $A_1(t)$, $A_2(t)$ bis $A_n(t)$ beschrieben ist), Aktivitätsereignisse entfernt sind, die für eine Sprachverständigkeit nicht relevant sind.

In der bereinigten Information ist ferner bevorzugt eine zeitliche Relation von Aktivitätsereignissen, die für eine Sprachverständlichkeit als relevant erachtet werden (die also nicht ausgefiltert werden), gegenüber der unbereinigten Information unverändert. Mit anderen Worten, diejenigen Aktivitätsereignisse, die als für eine Sprachverständigkeit relevant erachtet werden, bleiben in der bereinigten Information zumindest zeitlich, gegebenenfalls aber auch amplitudenmäßig, unverändert erhalten.

Das Verfahren 100 umfasst ferner noch ein Verwenden der bereinigten Information als Ansteuersignal für ein Cochlea-Implantat oder ein Ableiten des Ansteuersignals von der bereinigten Information. In anderen Worten, aus der bereinigten Information, die bereinigte Zeitverläufe $A_{1,ber}(t)$, $A_{2,ber}(t)$ bis $A_{n,ber}(t)$ beschreibt bzw. umfasst, wird ein Ansteuersignal abgeleitet, das einem Cochlea-Implantat zugeführt wird. So wird basierend auf den durch die Zeitverläufe $A_{1,ber}(t)$, $A_{2,ber}(t)$ bis $A_{n,ber}(t)$ beschriebenen Aktivitätsereignissen ein Signal abgeleitet, das es dem Cochlea-Implantat ermöglicht, basierend darauf, elektrische Stimulationsimpulse für Nervenfasern der Hörnerven (Spiralganglienzellen) zu erzeugen.

Für den Fall, dass die bereinigte Information ein bereinigtes Neurotransmitter-Vesikel-Auftreten an einer Mehrzahl von inneren Haarzellen beschreibt, kann beispielsweise die bereinigte Information direkt oder in codierter Form als Ansteuersignal für ein Cochlea-Implantat verwendet werden. In ähnlicher Weise kann beispielsweise auch eine bereinigte Information, die bereinigte Aktionspotentiale an einer Mehrzahl von Hörnerven beschreibt, direkt für eine Ansteuerung eines Cochlea-Implantats verwendet werden. Das Ableiten eines Ansteuersignals von der bereinigten Information kann aber auch noch eine weitere Umwandlung der bereinigten Informationen umfassen, so dass aus der bereinigten Information eine neue Information abgeleitet wird, die für eine Ansteuerung des Cochlea-Implantats geeignet ist. Die zuletzt genannte Vorgehensweise ist beispielsweise sinnvoll, wenn die bereinigte Information noch nicht direkt für eine Ansteuerung eines Cochlea-Implantats geeignet ist, wenn die bereinigte Information also beispielsweise eine Auslenkung von Stereozilien von inneren Haarzellen oder eine Konzentration oder Freisetzung von Ionen in der inneren Haarzelle beschreibt.

Zusammenfassend lässt sich also festhalten, dass erkannt wurde, dass durch ein Herausfiltern von Aktivitätsereignissen aus einem Aktivitätsmuster an einer Mehrzahl von inneren Haarzellen basierend auf einer Erkennung eines charakteristischen Musters in dem Aktivitätsmuster mit besonders geringem Aufwand und besonders guten Ergebnissen Aktivitätsereignisse eliminiert werden können, die für eine Sprachverständlichkeit nicht relevant sind.

Der Grund dafür besteht darin, dass ein Anregungsmuster bzw. Aktivitätsmuster an bzw. in einer Mehrzahl von inneren Haarzellen über der Zeit Charakteristika, die für eine Sprachverständlichkeit relevant sind, in besonders deutlicher Weise erkennen lässt. Hierzu ist festzuhalten, dass in Außenohr, Mittelohr und Innenohr (Cochlea) eine Transformation von Sprachsignalen in einer Weise erfolgt, dass Anregungsmuster bzw. Aktivitätsmuster an bzw. in den inneren Haarzellen bei Vorliegen von Sprachsignalen (oder auch von manchen Störsignalen) eine charakteristische Struktur aufweisen. Anhand der Aktivitätsmuster über der Zeit an der Mehrzahl von inneren Haarzellen ist somit in einfacher Weise ersichtlich, welche Anteile der Aktivitätsmuster tatsächlich für eine Sprachverständlichkeit relevant sind.

Für eine Sprachverständlichkeit relevant sind beispielweise solche Anteile, die, wenn sie einem neuronalen Netz als ein paralleles Signal zugeführt werden, eine besonders starke Reaktion des neuronalen Netzes hervorrufen, da eine Erkennung von Sprachsignalen durch einen Menschen durch ein Netz von Neuronen erfolgt.

Es wurde im Übrigen herausgefunden, dass beispielsweise Aktivitätsereignisse für eine Sprachverständlichkeit nicht bzw. nur wenig relevant sind, die nicht zu einem Aktionspotential auf einem Hörnerven führen. Ferner wurde erkannt, dass einzelne Aktivitätsereignisse, die nicht einer Gruppe von miteinander in Verbindung stehenden bzw. korrelierten Aktivitätsereignissen angehören, für eine Sprachverständlichkeit nicht bzw. nur wenig relevant sind. Vokale, Konsonanten und Laute zeichnen sich nämlich jeweils dadurch aus, dass sie an einer Mehrzahl von inneren Haarzellen eines Gehörmodells eine Mehrzahl von Aktivitätsereignissen hervorrufen, die insgesamt ein für einen Vokal, Konsonanten oder Laut charakteristisches Muster beschreiben. Für eine Sprachverständlichkeit sind dabei beispielsweise solche Aktivitätsereignisse relevant, die durch eine Wanderwelle auf der Basilarmembran des Gehörmodells hervorgerufen werden. Derartige Wanderwellen beschreiben beispielsweise einen Ansatz eines Vokals und erscheinen ferner auch innerhalb der Feinstruktur des Vokals. Details diesbezüglich werden im übrigen noch im Laufe der Beschreibung näher erläutert.

Im Folgenden wird beschrieben, wie unter Verwendung eines Gehörmodells basierend auf einem Audiosignal ein Nervenaktivitätsmuster auf einer Mehrzahl von Gehörnerven, die mit inneren Haarzellen gekoppelt sind, berechnet werden kann. Aus den folgenden Ausführungen ist im Übrigen ersichtlich, welche Zwischen- bzw. Endgrößen der Berechnung für eine Beschreibung des unbereinigten Aktivitätsmusters herangezogen werden können.

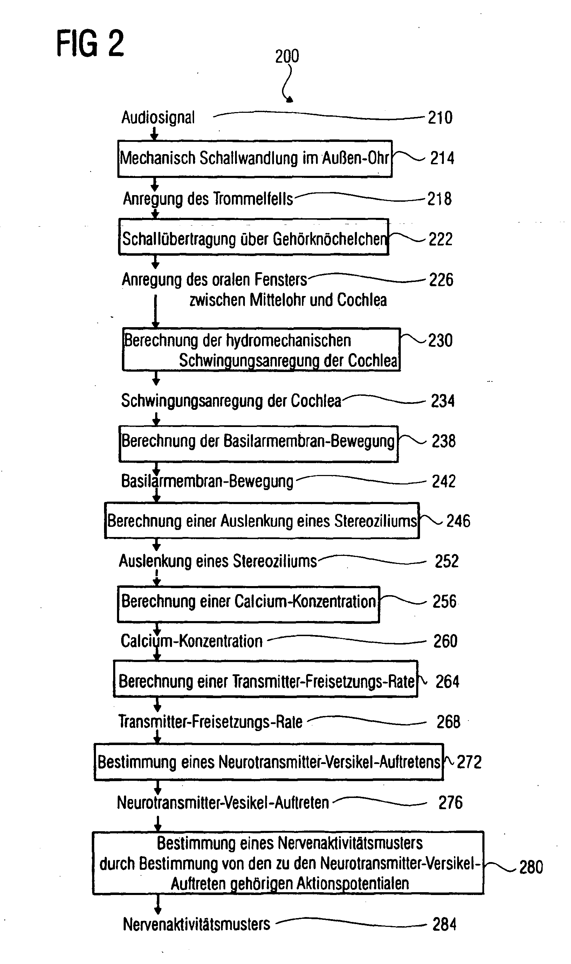

Die Fig. 2 zeigt zu diesem, Zweck eine schematische Darstellung des Ablaufs bei einer Simulation eines menschlichen Gehörs sowie der bei der Simulation auftretenden Zwischen- und Endergebnisse. Die schematische Darstellung der Fig. 2 ist in ihrer Gesamtheit mit 200 bezeichnet.

Die schematische Darstellung 200 der Fig. 2 beschreibt somit ein Simulationsmodell eines menschlichen Gehörs. Ein Audiosignal 210 dient als Eingangssignal für das Simulationsmodell 200. Basierend auf dem Audiosignal 210 wird in einem ersten Schritt 214 eine mechanische Schallwandlung in einem Außenohr ausgewertet, wodurch eine Anregung 218 eines Trommelfells ermittelt wird. In einem zweiten Schritt 222 wird eine Schallübertragung über Gehörknöchel berechnet bzw. simuliert, wodurch aus der Anregung 218 des Trommelfells eine Anregung 226 eines ovalen Fensters zwischen einem Mittelohr und einer Cochlea ermittelt wird. In einem dritten Schritt 230 wird eine hydromechanische Schwingungsanregung 234 der Cochlea berechnet bzw. simuliert. In einem vierten Schritt 238 wird aus der Schwingungsanregung 234 der Cochlea eine Basilarmembranbewegung 242 ermittelt. In einem fünften Schritt 246 wird von der Basilarmembranbewegung 242 auf eine Auslenkung 252 eines Stereoziliums gefolgert. Basierend auf der Auslenkung 252 des Stereoziliums wird sodann in einem sechsten Schritt 256 eine Calciumkonzentration 260 in einer Haarzelle berechnet. Die Calciumkonzentration 260 wird dann herangezogen, um in einem siebten Schritt 264 eine Transmitter-Freisetzungs-Rate 268 von Transmitter-Substanzen zu berechnen. Basierend auf der Transmitter-Freisetzungs-Rate 268 wird in einem achten Schritt 272 ein Neurotransmitter-Vesikel-Auftreten 276 hergeleitet, das ein Auftreten von Neurotransmitter-Vesikeln beschreibt. Schließlich wird in einem neunten Schritt 280 ein Nervenaktivitätsmuster 284 von dem Neurotransmitter-Vesikel-Auftreten 276 abgeleitet. Das Nervenaktivitätsmuster 284 beschreibt dabei näherungsweise eine bei einem gesunden Gehör auftretende Aktivität auf Nervenzellen eines (menschlichen oder tierischen) Hörnervs. Das Nervenaktivitätsmuster 284 ist somit gut geeignet, um eine Aussage über eine Stimulation von Hörnerven durch ein Cochlea-Implantat zu liefern.

Es wird darauf hingewiesen, dass im Rahmen des Simulationsmodells 200 mehrere der Schritte 214, 222, 230, 238, 246, 256, 264, 272, 280 zusammengefasst werden können, ohne ein entsprechendes Zwischenergebnis zu berechnen. In anderen Worten, es können mehrere Schritte in einem vereinfachten Schritt ohne Berechnung der in der graphischen Darstellung 200 gezeigten Zwischenschritte abgearbeitet werden.

Sowohl die Auslenkung 252 eines Stereoziliums, die Calciumkonzentration 260 in einer inneren Haarzelle, die Transmitter-Freisetzungs-Rate 268 in einer inneren Haarzelle, ein Neurotransmitter-Vesikel-Auftreten 276 in einer inneren Haarzelle oder ein an der inneren Haarzelle anliegendes Nervenaktivitätsmuster 284 (bzw. der zugehörige zeitliche Verlauf) können jeweils ein Aktivitätsmuster bzw. einen Teil eines Aktivitätsmusters an einer inneren Haarzelle darstellen.

In anderen Worten, ein zeitlicher Verlauf einer Auslenkung eines zu einer inneren Haarzelle gehörigen Stereoziliums stellt beispielsweise einen zeitlichen Verlauf dar, wie er in dem Flussdiagramm 100 mit $A_1(t)$ bezeichnet ist. Entsprechende zeitliche Verläufe von Auslenkungen von Stereozilien, die zu weiteren inneren Haarzellen gehören, bilden bei diesem Beispiel die weiteren nicht-bereinigten zeitlichen Verläufe $A_2(t)$ bis $A_n(t)$. Bei dem eben beschriebenen Beispiel werden beispielsweise derartige Auslenkungen der Stereozilien, die in dem Nervenaktivitätsmuster 284 nicht zu Aktionspotentialen führen, herausgefiltert, da derartige Auslenkungen der Stereozilien für eine Sprachverständigkeit nicht relevant sind.

Ebenso können beispielsweise die Konzentrationen 260 von Calcium-Ionen in einer Mehrzahl von inneren Haarzellen ein Aktivitätsmuster bilden, wobei ein zeitlicher Verlauf der Calciumkonzentration 260 in der i-ten inneren Haarzelle den i-ten zeitlichen Verlauf $A_i(t)$ bildet. Ein Aktivitätsereignis ist bei Betrachtung der Calciumkonzentration beispielsweise ein signifikanter Anstieg der Calciumkonzentration über einen bestimmten Schwellwert oder ein Abfall der Calciumkonzentration unter einem bestimmten Schwellwert.

Im übrigen können die zeitlichen Verläufe $A_i(t)$ beispielsweise auch nur eine Abweichung der gegenwärtigen Calciumkonzentrationen von Gleichgewichts-Calciumkonzentrationen beschreiben. Ein Aktivitätsereignis wird in diesem Fall durch eine signifikante Abweichung der jeweiligen Calcium-Konzentrationen nach oben bzw. nach unten beschrieben. Gemäß der vorliegenden Erfindung werden bei der Verwendung einer Beschreibung der Calciumkonzentration als das Aktivitätsmuster in dem Schritt 120 solche Schwankungen der Calciumkonzentration herausgefiltert, die nicht zu einer Sprachverständlichkeit beitragen bzw. für eine Sprachverständlichkeit nicht relevant sind. Damit entsteht eine bereinigte Information $A_{i,ber}(t)$, in der die Schwankungen der Calciumkonzentration, die für eine Sprachverständlichkeit nicht relevant sind, beispielsweise nicht mehr enthalten bzw. zu Null gesetzt sind.

Ferner kann auch der zeitliche Verlauf einer Transmitter-Freisetzungs-Rate 268 oder einer Transmitter-Freisetzungs-Wahrscheinlichkeit in einer Mehrzahl von inneren Haarzellen als Aktivitätsmuster über der Zeit verwendet werden. Die zeitlichen Verläufe $A_i(t)$ werden dabei durch die Transmitter-Freisetzungs-Rate oder Transmitter-Freisetzungs-Wahrscheinlichkeit in der i-ten inneren Haarzelle gebildet. Wird in dem zweiten Schritt 120 gemäß dem Flussdiagramm 100 basierend auf einer Erkennung eines charakteristischen Musters in dem Aktivitätsmuster beispielsweise festgestellt, dass eine nicht-verschwindende Transmitter-Freisetzungs-Rate 268 bzw. Transmitter-Freisetzungs-Wahrscheinlichkeit für eine Sprachverständlichkeit nicht relevant ist, so wird beispielsweise im Rahmen des zweiten Schritts 120 die gemäß Fig. 2 berechnete Transmitter-Freisetzungs-Rate 268 bzw. Transmitter-Freisetzungs-Wahrscheinlichkeit in dem bereinigten Aktivitätsmuster $A_{1,ber}(t)$ bis $A_{n,ber}(t)$ zu Null gesetzt. Im Übrigen wird darauf hingewiesen, dass bei der Betrachtung der Transmitter-Freisetzungs-Rate 268 bzw. Transmitter-Freisetzungs-Wahrscheinlichkeit beispielsweise das Vorliegen einer Transmitter-Freisetzungs-Rate größer Null bzw. eine Transmitter-Freisetzungs-Wahrscheinlichkeit größer Null als ein Aktivitätsereignis verstanden werden kann.

Ferner kann auch ein Neurotransmitter-Vesikel-Auftreten 276 in einer Mehrzahl von inneren Haarzellen ein Aktivitätsmuster bilden. Die zeitlichen Verläufe $A_i(t)$ werden dabei beispielsweise durch eine Anzahl an freigesetzten Neurotransmitter-Vesikeln gebildet. In anderen Worten, ein zeitlicher Verlauf $A_i(t)$ beschreibt in dem genanten Fall, wie viele Neurotransmitter-Vesikel beispielsweise während eines bestimmten Zeitraums bzw. Zeitintervalls freigesetzt vorliegen. Das Neurotransmitter-Vesikel-Auftreten 276 kann allerdings auch beschreiben, wie viele Neurotransmitter-Vesikel während eines Zeitraums bzw. eines Zeitintervalls neu freigesetzt werden. Der entsprechende zeitliche Verlauf kann ferner beispielsweise beschreiben, wie viele Neurotransmitter-Vesikel in der präsynaptischen inneren Haarzelle in einer Zeiteinheit freigesetzt werden oder in einem Zeitintervall oder zu einem Zeitpunkt insgesamt in freier Form vorhanden sind. Ferner kann das Neurotransmitter-Vesikel-Auftreten 276 aber auch beschreiben, wie viele Neurotransmitter-Vesikel beispielsweise pro Zeiteinheit aus der präsynaptischen inneren Haarzelle in den synaptischen Spalt diffundieren, der die präsynaptische innere Haarzelle mit einer Nervenfaser koppelt.

In anderen Worten, unter einem Neurotransmitter-Vesikel-Auftreten ist beispielsweise eine Anzahl von tatsächlich vorhandenen oder pro Zeiteinheit freigesetzten Neurotransmitter-Vesikeln zu verstehen, wobei es für die vorliegende Erfindung nicht relevant ist, wo genau innerhalb einer Haarzelle oder Synapse die entsprechende Anzahl an Neurotransmitter-Vesikeln oder die entsprechende Freisetzungsrate von Neurotransmitter-Vesikeln bestimmt wird. Es ist nämlich davon auszugehen, dass Neurotransmitter-Vesikel, die im präsynaptischen Teil der inneren Haarzelle freigesetzt werden, mit einer gewissen Zeitkonstante in den synaptischen Spalt diffundieren.

Im Übrigen wird darauf hingewiesen, dass ein Neurotransmitter-Vesikel-Auftreten sowohl qualitativ als auch quantitativ beschrieben werden kann, um ein Aktivitätsmuster im Sinne der vorliegenden Erfindung zu beschreiben. In anderen Worten, ein zu einem Neurotransmitter-Vesikel-Auftreten 276 gehöriger zeitlicher Verlauf $A_i(t)$ kann beispielsweise beschreiben, wie viele Neurotransmitter-Vesikel freigesetzt werden oder in freier Form vorhanden sind. De Verlauf $A_i(t)$ kann auch nur eine qualitative Information darüber geben, ob Neurotransmitter-Vesikel in einem Zeitintervall freigesetzt werden bzw. in freier Form vorhanden sind.

Bei der Betrachtung eines Neurotransmitter-Vesikel-Auftretens 276 in bzw. an einer Mehrzahl von inneren Haarzellen eines Gehörmodells als das Aktivitätsmuster können als Aktivitätsereignisse beispielsweise die Freisetzung einer Anzahl an Neurotransmitter-Vesikeln in einer Zeiteinheit, die größer als ein bestimmter Schwellwert ist, angesehen werden. Beispielsweise kann angenommen werden, dass immer dann ein Aktivitätsereignis auftritt, wenn überhaupt ein Neurotransmitter-Vesikel innerhalb eines Zeitintervalls freigesetzt wird. Ferner kann angenommen werden, dass ein Aktivitätsereignis vorliegt, wenn zumindest ein (oder allgemeiner: mehr als eine vorgegebene minimale Anzahl) freies Neurotransmitter-Vesikel in einer inneren Haarzelle vorliegt.

In anderen Worten, ein Aktivitätsereignis ist ein Ereignis, das in einer einzigen inneren Haarzelle auftritt. Ein Aktivitätsereignis macht sich typischerweise in einem lokalen Minimum oder Maximum auf einem der unbereinigten zeitlichen Verläufe $A_i(t)$ bis $A_n(t)$ oder in einer Über- bzw. Unterschreitung eines Schwellwerts bemerkbar.

Ferner kann als unbereinigtes Aktivitätsmuster auch ein Nervenaktivitätsmuster an einer Mehrzahl von inneren Haarzellen eines Gehörmodells verwendet werden. Das Nervenaktivitätsmuster beschreibt dabei die Aktivität bzw. den zeitlichen Verlauf der Aktivität auf mehreren verschiedenen Nervenfasern, die mit mehreren verschiedenen inneren Haarzellen gekoppelt sind. Jedem zeitlichen Verlauf $A_i(t)$ ist beispielsweise ein zeitlicher Verlauf eines Potentials bzw. einer Spannung an einer mit einer i-ten inneren Haarzelle gekoppelten Nervenfaser zugeordnet. Auf den einzelnen Nervenfasern treten dabei Aktionspotentiale auf, deren Auftreten durch die nicht-bereinigten Zeitverläufe $A_i(t)$ beschrieben wird. Unter einem Aktivitätsereignis ist in diesem Falle das Auftreten eines Aktionspotentials auf einer der n betrachteten Nervenfasern zu verstehen.

Zusammenfassend lässt sich also festhalten, dass in einem Verfahren gemäß dem Flussdiagramm 200 verschiedene Größen 252, 260, 268, 276 für eine Mehrzahl von verschiedenen inneren Haarzellen berechnet werden können, wobei ein Aktivitätsmuster durch eine Zusammenfassung der gleichen Größe für eine Mehrzahl von unterschiedlichen Haarzellen gebildet wird.

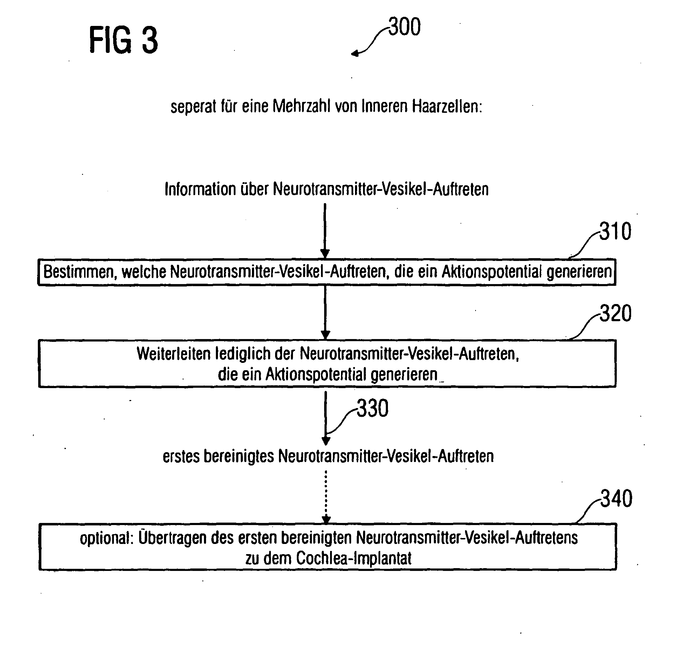

Im Folgenden wird anhand eines konkreten Beispiels beschrieben, wie aus den unbereinigten Zeitverläufe $A_i(t)$ bereinigte Zeitverläufe $A_{i,ber}(t)$ erzeugt werden. Fig. 3 zeigt daher ein Flussdiagramm eines Verfahrens zum Bestimmen eines ersten bereinigten Neurotransmitter-Vesikel-Auftretens aus einer Information über einen Neurotransmitter-Vesikel-Auftreten. Das Verfahren der in Fig. 3 ist im Übrigen in seiner Gesamtheit mit 300 bezeichnet.

Das Verfahren 300 der Fig. 3 wird im Übrigen typischerweise separat für eine Mehrzahl von inneren Haarzellen parallel durchgeführt. Es wird hier allerdings der Einfachheit halber das Verfahren 300 nur für eine einzige innere Haarzelle gezeigt. Das Verfahren 300 empfängt als eine Eingangsgröße eine Information über einen Neurotransmitter-Vesikel-Auftreten in einer inneren Haarzelle eines Gehörmodells. Wie oben beschrieben kann das Neurotransmitter-Vesikel-Auftreten beispielsweise eine Anzahl an in einem Zeitintervall im Mittel vorhandenen Neurotransmitter-Vesikeln im präsynaptischen oder postsynaptischen Teil der inneren Haarzelle beschreiben. Ferner kann das Neurotransmitter-Vesikel-Auftreten beschreiben, wie viele Neurotransmitter-Vesikel in einem bestimmten Zeitraum freigesetzt werden oder an einem bestimmten Ort (beispielsweise in dem synaptischen Spalt) eintreffen. Das Neurotransmitter-Vesikel-Auftreten ist im Übrigen durch einen zeitlichen Verlauf entweder in zeitkontinuierlicher oder zeitdiskreter Form gegeben. In anderen Worten, die entsprechenden oben genannten Größen werden entweder zeitkontinuierlich oder für eine Mehrzahl von Zeitintervallen bereit gestellt.

Ein Vorliegen eines Neurotransmitter-Vesikel-Auftretens beschreibt im Folgenden, dass die entsprechende Größe (Anzahl an vorhandenen oder freigesetzten Neurotransmitter-Vesikeln) einen nicht-verschwindenden, das heißt von Null verschiedenen, Wert annimmt.

In dem ersten Schritt 310 wird bestimmt, welche Neurotransmitter-Vesikel-Auftreten aus dem zeitlichen Verlauf der Neurotransmitter-Vesikel-Auftreten ein Aktionspotential generieren. In anderen Worten, der zeitliche Verlauf des Neurotransmitter-Vesikel-Auftretens wird analysiert, und es werden diejenigen Neurotransmitter-Vesikel-Auftreten (unabhängig davon, ob dies durch einen zeitkontinuierlichen oder zeitdiskreten Werteverlauf beschrieben wird) bestimmt, die tatsächlich ein Aktionspotential generieren, oder die alternativ zumindest zu einer Generierung eines Aktionspotentials beitragen.

Hierzu sei darauf hingewiesen, dass nicht jedes Neurotransmitter-Vesikel, das freigesetzt wird, automatisch auch in einem Aktionspotential resultiert. Beispielsweise ist es in manchen Fällen für die Freisetzung eines Aktionspotentials erforderlich, dass innerhalb eines bestimmten Zeitintervalls mindestens zwei Neurotransmitter-Vesikel (oder mindestens eine vorgegebene Anzahl an Neurotransmitter-Vesikeln) auftreten.

Tritt in dem entsprechenden Zeitintervall hingegen nur ein Neurotransmitter-Vesikel auf, wird dieses einzelne Neurotransmitter-Vesikel-Auftreten in dem unbereinigten Aktivitätsmuster dennoch als ein Aktivitätsereignis gewertet. Das eine, einzelne Neurotransmitter-Vesikel resultiert aber nicht in einem Aktionspotential, da innerhalb des vorgegebenen Zeitintervalls keine weiteren Neurotransmitter-Vesikel in der betrachteten inneren Haarzelle auftreten. Allgemein gesprochen kann somit festgehalten werden, dass beispielsweise ein Neurotransmitter-Vesikel-Auftreten kein Aktionspotentials generiert, wenn nicht innerhalb eines vorgegebenen Zeitintervalls im Anschluss an das entsprechende Neurotransmitter-Vesikel mindestens eine vorgegebene Anzahl an zusätzlichen Neurotransmitter-Vesikeln auftreten.

In anderen Worten, es kann beispielsweise der Verlauf der Neurotransmitter-Vesikel-Auftreten für verschiedene (bevorzugt zeitlich überlappte) Intervalle analysiert. Für Neurotransmitter-Vesikel, die nicht zumindest in einem Zeitintervall liegen, in dem insgesamt mehr als eine vorgegebene Anzahl an Neurotransmitter-Vesikeln liegen, wird festgestellt, dass die entsprechenden Neurotransmitter-Vesikel kein Aktionspotential generieren, und die entsprechenden Neurotransmitter-Vesikel-Auftreten werden nicht in die erste bereinigte Information übernommen.

Ferner wird darauf hingewiesen, dass im Anschluss an die Freisetzung eines Aktionspotentials aufgrund eines Neurotransmitter-Vesikel-Auftretens eine sog. Refraktärzeit auftritt, während derer neuerliche Neurotransmitter-Vesikel-Auftreten unwirksam sind. Wird daher durch eine Analyse des Neurotransmitter-Vesikel-Auftretens, die auch eine Bestimmung umfassen kann, zu welchen Zeitpunkten, basierend auf dem Neurotransmitter-Vesikel-Auftreten Aktionspotentiale generiert werden, festgestellt, dass bestimmte Neurotransmitter-Vesikel-Auftreten während einer Refraktärzeit (im Anschluss an die Freisetzung eines Aktionspotentials) liegen, so wird für die bestimmten Neurotransmitter-Vesikel-Auftreten festgestellt, dass diese kein Aktionspotential generieren.

Ist somit bekannt, welche Neurotransmitter-Vesikel-Auftreten kein Aktionspotential generieren oder nicht zur Erzeugung eines Aktionspotentials beitragen, so kann daraus gefolgert werden, welche Neurotransmitter-Vesikel-Auftreten ein Aktionspotential generieren oder zur Erzeugung eines Aktionspotentials zumindest beitragen.

Ein zweiter Schritt 320 umfasst daher ein Weiterleiten lediglich der Neurotransmitter-Vesikel-Auftreten, die ein Aktionspotential generieren, oder die zu einer Erzeugung eines Aktionspotentials beitragen. Somit entsteht ein erstes bereinigtes Neurotransmitter-Vesikel-Auftreten, in dem nur noch Neurotransmitter-Vesikel enthalten sind, die tatsächlich ein Aktionspotential generieren oder zu einer Erzeugung eines Aktionspotentials zumindest beitragen.

Das erste bereinigte Neurotransmitter-Vesikel-Auftreten ist im Übrigen mit 330 bezeichnet.

Ein vierter optionaler Schritt 340 umfasst im Übrigen noch ein Übertragen des ersten bereinigten Neurotransmitter-Vesikel-Auftretens zu einem Cochlea-Implantat.

Das Verfahren 300 kann in einer vielfältigen Weise modifiziert werden. Beispielsweise kann das Bestimmen, welche Neurotransmitter-Vesikel-Auftreten ein Aktionspotential generieren, durch ein Bestimmen, welche Neurotransmitter-Vesikel-Auftreten kein Aktionspotential generieren, ersetzt werden.

Das Bestimmen, welche Neurotransmitter-Vesikel-Auftreten ein Aktionspotential generieren oder zu einer Erzeugung eines - Aktionspotentials zumindest beitragen, kann beispielsweise ein vollständiges Ableiten von Aktionspotentialen aus dem zeitlichen Verlauf des Neurotransmitter-Vesikel-Auftretens (also der Information über das Neurotransmitter-Vesikel-Auftreten) umfassen. Basierend auf der Bestimmung der Aktionspotentiale aus dem zeitlichen Verlauf der Neurotransmitter-Vesikel-Auftreten kann dann gefolgert werden, welche der Neurotransmitter-Vesikel-Auftreten zur Erzeugung eines Aktionspotentials beitragen. Ferner kann auch gefolgert werden, welche der Neurotransmitter-Vesikel-Auftreten unmittelbar die Auslösung eines Aktionspotentials zur Folge haben. Jedes Aktionspotential kann nämlich eindeutig auf ein auslösendes Neurotransmitter-Vesikel zurückgeführt und zugeordnet verden.

Das zugeordnete auslösende Neurotransmitter-Vesikel-Auftreten kann somit als ein Neurotransmitter-Vesikel-Auftreten betrachtet werden, das ein Aktionspotential generiert. In dem eben beschriebenen Fall werden somit in dem zweiten Schritt 320 lediglich diejenigen Neurotransmitter-Vesikel-Auftreten weitergeleitet, die ein Aktionspotential unmittelbar generieren. In anderen Worten, tragen mehrere Neurotransmitter-Vesikel zu der Auslösung eines Aktionspotentials bei, so wird lediglich das letzte Neurotransmitter-Vesikel-Auftreten, durch das ein Potential-Schwellwert für eine Auslösung eines Neurotransmitter-Vesikel überschritten wird, weitergeleitet.

Andererseits ist es auch möglich, dass sämtliche Neurotransmitter-Vesikel-Auftreten, die zu einer Erzeugung eines Aktionspotentials beitragen, bei der Erzeugung des ersten bereinigten Neurotransmitter-Vesikel-Auftretens weitergeleitet werden.

Im übrigen ist eine Auslösung eines Aktionspotentials mit dem Auftreten eines charakteristischen Musters in dem Aktivitätsmuster gekoppelt. So resultiert ein Auftreten einer Mehrzahl von Neurotransmitter-Vesikel innerhalb eines bestimmten Zeitraums (also ein zeitlich "konzentriertes" Auftreten von mehreren Neurotransmitter-Vesikeln) in einer Freisetzung eines Aktionspotentials. Das entsprechende charakteristische Muster kann somit auch direkt in dem Aktivitätsmuster erkannt werden.

Zusammenfassend lässt sich also festhalten, dass zumindest zwei Möglichkeiten bestehen, um zu entscheiden, welche Neurotransmitter-Vesikel-Auftreten weitergeleitet werden. Beispielsweise können im Rahmen der anhand der in Fig. 3 beschriebenen ersten Rückfilterung nur die Neurotransmitter-Vesikel gespeichert und selektiv an das Cochlea-Implantat weitergeleitet werden, die ein Aktionspotential generieren. Alternativ dazu ist es möglich, sämtliche Neurotransmitter-Vesikel-Auftreten in das erste bereinigte Neurotransmitter-Vesikel-Auftreten aufzunehmen, die zu einer Erzeugung eines Aktionspotentials beitragen, und lediglich diejenigen Neurotransmitter-Vesikel-Auftreten aus dem unbereinigten Neurotransmitter-Vesikel-Auftreten zu eliminieren, die nicht zu einer Erzeugung eines Aktionspotentials beitragen, da sie beispielsweise innerhalb einer Refraktärzeit der zugehörigen Synapse bzw. des zugehörigen Hörnerven auftreten.

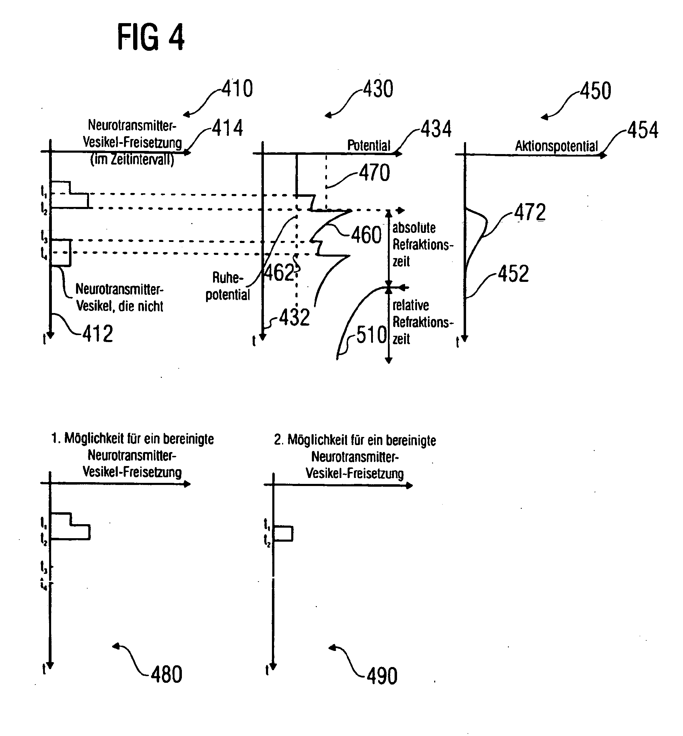

Weitere Details diesbezüglich werden ferner noch anhand der Fig. 4 beschrieben. Fig. 4 zeigt eine schematische Darstellung der Abläufe bei einem Ableiten eines ersten bereinigten Neurotransmitter-Vesikel-Auftretens aus einer Information über ein Neurotransmitter-Vesikel-Auftreten. Eine erste graphische Darstellung 410 beschreibt eine Information über ein Neurotransmitter-Vesikel-Auftreten über der Zeit. An einer Abszisse 412 ist dabei die Zeit angetragen, während eine Ordinate 414 eine Neurotransmitter-Vesikel-Freisetzung pro Zeitintervall beschreibt. Die Neurotransmitter-Vesikel-Freisetzung pro Zeitintervall nimmt dabei bevorzugt diskrete Werte an. Eine zweite graphische Darstellung 430 beschreibt ein Potential in einem synaptischen Spalt als Funktion der Zeit. Eine Abszisse 432 beschreibt dabei die Zeit, während hingegen eine Ordinate 434 das Potential beschreibt. Eine dritte graphische Darstellung 450 zeigt einen zeitlichen Verlauf eines Aktionspotentials auf einer Nervenfaser, wobei das Aktionspotential durch die in der graphischen Darstellung 410 gezeigte Neurotransmitter-Vesikel-Freisetzung bzw. durch das in der graphischen Darstellung 430 gezeigte postsynaptische Potential verursacht wird. Eine Abszisse 452 beschreibt wiederum die Zeit, während eine Ordinate 454 einen Spannungsverlauf auf einer Nervenfaser beschreibt. Ein Verlauf des postsynaptischen Potentials ist im Übrigen mit 460 gekennzeichnet.

Das postsynaptische Potential nimmt in einem Gleichgewichtszustand ein Ruhepotential 462 ein. Ansprechend auf eine Freisetzung eines Neurotransmitter-Vesikels erhöht sich da postsynaptische Potential V, um dann mit einer bestimmten Zeitkonstante wieder abzuklingen. So tritt beispielsweise zu einem Zeitpunkt $t_1$ eine Neurotransmitter-Vesikel-Freisetzung auf, woraufhin sich das postsynaptische Potential V erhöht. Zu einem zweiten Zeitpunkt $t_2$ werden beispielsweise zwei weitere Neurotransmitter-Vesikel freigesetzt, so dass das postsynaptische Potential V weiter ansteigt und schließlich einen zeitabhängigen Schwellenwert 470 überschreitet. Daraufhin wird auf der Nervenfaser ein Aktionspotential ausgelöst, wie aus der dritten graphischen Darstellung ersichtlich ist (vgl. Kurve 472). Während das zeitabhängige Schwellenpotential 470 vor der Auslösung eines Aktionspotentials nahezu konstant ist, steigt das zeitabhängige Schwellenpotential 770 direkt nach der Auslösung eines Aktionspotentials auf einen sehr hohen bzw. unendlichen Wert an und verbleibt dort während einer absoluten Refraktärzeit. Während der absoluten Refraktärzeit ist somit keine weitere Auslösung eines Aktionspotential-s-möglich. So treten zwar zu Zeitpunkten $t_3$, $t_4$ weitere Neurotransmitter-Vesikel-Freisetzungen auf, die zu einer Erhöhung des postsynaptischen Potentials V führen, es wird aber kein weiteres Aktionspotential freigesetzt, da sich die Synapse bzw. die Nervenfaser innerhalb der absoluten Refraktärzeit befindet.

Es sei hier ferner darauf hingewiesen, dass sich an die absolute Refraktärzeit eine relative Refraktärzeit anschließt, während der das Schwellenpotential 470 langsam bis auf den Gleichgewichtswert 462 zurückgeht.

Basierend auf der anhand der graphischen Darstellungen 410, 430 und 450 beschriebenen Analyse bestehen zwei Möglichkeiten, eine bereinigte Neurotransmitter-Vesikel-Freisetzung abzuleiten.

Bei dem ersten Ansatz werden in die bereinigte Neurotransmitter-Vesikel-Freisetzung alle die Neurotransmitter-Vesikel-Freisetzungen übernommen, die zu einer Erzeugung von Aktionspotentials beitragen. Dies sind beispielsweise die Neurotransmitter-Vesikel-Freisetzungen, die im Anschluss an die Zeitpunkte $t_1$ und $t_2$ auftreten. Hingegen werden die Neurotransmitter-Vesikel-Freisetzungen, die zu den Zeitpunkten $t_3$ und $t_4$ auftreten, nicht in die bereinigte Neurotransmitter-Vesikel-Freisetzung übernommen. Zwar tragen auch die zu den Zeitpunkten $t_3$ und $t_4$ auftretenden Neurotransmitter-Vesikel-Freisetzungen zu der Erhöhung des postsynaptischen Potentials V bei, jedoch klingt das postsynaptische Potential V vor der Freisetzung eines nächsten Aktionspotentials soweit ab, dass die Neurotransmitter-Vesikel-Freisetzungen zu den Zeitpunkten $t_3$ und $t_4$ keinen Einfluss auf eine Freisetzung eines Aktionspotentials haben. In anderen Worten, die Neurotransmitter-Vesikel-Freisetzungen zu den Zeitpunkten $t_3$ und $t_4$ beeinflussen, dass das Potential beim Auftreten eines nächsten Aktionspotentials so wenig, dass ein Zeitpunkt eines Auftretens eines nächsten Aktionspotentials durch die Neurotransmitter-Vesikel-Auftreten zu den Zeitpunkten $t_3$ und $t_4$ höchstens in vernachlässigbarer Weise beeinflusst wird.

Die graphische Darstellung 480 zeigt somit, dass in ein bereinigtes Neurotransmitter-Vesikel-Auftreten beispielsweise nur die Neurotransmitter-Vesikel-Auftreten zu den Zeitpunkten $t_1$ und $t_2$ übernommen werden.

Eine zweite graphische Darstellung 490 beschreibt eine zweite Möglichkeit für eine bereinigte Neurotransmitter-Vesikel-Freisetzung. So ist festzuhalten, dass die Freisetzung des Aktionspotentials 472 ansprechend auf die Freisetzung eines Neurotransmitter-Vesikels zu dem Zeitpunkt $t_2$ erfolgt. In anderen Worten, das Neurotransmitter-Vesikel, das zu dem Zeitpunkt $t_2$ auftritt, "erzeugt" das Aktionspotential 472 bzw. löst das Aktionspotential 472 unmittelbar aus. Somit besteht die Möglichkeit, in eine bereinigte Neurotransmitter-Vesikel-Freisetzung lediglich das das Aktionspotential tatsächlich auslösende Neurotransmitter-Vesikel zu übernehmen. Somit zeigt die graphische Darstellung 490 lediglich ein einziges Neurotransmitter-Vesikel, das zu dem Zeitpunkt $t_2$ auftritt, und das den Ausschlag für eine Auslösung eines Aktionspotentials 472 ergibt.

In anderen Worten, es wird ein Muster in dem Neurotransmitter-Vesikel-Auftreten erkannt, das zu einer Auslösung eines Aktionspotentials führt, beispielsweise das Vorliegen einer Anzahl an zeitlich kurz aufeinanderfolgenden Neurotransmitter-Vesikel-Auftreten. Diese werden in das bereinigte Aktivitätsmuster übernommen. Ferner wird erkannt, dass kurz nach dem ersten Neurotransmitter-Vesikel-Auftreten (zu den Zeitpunkten t1, t2) ein weiteres Neurotransmitter-Vesikel-Auftreten folgt (zu den Zeitpunkten t3, t4). Das weitere Neurotransmitter-Vesikel-Auftreten wird nicht in das bereinigte Neurotransmitter-Vesikel-Auftreten übernommen. Allgemein gesprochen werden somit in dem Aktivitätsmuster beispielsweise solche charakteristische Muster (direkt durch eine Mustererkennung oder indirekt über eine Ableitung einer Zwischengröße von dem Aktivitätsmuster, beispielsweise durch eine lineare oder nicht-lineare Filterung mit anschließender Schwellwertentscheidung) erkannt, die zu einer Auslösung eines Aktionspotentials führen. Basierend auf der Erkennung eines charakteristischen Musters wird dann das bereinigte Aktivitätsmuster ermittelt.

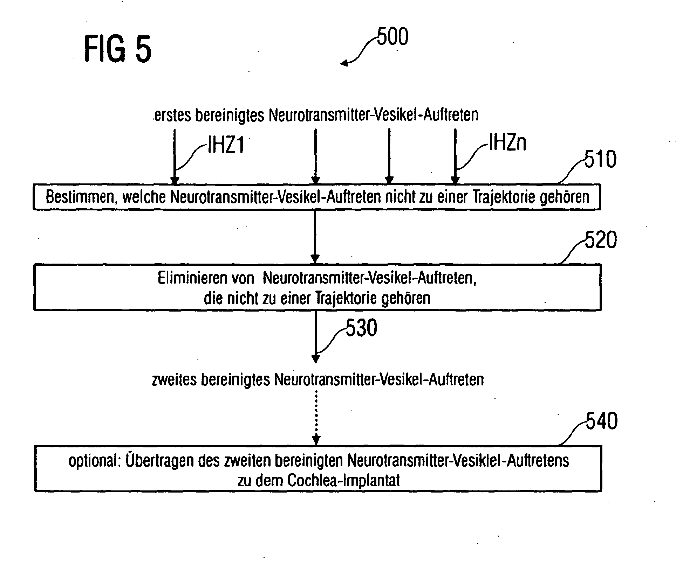

Bei einem weiteren Ausführungsbeispiel der vorliegenden Erfindung wird ein weiteres Konzept eingesetzt, das es ermöglicht, aus dem Aktivitätsmuster über der Zeit an der Mehrzahl von inneren Haarzellen eines Gehörmodells eine Information herauszufiltern, die für eine Sprachverständlichkeit nicht relevant ist. Zur Erläuterung dieses Konzepts zeigt die Fig. 5 ein Flussdiagramm eines Verfahrens zum Ableiten eines zweiten bereinigten Neurotransmitter-Vesikel-Auftretens aus dem ersten bereinigten Neurotransmitter-Vesikel-Auftreten. Das erfindungsgemäße Verfahren der Fig. 5 ist in seiner Gesamtheit mit 500 bezeichnet. Das Verfahren 500 empfängt als Eingangsinformation beispielsweise das erste bereinigte Neurotransmitter-Vesikel-Auftreten. Basierend auf dem ersten bereinigten Neurotransmitter-Vesikel-Auftreten wird in einem ersten Schritt 510 bestimmt, welche Neurotransmitter-Vesikel-Auftreten nicht zu einer Trajektorie gehören. In einem zweiten Schritt 520 werden Neurotransmitter-Vesikel-Auftreten, die nicht zu einer Trajektorie gehören, eliminiert, um ein zweites bereinigtes Neurotransmitter-Vesikel-Auftreten 530 zu erzeugen. Optional kann in einem weiteren Schritt 540 das zweite bereinigte Neurotransmitter-Vesikel-Auftreten zu dem Cochlea-Implantat übertragen werden.

Es sei hierbei darauf hingewiesen, dass bei dem Verfahren 500 das erste bereinigte Neurotransmitter-Vesikel-Auftreten an mehreren inneren Haarzellen (IHZI bis IHZn) gleichzeitig und gekoppelt verarbeitet wird. Bei dem Bestimmen 510, welche Neurotransmitter-Vesikel-Auftreten nicht zu einer Trajektorie gehören (bzw. welche Neurotransmitter-Vesikel-Auftreten zu einer Trajektorie gehören) werden die Neurotransmitter-Vesikel-Auftreten an mehreren inneren Haarzellen gemeinsam verarbeitet, da eine Trajektorie stets das Vorliegen von Aktivitätsereignissen an einer Mehrzahl von inneren Haarzellen erfordert.

Um ein Verständnis des Verfahrens 500 zu erleichtern, wird im Folgenden der Aufbau der Cochlea sowie die Entstehung von Trajektorien bzw. Verzögerungstrajektorien kurz erläutert.

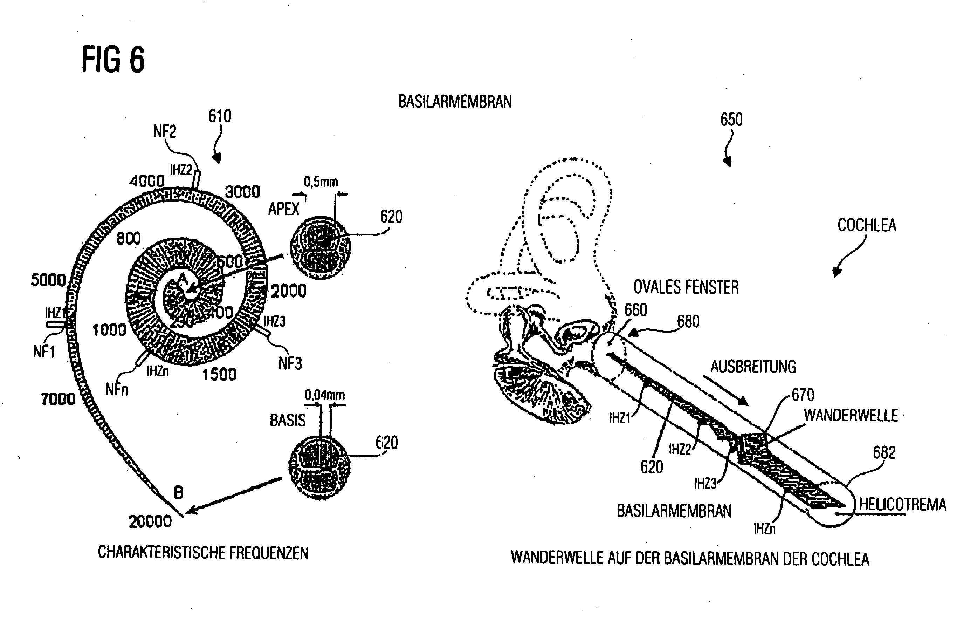

Fig. 6 zeigt zu diesem Zweck eine graphische Darstellung einer Geometrie der Basilarmembran und eine Reaktion der Basilarmembran auf eine Anregung.

Eine erste graphische Darstellung 610 zeigt, dass eine Breite einer Basilarmembran 620 von der Basis der Cochlea zu dem Ende (Apex) der Cochlea hin um etwa. den Faktor 10 zunimmt. Ferner zeigt die graphische Darstellung 610, verschiedene charakteristische Frequenzen (in Hertz), hinsichtlich derer an verschiedenen Orten der Cochlea eine maximale Empfindlichkeit besteht. In der Nähe der Basis der Cochlea werden Frequenzen in der Größenordnung von 20.000 Hertz am stärksten wahrgenommen. Von der Basis der Cochlea aus betrachtet, nimmt die Frequenz, für die sich eine maximale Empfindlichkeit ergibt, kontinuierlich ab. Die graphische Darstellung 610 zeigt ferner vier beispielhafte innere Haarzellen IHZ1, IHZ2, IHZ3, IHZn, die entlang der Cochlea angeordnet sind, und die mit zugehörigen Nervenfasern NF1, NF2, NF3, NFn gekoppelt sind. Unter der Annahme einer sinusförmigen Anregung der Cochlea spricht somit beispielsweise die erste innere Haarzelle IHZ1 am stärksten bei einer Anregung mit einer Frequenz von etwa 6.000 Hz an. Die zweite innere Haarzelle IHZ2 weist hingegeben beispielsweise eine maximale Empfindlichkeit bei einer Anregung mit einer Frequenz von etwa 3.300 Hz auf. Analog weisen die übrigen inneren Haarzellen IHZ3, IHZn andere Frequenzen maximaler Empfindlichkeit auf.

Eine zweite graphische Darstellung 650 beschreibt ferner eine Einkopplung einer akustischen Welle in die Cochlea über ein ovales Fenster 660. Die Einkopplung über das ovale Fenster 660 erzeugt eine Wanderwelle 670 in der Cochlea, die von einer Basis 680 der Cochlea zu einem Apex 682 der Cochlea läuft und dabei die Basilarmembran 620 auslenkt. Die Nervenzellen, die näher bei der Basis 680 die Cochlea gelegen sind, werden früher angeregt, als Nervenzellen, die weiter von der Basis 680 der Cochlea entfernt sind. Mit anderen Worten, der Ort der Wanderwelle 670 als Funktion der Zeit kann als Trajektorie der Wanderwelle 670 angesehen werden. Die Trajektorie kann freilich auch auf diskrete Nervenzellen abgebildet werden, so dass eine Trajektorie ebenso beschreibt, in welcher zeitlichen Folge mehrere räumlich getrennte Nervenzellen durch eine Wanderwelle angeregt werden.

In dem gezeigten Beispiel wird beispielsweise die innere Haarzelle IHZ1 früher von der Wanderwelle 670 angeregt als die anderen inneren Haarzellen IHZ2, IHZ3, IHZn. Die erste innere Haarzelle IHZ1 liegt nämlich näher an dem ovalen Fenster 660, wobei sich die Wanderwelle 670 von dem ovalen Fenster (also von der Basis 680 der Cochlea) zu dem Apex 682 der Cochlea ausbreitet. Somit werden nacheinander die erste innere Haarzelle IHZ1, die zweite innere Haarzelle IHZ2, die dritte innere Haarzelle IHZ3 und die n-te innere Haarzelle IHZn angeregt. In anderen Worten, eine einzige Wanderwelle erzeugt an den gezeigten inneren Haarzellen Aktivitätsereignisse in einer zeitlichen Folge, wobei die zeitlichen Abstände durch die Ausbreitungsgeschwindigkeit der Wanderwelle 670 und die Lage der entsprechenden inneren Haarzellen IHZ1, IHZ2, IHZ3, IHZn bestimmt werden. Es ist allerdings festzuhalten, dass die Aktivitätsereignisse an den inneren Haarzellen (bei Betrachtung in einer zweidimensionalen Darstellung in Abhängigkeit von der Zeit und dem Index i der inneren Haarzellen IHZi eine Trajektorie bilden, die einer zeitlichen Trajektorie eines Orts maximaler Auslenkung der Wanderwelle 670 entspricht.

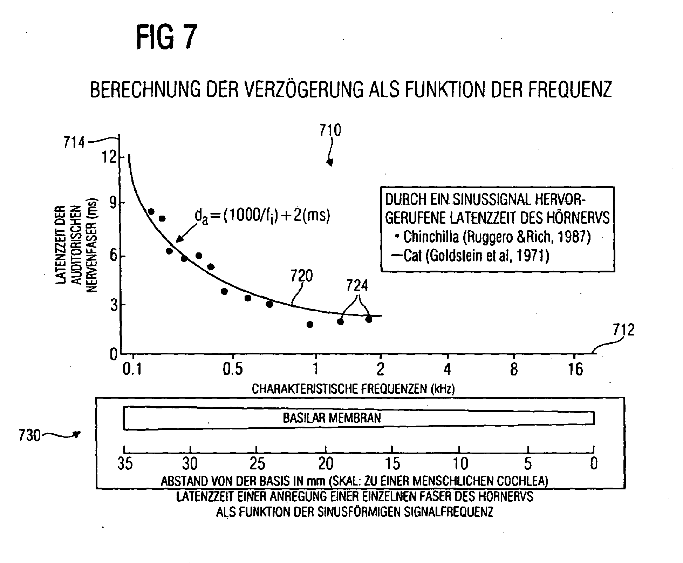

Zur weiteren Verdeutlichung zeigt die Fig. 7 eine graphische Darstellung einer Verzögerung bei einer Ausbeutung von Audiosignalen verschiedener Frequenz auf einer Basilarmembran. Eine erste graphische Darstellung 510 beschreibt eine Latenzzeit von auditorischen Nervenfasern als Funktion einer charakteristischen Frequenz. Eine Frequenz ist hierbei an einer Abszisse 712 in einem Bereich zwischen 0,1 kHz und 16 kHz angetragen. Eine Ordinate 714 beschreibt eine Latenzzeit der auditorischen Nervenfaser in einem Bereich zwischen 0 und 12 ms. Eine Kurve 720 beschreibt einen Verlauf einer durch ein Sinussignal hervorgerufenen Latenzzeit des Hörnervs über der Frequenz für eine Katze. Messpunkte 724 beschreiben einen ähnlichen Verlauf für einen Chinchilla. Es wurde herausgefunden, dass die Latenzzeit auf auditorischen Nervenfasern als Funktion einer charakteristischen Frequenz $f_i$ für die betreffende Nervenfaser durch die folgende Gleichung beschrieben werden kann:

$$b_a = (1000/f_i) + 2\ ms.$$

Der entsprechende Zusammenhang ist im Detail beispielsweise in dem Artikel "A Space-Time Theory of Pitch and Timbre Based on Cortical Expansion of the Cochlea Traveling Wave Delay" von S. Greenberg, D. Poeppel and T. Roberts, vorgestellt auf dem XI[th] Int. Symp. on Hearing, Grantham, ausführlich erläutert. Für Einzelheiten wird daher auf den entsprechenden Artikel verwiesen.

Es wird ferner darauf hingewiesen, dass eine zweite graphische Darstellung 730 einen Abstand von zu einer charakteristischen Frequenz gehörigen Haarzellen von einer Basis einer menschlichen Cochlea zeigt. Hieraus wird wiederum deutlich, dass zu hohen Frequenzen gehörige Haarzellen nahe bei der Basis der menschlichen Cochlea angeordnet sind, während zu niedrigen Frequenzen gehörige Haarzellen entfernt von der Basis der menschlichen Cochlea angeordnet sind. Die vorliegende Darstellung bestehend aus der ersten graphischen Darstellung 710 und der zweiten graphischen Darstellung 730 zeigt allerdings auch, dass eine Latenzzeit des auditorischen Nervs für tiefe Frequenzen (unter ca. 0, 5 kHz) stark ansteigt. Daraus kann gefolgert werden, dass eine Ausbreitungsgeschwindigkeit in der Nähe des Endes der Basilarmembran (also entfernt von der Basis der Cochlea) deutlich abnimmt. Die beschriebene Latenzzeit der auditorischen Nervenfasern resultiert in einer Krümmung von Trajectorien in Aktivitätsmustern an inneren Haarzellen, die an verschiedenen Orten der Cochlea angeordnet sind.

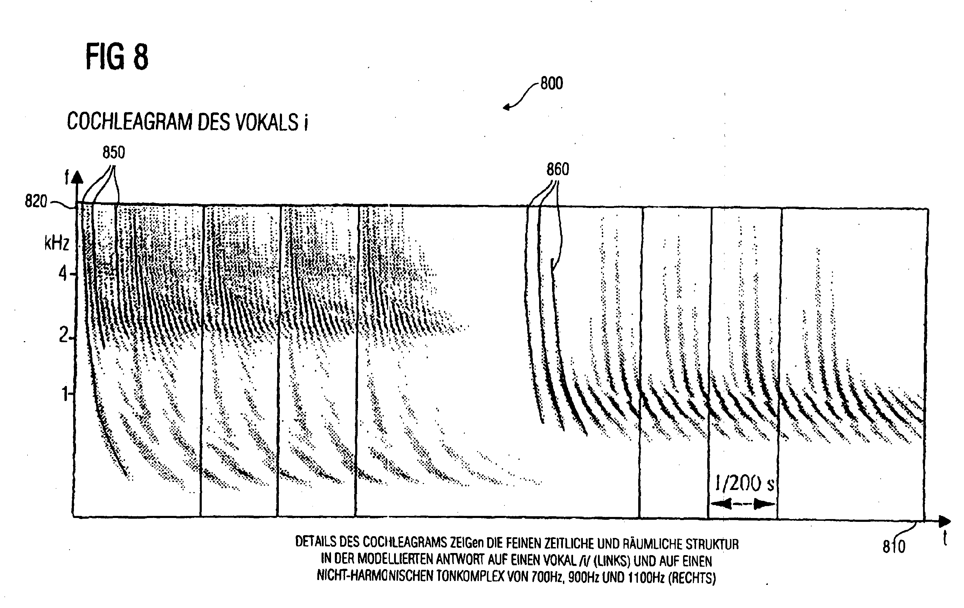

Zur weiteren Verdeutlichung zeigt die Fig. 8 eine graphische Darstellung eines Cochleogramms für einen Vokal "i" und für einen nicht-harmonischen Tonkomplex von 700 Hz, 900 Hz und 1100 Hz. Eine Abszisse 810 beschreibt hierbei die Zeit, während eine Ordinate 820 eine Frequenz beschreibt. Das Cochleogramm an sich beschreibt die Intensität einer Anregung von verschiedenen Regionen der Cochlea, die eine maximale Empfindlichkeit für verschiedene Frequenzen aufweisen. In anderen Worten, das Cochleogramm beschreibt, wie stark und zu welchen Zeitpunkten innere Haarzellen angeregt werden, die eine maximale Empfindlichkeit für verschiedene Frequenzen aufweisen. Mit anderen Worten, aus dem Cochleogramm kann gefolgert werden, wann Aktivitätsereignisse in verschiedenen Haarzellen auftreten. Zeigt das Cochleogramm beispielsweise eine Aktivität bei einer Frequenz von 1 kHz, so bedeutet dies, dass für diesen Zeitpunkt Aktivitätsereignisse in einer inneren Haarzelle auftreten, die eine maximale Empfindlichkeit bei einer entsprechenden Frequenz von beispielsweise 1 kHz aufweist. Das Auftreten der entsprechenden Aktivitätsereignisse in der Haarzelle mit der maximalen Empfindlichkeit bei 1 kHz ist in der graphischen Darstellung 800 beispielsweise mit einem "x" gekennzeichnet.

In dem Cochleogramm 800 des Vokals "i" sind deutlich Trajektorien erkennbar, von denen einige ausgewählte Trajektorien gekennzeichnet und mit 850 bezeichnet sind. In ähnlicher Weise zeigt auch das Cochleogramm des nicht-harmonischen Tonkomplexes Trajektorien, die mit 860 bezeichnet sind.

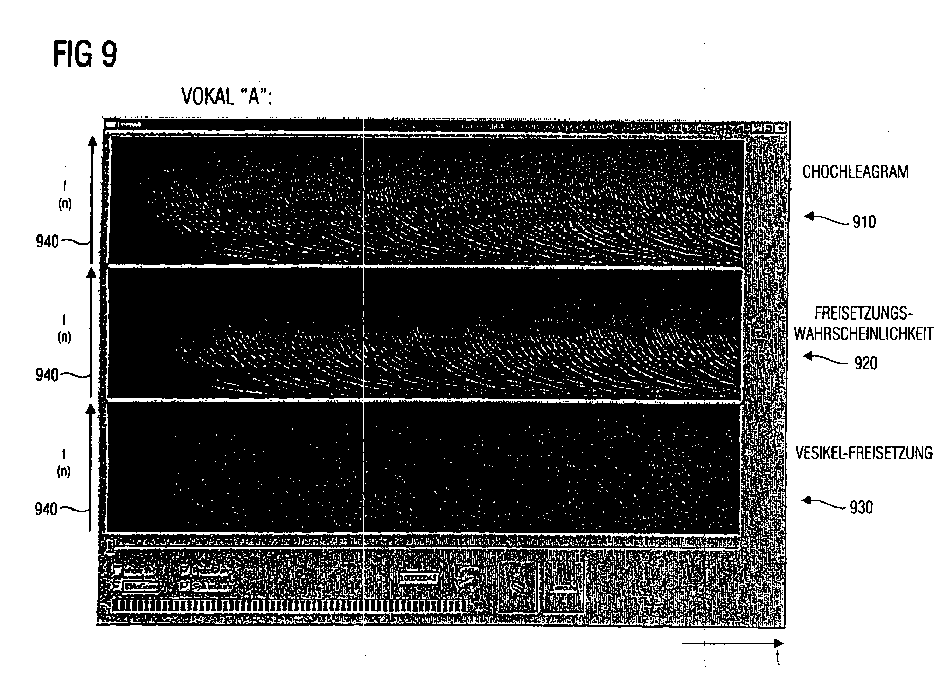

Fig. 9 zeigt eine weitere graphische Darstellung eines Cochleogramms sowie eine Transmitter-Freisetzungs-Wahrscheinlichkeit und eine Transmitter-Vesikel-Freisetzung für einen Vokal "A". Das Cochleogramm ist hierbei in einer ersten graphischen Darstellung 910 gezeigt. Eine zweite graphische Darstellung 920 beschreibt eine auf dem Cochleogramm basierende Transmitter-Freisetzungs-Wahrscheinlichkeit. Das Cochleogramm beschreibt eine Anregung der Basilarmembran über der Zeit und der Frequenz. bzw. eine Anregung von verschiedenen inneren Haarzellen, die eine maximale Empfindlichkeit für verschiedene Frequenzen aufweisen, in Abhängigkeit von der entsprechenden Frequenz der maximalen Empfindlichkeit. Basierend auf dem Cochleogramm kann, wie schon oben beschrieben, eine Freisetzungswahrscheinlichkeit für Neurotransmitter-Vesikel durch Analyse der mechanischen, chemischen und elektrischen Abläufe in einer inneren Haarzelle berechnet werden. Basierend auf der Freisetzungswahrscheinlichkeit k(t) kann ferner eine Neurotransmitter-Vesikel-Freisetzung bzw. ein Neurotransmitter-Vesikel-Auftreten durch eine stochastische Auswertung berechnet werden. Ein Beispiel für eine entsprechende Neurotransmitter-Vesikel-Freisetzung bzw. ein Neurotransmitter-Vesikel-Auftreten ist in der dritten graphischen Darstellung 930 gezeigt. Es ist hierbei ersichtlich, dass auch die Neurotransmitter-Vesikel-Freisetzung über Zeit und Frequenz charakteristische Trajektorien aufweist. Diese Trajektorien werden durch eine Modellierung des synaptischen Spalts auf Trajektorien von Aktionspotentialen (also Trajektorien in dem Nervenaktivitätsmuster) abgebildet. Die an den Ordinaten 940 angetragene Frequenz kann im Übrigen jeweils zugehörigen. Haarzellen bzw. Nervenfasern (mit einem Index i) zugeordnet werden. Somit treten die gezeigten Trajektorien in sehr ähnlicher Form auch in dem Nervenaktivitätsmuster auf.

Aus den obigen Ausführungen wird klar, dass an den Aktivitätsmustern der inneren Haarzellen deutliche Trajektorien auftreten. In anderen Worten, in einer Darstellung des Aktivitätsmusters über der Zeit treten linienförmige Verläufe auf, die als Trajektorien bezeichnet werden können. Eine Trajektorie ist also eine gerade oder gekrümmte Linie, die in einer zweidimensionalen Darstellung von Aktivitätsereignissen über der Zeit und an einer Mehrzahl von inneren Haarzellen mehrere Aktivitätsereignisse verbindet. Eine Trajektorie ist typischerweise eine Linie, die mehrere Aktivitätsereignisse in der genannten zweidimensionalen Darstellung verbindet und dabei die Richtung der Krümmung nicht ändert. Eine Trajektorie ist ferner typischerweise zumindest einmal stetig differenzierbar, weist also weder Unstetigkeiten noch Knicke auf. Bevorzugt ist eine Trajektorie sogar zweimal stetig differenzierbar, weist also eine konstante oder kontinuierlich veränderliche Krümmung auf.

Im übrigen weist eine Trajektorie bevorzugt zumindest näherungsweise die Form einer Hyperbel auf. Eine Trajektorie kann somit durch eine Suche nach einer hyperbelförmigen Kurve erkannt werden. Auch die anderen genannten Kriterien können für eine Identifizierung einer Trajektorie verwendet werden. Unter einer Hyperbelform ist dabei sowohl die Form einer Hyperbel in einem rechwinkligem als auch in einem schrägwinkligen zweidimensionalen Koordinatensystem zu verstehen.

Ferner kann das Erkennen des Vorliegens einer Trajektorie beispielsweise davon abhängig gemacht werden, dass eine Mindestanzahl an Aktivitätsereignisse auftreten, die, zumindest näherungsweise, auf einer wie oben definierten geraden oder gekrümmten Linie liegen (wenn sie im Rahmen einer zweidimensionalen Darstellung von Aktivitätsereignissen von mehreren inneren Haarzellen dargestellt sind). Dabei kann eine Toleranzgrenze vorgegeben sein und ausgewertet werden, die definiert, wie weit (beispielsweise im Sinne eines zeitlichen Abstands) ein Aktivitätsereignis von einer idealen Trajektorie bzw. Linie entfernt sein darf, um noch als zu der Trajektorie gehörig erkannt zu werden.

Hinsichtlich der Definition von (Verzögerungs-) Trajektorien wird im Übrigen auf die Veröffentlichung "A Space-Time Theory of Pitch and Timbre Based on Cortical Expansion of the Cochlea Traveling Wave Delay" (Ort-Zeit-Theorie von Tonhöhe und Klangfarbe basierend auf einer cortischen Ausbreitung der Verzögerung einer Wanderwelle auf der Cochlea) von S. Greenberg und anderen verweisen (Proceedings of the XI[th] Int. Symp. on Hearing, Grantham, 1997). Es hat sich nämlich gezeigt, dass die Bewegung der Basilarmembran in einer wohldefinierten Weise von der Basis (der Cochlea) zu einem Ort maximaler Auslenkung fortschreitet, hinter dem die Bewegung relativ schnell gedämpft wird. Der Durchgang der Wanderwelle ist an der Basis der Cochlea extrem schnell, verlangsamt sich aber dramatisch für Spitzen-Auslenkungen an dem Apex der Cochlea. Verzögerungstrajektorien der Anregung der Cochlea die sich in Trajektorien in dem Aktivitätsmuster wiederspiegeln, können effizient durch die folgende einfache Gleichung modelliert werden:

$$d_a = f_i^{-1} + k$$

Damit kann die Verzögerung $d_a$ auf der Cochlea unter Verwendung der gegebenen Frequenz $f_i$ und der Verzögerungskonstante k berechnet werden. Die genannte Gleichung beschreibt, dass die Verzögerungstrajektorien eine hyperbelartige Charakteristik aufweisen. In anderen Worten, eine Wanderwelle auf der Basilarmembran, die durch ein bestimmtes akustisches Ereignis (beispielsweise einen Klick) ausgelöst wird, verursacht zuerst Aktivitätsereignisse in inneren Haarzellen, die maximale Empfindlichkeit für hohe Frequenzen aufweisen, und erst später Aktivitätsereignisse in inneren Haarzellen, die maximale Empfindlichkeit für tiefere Frequenzen aufweisen. Eine Trajektorie kann somit allgemein auch als eine Linie aufgefasst werden, die in einer zweidimensionalen zeitlichen Darstellung beschriebene Aktivitätsereignisse an verschiedenen inneren Haarzellen verbindet, die sich aufgrund einer Wanderwelle auf der Basilarmembran oder eines breitbandigen Klicks in dem Audiosignal ergeben.

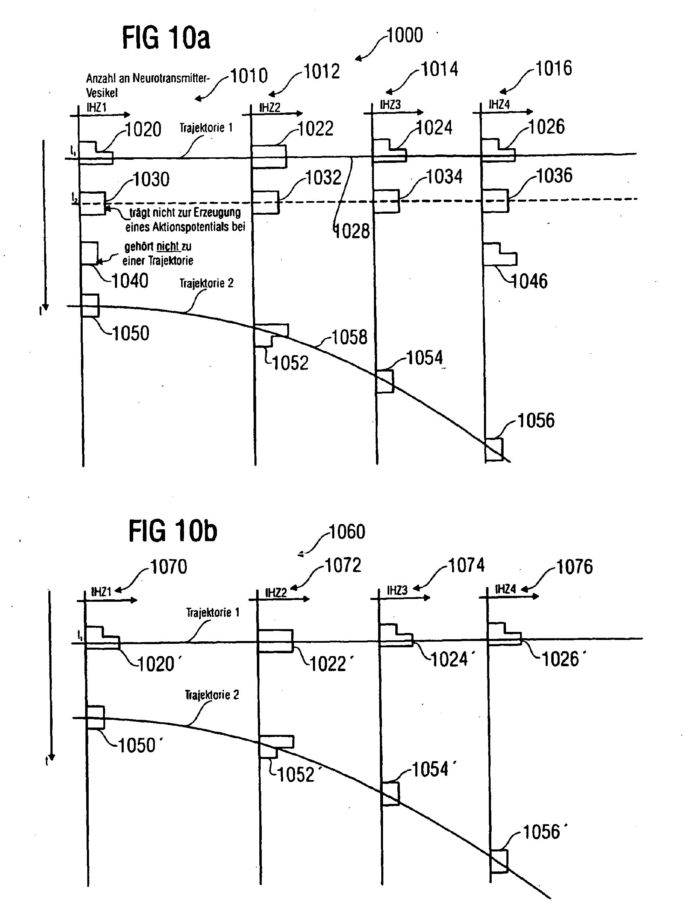

Zur weiteren Verdeutlichung des erfindungsgemäßen Konzepts zeigt die Fig. 10a eine grafische Darstellung eines Aktivitätsmusters, das ein Neurotransmitter-Vesikel-Auftreten an einer Mehrzahl von inneren Haarzellen über der Zeit beschreibt. Die grafische Darstellung der Fig. 10a ist in ihrer Gesamtheit mit 1000 bezeichnet. Die grafische Darstellung zeigt in vier zeitlichen Verläufen 1010, 1012, 1014, 1016 die Anzahl an Neurotransmitter-Vesikeln, die an jeweiligen synaptischen Spalten einer Mehrzahl von inneren Haarzellen auftreten. In anderen Worten, der zeitliche Verlauf 1010 beschreibt beispielsweise die Anzahl an Neurotransmitter-Vesikel, die an einer ersten inneren Haarzelle auftreten. Analog beschreibt die zweite grafische Darstellung 1012 die Anzahl an Neurotransmitter-Vesikeln, die an einer zweiten inneren Haarzelle auftreten. Die dritte grafische Darstellung 1014 zeigt die Anzahl der Neurotransmitter-Vesikeln, die an einer dritten inneren Haarzelle auftreten und der vierte zeitliche Verlauf 1016 zeigt die Anzahl an Neurotransmitter-Vesikeln die an einer vierten inneren Haarzelle auftreten. Es wird dabei davon ausgegangen, dass die erste innere Haarzelle IHZ1 eine maximale Empfindlichkeit für eine erste Frequenz aufweist, die zweite innere Haarzelle IHZ2 eine maximale Empfindlichkeit für eine zweite Frequenz aufweist, die dritte innere Haarzelle IHZ3 eine maximale Empfindlichkeit für eine dritte Frequenz aufweist und die vierte innere Haarzelle IHZ4 eine maximale Empfindlichkeit für eine vierte Frequenz aufweist, wobei die erste Frequenz größer als die zweite Frequenz ist, wobei die zweite Frequenz größer als die dritte Frequenz ist und wobei die dritte Frequenz größer als die vierte Frequenz ist. Die vier inneren Haarzellen IHZ1 bis IHZ4 können beispielsweise entweder in einer linearen Weise entlang der Cochlea eines (z.B. menschlichen) Gehörs oder einer logarithmischen Weise angeordnet sein. Die inneren Haarzellen können beispielsweise so angeordnet sein, dass das Verhältnis der ersten Frequenz und der zweiten Frequenz zumindest näherungsweise gleich dem Verhältnis der zweiten Frequenz zu der dritten Frequenz ist.

Die Anzahl an Neurotransmitter-Vesikeln die in den Zeitverläufen 1010, 1012, 1014, 1016 angetragen ist, kann beispielsweise die Anzahl an pro Zeiteinheit freigesetzten Neurotransmitter-Vesikeln beschreiben. Ebenso können die zeitlichen Verläufe 1010, 1012, 1014, 1016 aber auch die Zahl der insgesamt in dem präsynaptischen oder postsynaptischen Bereichen der Haarzelle in freigesetzter Form vorhandenen Neurotransmitter-Vesikeln beschreiben.

Aus den zeitlichen Verläufen 1010, 1012, 1014, 1016 ist ersichtlich, dass beispielsweise zu einem Zeitpunkt t1 Neurotransmitter-Vesikel 1020, 1022, 1024, 1026 und allen vier inneren Haarzellen IHZ1, IHZ2, IHZ3, IHZ4 auftreten. Somit können die vier Neurotransmitter-Vesikel-Auftreten in der Darstellung über der Zeit und der Mehrzahl von inneren Haarzellen durch eine gerade Linie 1028 verbunden werden. Die gerade Linie 1028 bildet somit eine erste Trajektorie. Zu einem zweiten Zeitpunkt t2, zeitlich verhältnismäßig kurz nach dem ersten Zeitpunkt t1, treten bei dem gezeigten Beispiel wiederum vier Neurotransmitter-Vesikel 1030, 1032, 1034, 1036 auf. Das Auftreten der Neurotransmitter-Vesikel 1030, 1032, 1034, 1036 ist jedoch so kurz nach dem Auftreten der Neurotransmitter-Vesikel 1020, 1022, 1024, 1026, dass sich die Synapsen der entsprechenden inneren Haarzellen IHZ1, IHZ2, IHZ3, IHZ4 bzw. die entsprechenden Hörnerven-Fasern zu dem Zeitpunkt t2 noch in einer Refraktärzeit befinden. Im Anschluss an die Auslösung eines Aktionspotenzials aufgrund eines Neurotransmitter-Vesikel-Auftretens ist die Synapse bzw. die mit der Synapse gekoppelte Nervenfaser nämlich nicht in der Lage, einen weiteren Aktivitätsimpuls auszugeben, selbst wenn Neurotransmitter-Vesikel vorliegen. Die grafische Darstellung 1000 der Fig. 10a zeigt ferner ein weiteres Neurotransmitter-Vesikel-Auftreten an der ersten inneren Haarzelle IHZ1, das mit 1040 bezeichnet ist, sowie ein weiteres Neurotransmitter-Vesikel-Auftreten 1046 an der vierten inneren Haarzelle IHZ4. Die Neurotransmitter-Vesikel-Auftreten 1040, 1046 gehören jedoch nicht zu einer Trajektorie. Es treten nämlich beispielsweise an der zweiten inneren Harrzelle IHZ2 und an der dritten inneren Haarzelle IHZ3 keine Neurotransmitter-Vesikel auf, die in der grafischen Darstellung 1000 auf einer geraden oder gekrümmten Verbindungslinie der Neurotransmitter-Vesikel-Auftreten 1040, 1046, die eine gültige Trajektorie bildet, liegen.

Die Darstellung 1000 zeigt ferner weitere Neurotransmitter-Vesikel-Auftreten 1050, 1052, 1054, 1056, die in der zweidimensionalen grafischen Darstellung 1000 durch eine zeitlich monoton verlaufende Verbindungslinie 1058 verbunden werden können. In anderen Worten, die Neurotransmitter-Vesikel-Auftreten 1050, 1052, 1054, 1056 liegen, wie auf der grafischen Darstellung 1000 unschwer zu erkennen ist, auf einer Trajektorie 1058. Das Vorliegen einer Trajektorie kann beispielsweise daran erkannt werden, dass die Zeitpunkte, zu denen die Neurotransmitter-Vesikel-Auftreten 1050, 1052, 1054, 1056 stattfinden, in monotoner Weise mit der Lage der vier inneren Haarzellen IHZ1, IHZ2, IHZ3, IHZ4 entlang der Cochlea korrespondieren. In anderen Worten, je weiter eine der betrachteten inneren Haarzellen IHZ1, IHZ2, IHZ3, IHZ4 bei dem Ende der Cochlea befindlich ist, desto später (zeitlich betrachtet) findet das zugehörige Neurotransmitter-Vesikel-Auftreten 1050, 1052, 1054, 1056 statt. Die Verbindungslinie der Neurotransmitter-Vesikel-Auftreten 1050, 1052, 1054, 1056 weist ferner eine einheitliche Krümmung auf, wechselt also die Krümmungsrichtung nicht. Ferner kann das Vorliegen einer Trajektorie auch daran erkannt werden, dass die Verbindungslinie 1058 zumindest näherungsweise eine hyperbelartige Form aufweist.

Fig. 10b zeigt ferner eine grafische Darstellung eines bereinigten Aktivitätsmusters, das ein bereinigtes Neurotransmitter-Vesikel-Auftreten an einer Mehrzahl von inneren Haarzellen über der Zeit beschreibt. Die grafische Darstellung der Fig. 10b ist in ihrer Gesamtheit mit 1060 bezeichnet. Die graphische Darstellung 1060 zeigt bereinigte Zeitverläufe 1070, 1072, 1074, 1076 an den selben vier inneren Haarzellen IHZ1, IHZ2, IHZ3, IHZ4, für die die grafische Darstellung 1000 unbereinigte Zeitverläufe zeigt. Aus der grafischen Darstellung 1060 ist ersichtlich, dass in die bereinigten Zeitverläufe 1070, 1072, 1074, 1076 diejenigen Neurotransmitter-Vesikel-Auftreten übernommen werden, die zu Trajektorien 1028, 1058 gehören. Die übernommenen Neurotransmitter-Vesikel-Auftreten sind in der grafischen Darstellung 1060 mit den gleichen Bezugszeichen bezeichnet wie die ursprünglichen Neurotransmitter-Vesikel-Auftreten in der grafischen Darstellung 1000, wobei den Bezugszeichen der übernommenen Neurotransmitter-Vesikel-Auftreten in der grafischen Darstellung 1060 ein "'" hinzugefügt ist.

In anderen Worten, in das bereinigte Aktivitätsmuster, das in der grafischen Darstellung 1060 gezeigt ist, sind diejenigen Neurotransmitter-Vesikel-Auftreten übernommen, die zu einer Trajektorie gehören. Andersherum kann ebenso festgestellt werden, dass aus dem bereinigten Aktivitätsmuster der grafischen Darstellung 1060 diejenigen Neurotransmitter-Vesikel-Auftreten entfernt sind, die nicht zur Erzeugung eines Aktionspotential beitreten (Neurotransmitter-Vesikel-Auftreten 1030, 1032, 1034, 1036), oder die nicht zu einer Trajektorie gehören (Neurotransmitter-Vesikel-Auftreten 1040, 1046). Ferner ist festzuhalten, dass beispielsweise die Neurotransmitter-Vesikel-Auftreten 1030, 1032, 1034, 1036, die nicht zur Anregung eines Aktionspotenzials beitragen, folglich auch nicht zu einer Sprachverständlichkeit beitragen. Ferner tragen in manchen Fällen Neurotransmitter-Vesikel-Auftreten 1040, 1046, die nicht zu einer Trajektorie gehören, nicht bzw. nicht wesentlich zu einer Sprachverständlichkeit beispielsweise von Vokalen bei. Es wurde nämlich herausgefunden, dass die Identifizierung von Vokalen durch eine Auswertung der entsprechenden Trajektorien (beispielsweise 1028, 1058) erfolgen kann.

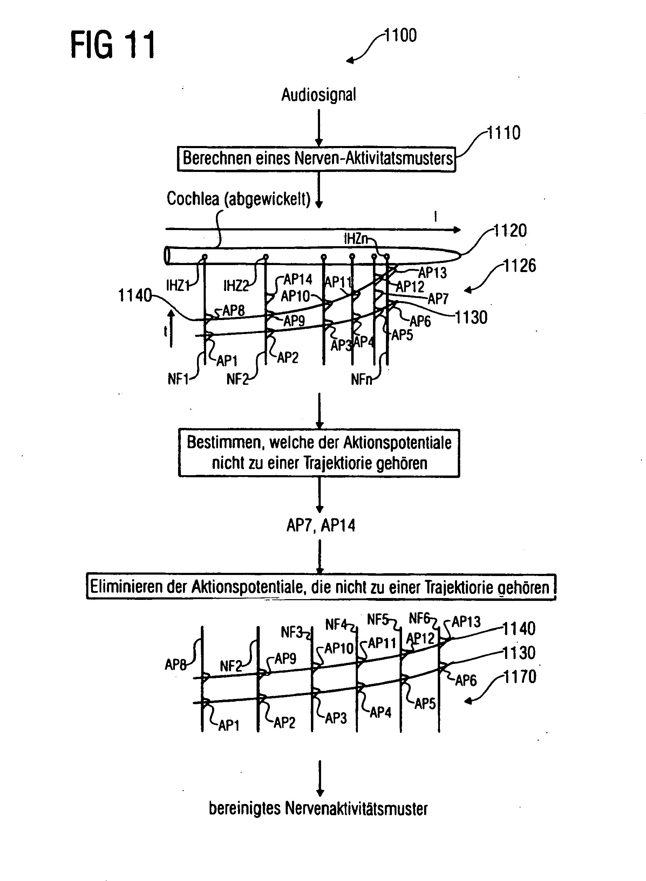

Fig. 11 zeigt ein Flussdiagramm eines Verfahrens zum Ableiten eines bereinigten Nervenaktivitätsmusters aus einem Audiosignal. Das Verfahren der Fig. 11 ist in seiner Gesamtheit mit 1100 bezeichnet. Das Verfahren umfasst einen ersten Schritt 1110, in dem basierend auf dem Audiosignal ein Nervenaktivitätsmuster berechnet wird. Das Aktivitätsmuster beschreibt eine Aktivität auf einer Mehrzahl von Nervenfasern als Funktion der Zeit. In anderen Worten, das Nervenaktivitätsmuster beschreibt mehrere zeitliche Verläufe von Aktionspotentialen an einer Mehrzahl von Hörnerven-Fasern eines Gehörmodells. Alternativ kann das Nervenaktivitätsmuster auch einen zeitlichen Verlauf einer Auslösung von Aktionspotentialen an einer Mehrzahl von synaptischen Spalten, die zu einer Mehrzahl von inneren Haarzellen gehören, beschreiben. Das Flussdiagramm zeigt zur Verdeutlichung eine abgewickelte Cochlea 1120 eines menschlichen Gehörmodels zusammen mit einer Mehrzahl von inneren Haarzellen IHZ1 bis IHZn, die entlang der Cochlea 1120 angeordnet sind, und die maximale Empfindlichkeiten für verschiedene Frequenzen aufweisen. Die inneren Haarzellen IHZ1 bis IHZn sind dabei mit zugehörigen Nervenfasern NF1, NF2 bis NFn gekoppelt und liefern an die zugehörigen Nervenfasern NF1, NF2 bis NFn entsprechende Nervenaktivitätsimpulse bzw. Aktionspotenziale. Die Aktionspotenziale werden durch die Freisetzung von Neurotransmitter-Vesikeln in einen synaptischen Spalt ausgelöst, über den eine jeweilige innere Haarzelle IHZi mit einer zugehörigen Nervenfaser NFi gekoppelt ist. Es ist ersichtlich, dass sechs durch die Cochlea 1020 erzeugte Aktionspotenziale AP1, AP2, AP3, AP4, AP5, AP6 auf einer ersten Trajektorie 1130 liegen. Weitere sechs Aktionspotenziale AP8, AP9, AP10, AP11, AP12, AP13 liegen auf einer zweiten Trajektorie 1140. In dem gezeigten Nervenaktivitätsmuster das die Aktionspotenziale auf der Mehrzahl von inneren Haarzellen IHZ1 bis IHZn über der Zeit beschreibt, treten allerdings auch zwei Aktionspotenziale AP7, AP14 auf, die nicht zu einer Trajektorie gehören. Für die Definition der Trajektorien wird im Übrigen auf die obigen Ausführungen verwiesen. In anderen Worten, eine Trajektorie in dem Nervenaktivitätsmuster ist ebenso definiert wie eine Trajektorie bei einer zweidimensionalen grafischen Darstellung des Neurotransmitter-Vesikel-Auftretens, wobei lediglich an die Stelle der einzelnen Neurotransmitter-Vesikel-Auftreten die entsprechenden Aktionspotenziale treten.

Das in Flussdiagramm 1100 gezeigte Verfahren umfasst ferner einen zweiten Schritt 1150 des Bestimmens, welche der durch das Nervenaktivitätsmuster beschriebenen Aktionspotenziale nicht zu einer Trajektorie 1130, 1140 gehören. Das Bestimmen 1150 liefert bei dem gezeigten Beispiel als Ergebnis die Aktionspotenziale AP7 und AP14.

Das Verfahren 1100 umfasst ferner einen dritten Schritt 1160, in dem Aktionspotenziale, die nicht zu einer Trajektorie gehören, in dem Nervenaktivitätsmuster eliminiert werden. Dabei kann entweder die ursprüngliche Darstellung des Nervenaktivitätsmuster durch Löschen bzw. Unterdrücken der nicht zu einer Trajektorie gehörigen Aktionspotenziale aufbereitet werden, oder es kann eine Kopie des ursprünglichen, nicht bereinigten Nervenaktivitätsmusters erstellt werden, in die nur Aktionspotenziale übernommen werden, die zu einer Trajektorie 1130, 1140 gehören. Somit entsteht ein bereinigtes Nervenaktivitätsmuster 1170 das beispielshalber die nicht zu Trajektorien gehörigen Aktionspotenziale AP7, AP14 nicht mehr umfasst bzw. beschreibt, ansonsten aber mit dem ursprünglichen, nicht bereinigten Nervenaktivitätsmuster 1126 übereinstimmt.

Es zeigt sich somit, dass das Verfahren auf verschiedene Arten von Aktivitätsereignissen anwendbar ist, die in einem menschlichen oder tierischen Gehör auftreten, beispielsweise auf Neurotransmitter-Vesikel-Auftreten oder auf Aktionspotenziale. In diesem Fall beschreibt das Aktivitätsmuster also einen zeitlichen Verlauf der entsprechenden Größe.

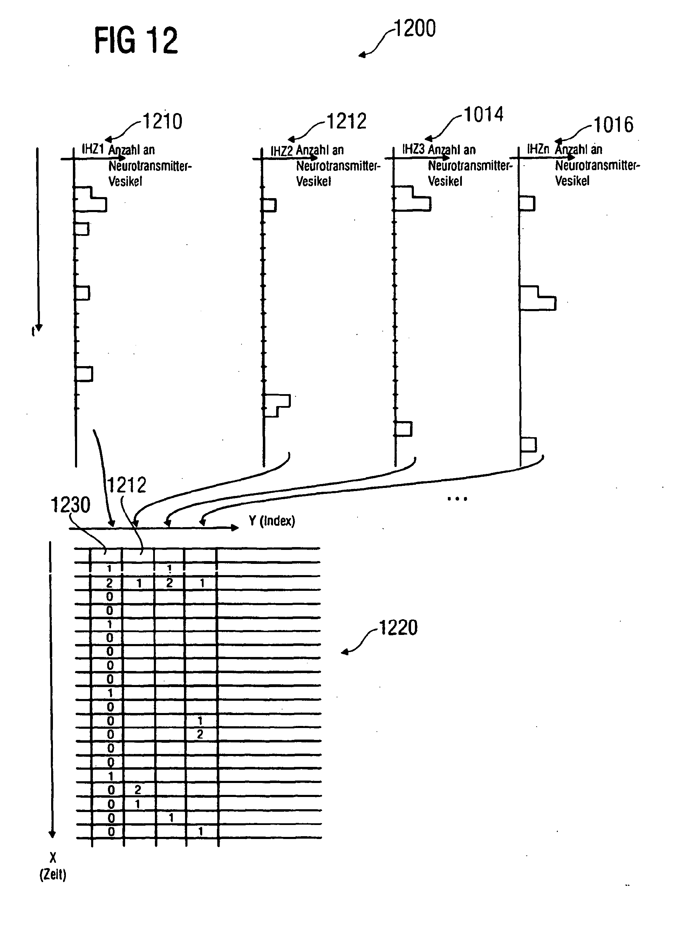

Fig. 12 zeigt eine schematische Darstellung einer Vorgehensweise zur Erzeugung einer zweidimensionalen Darstellung aus einem Aktivitätsmuster über der Zeit an einer Mehrzahl von inneren Haarzellen eines Gehörmodells. Die schematische Darstellung der Fig. 12 ist in ihrer Gesamtheit mit 1200 bezeichnet. Bei der beispielhaften Vorgehensweise werden zeitliche Verläufe 1210, 1212, 1214, 1216 empfangen und in eine zweidimensionale, beispielsweise tabellarische Darstellung 1220 umgewandelt. Die Zeitverläufe 1210, 1212, 1214, 1216 beschreiben dabei beispielsweise die jeweilige Anzahl von Neurotransmitter-Vesikeln an mehreren inneren Haarzellen, z.B. IHZn, analog wie dies beispielsweise anhand der Figuren 10a und 10b gezeigt wurde. Die Zeitverläufe 1210, 1212, 1214, 1216 können beispielsweise zeitdiskretisiert sein. Somit beschreibt die tabellarische Darstellung 1220 die Anzahl an Neurotransmitter-Vesikeln, die in dem durch die entsprechende Zeile beschriebenen Zeitintervall in den synaptischen Spalt freigesetzt werden. Analog beschreibt beispielsweise die zweite Spalte 1232 der Tabelle 1220 den zweiten Zeitverlauf 1212.

Somit bildet beispielsweise die Tabelle 1220 eine zweidimensionale Darstellung der Aktivitätsereignisse (beispielsweise der Anzahl an Neurotransmitter-Vesikeln) über der Zeit einer Mehrzahl von inneren Haarzellen.

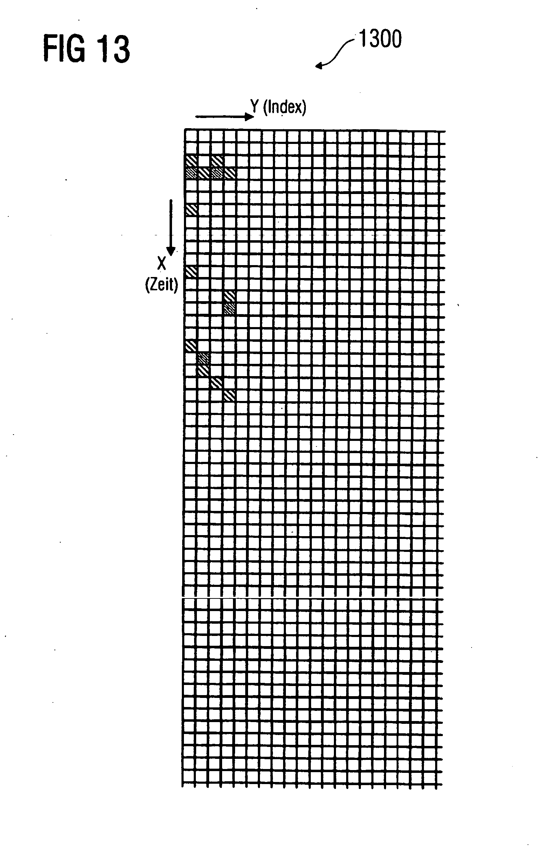

Fig. 13 zeigt zur besseren Veranschaulichung eine weitere zweidimensionale graphische Darstellung eines Aktivitätsmusters über der Zeit an einer Mehrzahl von inneren Haarzellen eines Gehörmodells. Die grafische Darstellung der Fig. 13 ist in ihrer Gesamtheit als 1300 bezeichnet. Anhand der grafischen Darstellung 1300 der Fig. 13 ist ersichtlich, wie beispielsweise die Tabelle 1220 gemäß Fig. 12 in eine Darstellung von Bildpunkten umgewandelt werden kann, bzw. inwiefern die Zeitverläufe 1210, 1212, 1214, 1216, die Anzahlen von Neurotransmitter-Vesikeln an einer Mehrzahl von Haarzellen beschreiben, als ein zweidimensionales Muster aufgefasst werden können.

So kann beispielsweise die Tabelle 1120 als eine Beschreibung eines Musters von Bildpunkten aufgefasst werden, wobei eine erste Dimension der Tabelle (beispielsweise eine x-Richtung) einer ersten Richtung des zweidimensionalen Musters 1300 entspricht, und wobei eine zweite Dimension der Tabelle einer zweiten Richtung, z.B. einer y-Richtung, entspricht. Mit anderen Worten, die erste Richtung, also die x-Richtung in der zweidimensionalen Darstellung, kann beispielsweise der Zeit entsprechen, und die zweite Richtung, also die y-Richtung in der grafischen Darstellung 1300, kann beispielsweise einen Index der inneren Haarzellen beschreiben. Somit kann das Aktivitätsmuster über der Zeit, das die Aktivitätsereignisse an der Mehrzahl von inneren Hörzellen über der Zeit beschreibt als eine zweidimensionale Darstellung bzw. ein zweidimensionales Bildmuster 1300 aufgefasst werden, dem mit fortschreitender Zeit in der ersten Richtung (x-Richtung) immer neue Informationen hinzugefügt werden. Aufgrund der anhand von Fig. 13 beschriebenen Interpretation des Aktivitätsmusters über der Zeit als eine zweidimensionale Grafik ist verständlich, dass auf das Aktivitätsmuster über der Zeit Methoden der Mustererkennung angewendet werden können, um die oben beschrieben Trajektorien zu erkennen.

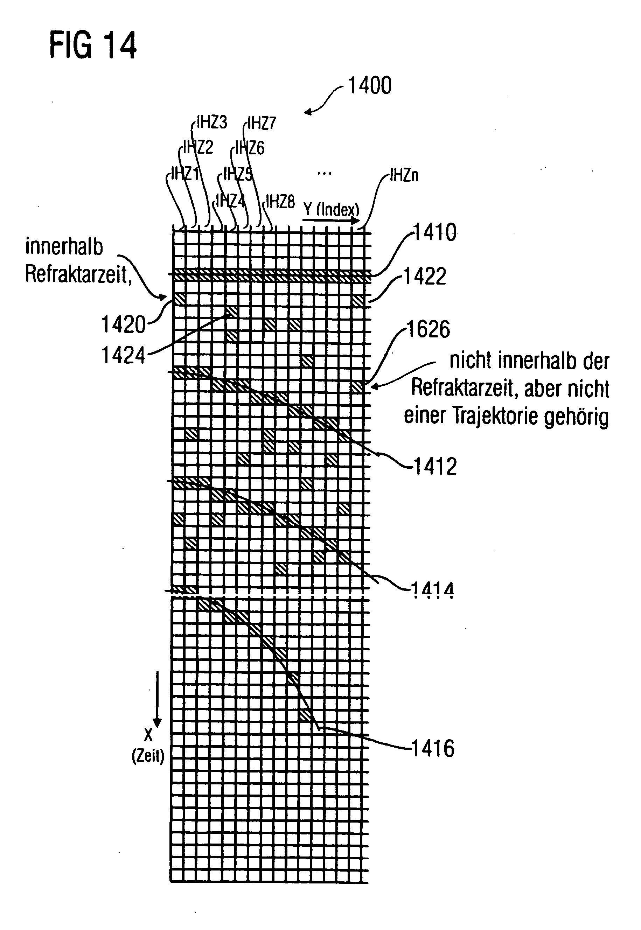

Fig. 13 zeigt zur weiteren Veranschaulichung eine dritte grafische Darstellung eines Aktivitätsmusters über der Zeit an einer Mehrzahl von inneren Haarzellen eines Gehörmodells, wobei das Aktivitätsmuster über der Zeit wiederum als ein zweidimensionales Muster von Bildpunkten dargestellt ist. Das zweidimensionale Muster der Fig. 14 ist in seiner Gesamtheit mit 1400 bezeichnet. Bildspalten des zweidimensionalen Bildmusters 1400 sind den Aktivitätsereignissen an verschiedenen inneren Haarzellen IHZ1 bis IHZn zugeordnet. Die Zeilen des Bildmusters 1400 sind ferner Zeitpunkten bzw. Zeitintervallen zugeordnet. In der grafischen zweidimensionalen Darstellung 1400 der Fig. 14 sind somit gerade sowie gebogene Liniensegmente 1410, 1412, 1414, 1416 ersichtlich, die jeweils eine Trajektorie in dem Aktivitätsmuster beschreiben. Die grafische Darstellung 1400 zeigt ferner weitere einzelne Aktivitätsereignisse, (z.B. Aktivitätsereignisse 1420, 1422, 1424, 1426), die nicht zur Trajektorie gehören oder die innerhalb einer Refraktärzeit der zugehörigen Synapse oder der zugehörigen Nervenfaser auftreten. Beispielsweise folgt das Aktivitätsereignis 1420 kurze Zeit nach dem Auftreten eines Aktivitätsereignisses auf der ersten inneren Haarzelle IHZ1 im Rahmen der ersten Trajektorie 1410. Somit befindet sich die Synapse der mit der ersten inneren Haarzelle IHZ1 gekoppelten Nervenfaser NF1 innerhalb einer Totzeit, wenn das Aktivitätsereignis 1420 auftritt. Somit kann im Rahmen einer Mustererkennung erkannt werden, dass beispielsweise das Aktivitätsereignis 1420 für eine Sprachverständlichkeit nicht relevant ist, da es in einem menschlichen Gehör erst gar nicht in einer Auslösung eines Aktivitätspotenzials auf einer Nervenfaser resultieren würde. Ähnliches gilt für das Aktivitätsereignis 1422.

Ferner können Aktivitätsereignisse identifiziert werden, die nicht zu einer Trajektorie gehören, die also nicht zu einem linienförmigen Verlauf in der grafischen Darstellung 400 gehören. Dies trifft beispielsweise für das Aktivitätsereignis 1426 zu. Somit kann im Rahmen der Mustererkennung die auf das Muster der grafischen Darstellung 1410 angewendet wird, erkannt werden, dass das nicht zu der Trajektorie gehörige Aktivitätsereignis 1426 beispielsweise nicht für Sprachverständigkeit zumindest eines Lauts relevant ist.

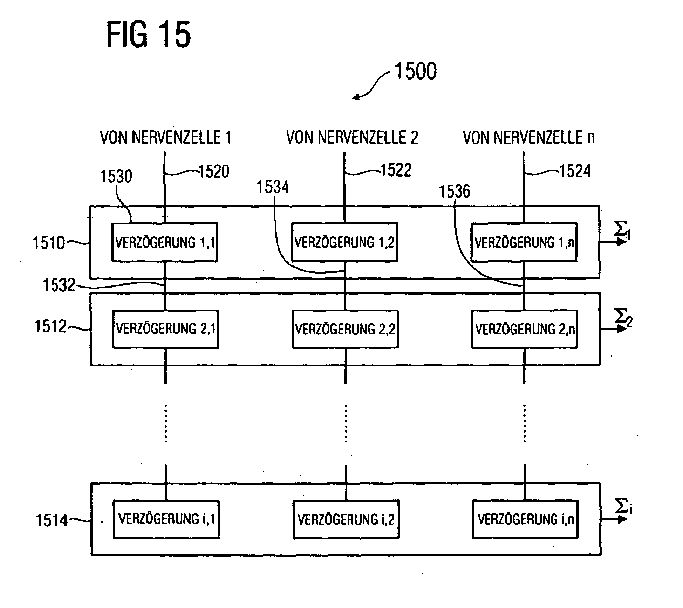

Figur 15 zeigt ein Blockschaltbild einer Vorrichtung zum erfindungsgemäßen Verarbeiten des Nervenaktivitätsmusters. Die in der Figur 15 gezeigten Vorrichtung ist in ihrer Gesamtheit mit 1500 bezeichnet. Die gezeigte Vorrichtung 1300 weist eine Mehrzahl von Stufen 1510, 1512, 1514 auf, wobei die erste Stufe 1510 parallel Signal 1520, 1522, 1524 von Nervenzellen empfängt. Die Signale 1520, 1522, 1524 beschreiben bevorzugt Aktionspotentiale auf Nervenfasern, die mit den entsprechenden Nervenzellen bzw. inneren Haarzellen gekoppelt sind, und beschreiben somit das Nervenaktivitätsmuster. Die Signale können aber auch ein anderes Aktivitätsmuster, beispielsweise ein Neurotransmitter-Vesikel-Auftreten in einer Mehrzahl von inneren Haarzellen beschreiben.

In einer ersten Stufe 1510 wird dann beispielsweise das erste Nervensignal 1520 in einer ersten Verzögerungseinrichtung 1530 einer Verzögerung unterworfen und dann als verzögertes Nervensignal 1532 an eine zweite Stufe 1512 weitergeleitet. In ähnlicher weise wird auch das zweite Nervensignal 1522 in der ersten Stufe 1510 verzögert und als verzögertes Nervensignal an die zweite Stufe 1512 weitergeleitet. In der gleichen Weise werden auch die übrigen Nervensignale in der ersten Stufe 1510 verarbeitet (also beispielsweise auch das n-te Nervensignal 1524).

Die zweite Stufe 1512 ist parallel zu der ersten Stufe 1510 ausgelegt, ermöglicht also wiederum die verzögerte Weiterleitung der verzögerten Nervensignale 1532, 1534, 1536, wodurch zweimal verzögerte Nervensignale entstehen. Eine Vorrichtung zur erfindungsgemäßen Verarbeitung des Nervenaktivitätsmusters umfasst eine Mehrzahl von hintereinander geschalteten Stufen, die gleich wie die erste Stufe 1510 bzw. die zweite Stufe 1512 aufgebaut sind. Die Nervensignale 1520, 1522, 1524 werden also parallel durch die Mehrzahl von Stufen 1510, 1512, 1514 weitergeleitet, wobei jede Stufe eine einstellbare Verzögerung zu den Nervensignalen hinzufügt.

Weiterhin ist jede der Stufen 1510, 1512, 1514 ausgelegt, um eine Summe der an ihr einlaufenden bzw. der aus ihr auslaufenden Nervensignale (bzw. m-mal verzögerten Nervensignale) zu bilden. Ferner sind die Stufen 1510, 1512, 1514 bevorzugt ausgelegt, um diese Summe mit einem einstellbaren Schwellwert zu vergleichen, um festzustellen, ob zu einem gegebenem Zeitpunkt mindestens eine vorgegebene Anzahl von Nervensignalen bzw. verzögerten Nervensignalen (also einlaufende Nervensignale oder auslaufende Nervensignale) aktiv sind (bzw. ein Aktionspotential aufweisen).

Es wird ferner bevorzugt, dass die Verzögerungen der in den Stufen 1510, 1512, 1514 vorhandenen Verzögerungseinrichtungen unterschiedlich eingestellt sind, sodass beispielsweise ein erstes Nervensignal 1520 bei einem Durchlaufen der Stufen 1510, 1512, 1514 einer anderen Verzögerung unterliegt als das zweite Nervensignal 1522. Verzögerungen können beispielsweise so eingestellt sein, dass sich für die Nervensignale 1520, 1522, 1524 unterschiedliche Gesamtverzögerungen beim Durchlaufen durch die Stufen 1510, 1512, 1514 ergeben (wobei es freilich zulässig ist, dass beispielsweise zwei Nervensignale in der gleichen Weise verzögert werden). In anderen Worten, die Einrichtung 1500 ist bevorzugt so ausgelegt, das sich nicht für alle Nervensignale die gleichen Verzögerungen ergeben. Außerdem ist es vorteilhaft, dass bei Vorhandensein von j Stufen 1510, 1512, 1514 mindestens (j-1) Stufen 1510, 1512 so ausgelegt sind, dass die in einer Stufe enthaltenen Verzö

gerungseinrichtungen für die Mehrzahl von Nervensignalen nicht alle die gleiche Verzögerung aufweisen. Dadurch kann erreicht werden, dass ein in eine erfindungsgemäße Einrichtung 1500 einlaufendes Nervenaktivitätsmuster über der Zeit beim Durchlauf durch die beschriebene Einrichtung zeitlich so verzerrt wird, dass einzelne Nervensignale gegenüber anderen Nervenssignalen zeitlich verschoben werden. Durch die Verzerrung können in einer zeitlichen Darstellung gebogene linienartige Muster, also Trajektorien, in dem Nervenaktivitätsmuster gerade gebogen werden.

Ferner wird darauf hingewiesen, dass durch die Summenbildung innerhalb einer Stufe erkannt werden kann, wenn eine ursprünglich gebogene Trajektorie in dem Nervenaktivitätsmuster zu einer geraden Linie verbogen wurde (wobei eine geradegebogene Linie dadurch beschrieben bzw. erkannt wird, dass eine vorgegebene Anzahl der verzögerten Nervensignale nahezu gleichzeitig bzw. zeitlich überlappend ein Aktionspotential aufweisen).

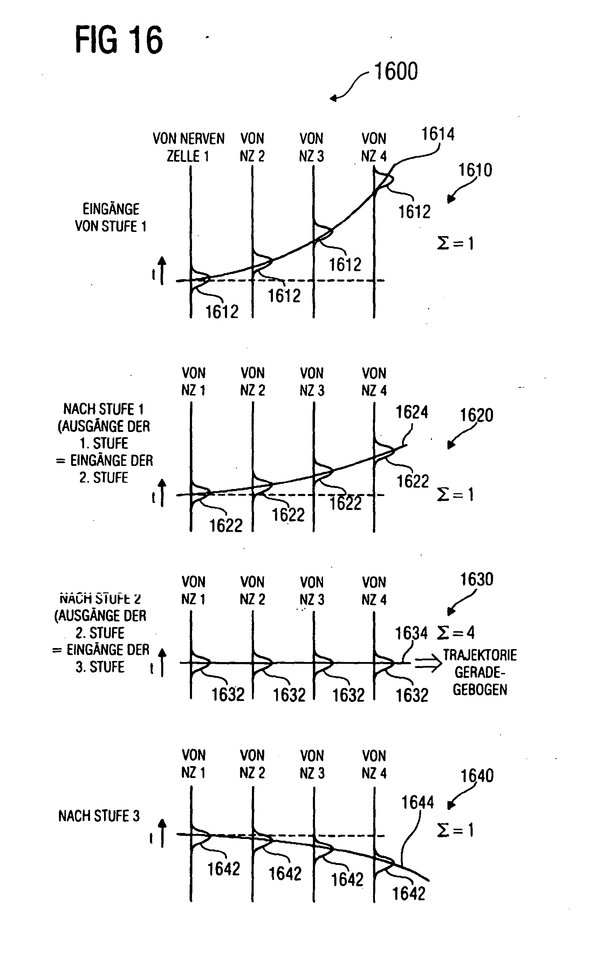

Die Funktionsweise der Einrichtung 1500 soll anhand der Figur 16 veranschaulicht werden. Figur 16 zeigt eine beispielhafte grafische Darstellung der Signale in einer Vorrichtung 1500 zum erfindungsgemäßen Verarbeiten des Nervenaktivitätsmusters. Die grafische Darstellung der Figur 16 ist in ihrer Gesamtheit mit 1600 bezeichnet.

Eine erste graphische Darstellung 1610 beschreibt hierbei ein beispielhaftes Nervenaktivitätsmuster an Eingängen der Vorrichtung 1500. Gezeigt sind beispielhaft die Signale von vier Nervenzellen (bzw. auf vier Nervenfasern) in einem zeitlichen Verlauf. Im übrigen wird darauf hingewiesen, dass die Aktionspotentiale 1612 eine Trajektorie 1614 bilden. Wie gezeigt, weist die Trajektorie 1614 in der zeitlichen Darstellung eine starke Krümmung auf, da die Aktionspotentiale 1612 von den verschiedenen Nervenfasern an den Eingängen der ersten Stufe 1510 einen deutlichen zeitlichen Versatz aufweisen. Somit liegt in der ersten Stufe 1510 zu einem festen Zeitpunkt nur jeweils ein Aktionspotential vor, sodass ein Schwellwert für eine Summe der an der ersten Stufe anliegenden Aktionspotentiale, der beispielsweise zu zwei gesetzt ist, nicht überschritten wird. Folglich liefert die erste Stufe kein Ausgangssignal an einem Schwellwertausgang.

Eine zweite grafische Darstellung 1620 beschreibt die Verhältnisse an einem Ausgang der ersten Stufe 1510. Hierbei wird davon ausgegangen, dass in der ersten Stufe 1510 das von der erste Nervenzelle NZ 1 gelieferte Nervensignal stärker verzögert wird als die von den anderen Stufen gelieferten Nervensignale. Im übrigen wird davon ausgegangen, dass bei dem gegebenem Beispiel das von der vierten Nervenzelle NZ4 gelieferte Nervensignal am wenigsten verzögert wird, während das Nervensignal von der dritten Nervenzelle NZ3 etwas mehr verzögert wird, und wobei die Verzögerung für Nervensignalen von den Nervenzellen NZ2 und NZ1 immer stärker ansteigt. Allgemein gesprochen werden Signale, die zu Nervenzellen gehören, die auf eine niedrigere Frequenz ansprechen, weniger stark verzögert als Nervensignale von Nervenzellen, die höhere Frequenzen detektieren.

Die zweite grafische Darstellung zeigt somit wiederum Aktionspotentiale 1624 als Funktion der Zeit, wobei die Aktionspotentiale 1622 eine Trajektorie 1624 bilden. Wie aus der zweiten grafischen Darstellung 1620 ersichtlich ist, ist die Krümmung der Trajektorie 1624 an den Ausgängen der ersten Stufe geringer als eine (zeitlich-örtliche bzw. zeitlich-frequenzmäßige) Krümmung der Trajektorie 1616 an den Eingängen der ersten Stufe. Dies resultiert aus der unterschiedlichen Verzögerung der zu verschiedenen Nervenzellen gehörigen Nervensignale in den Verzögerungseinrichtungen (z.B. 1530) der ersten Stufe. Dadurch wird also eine gekrümmte Trajektorie gleichsam geradegebogen. Wie aus der zweiten grafischen Darstellung 1620 ersichtlich, weist die zweite Trajektorie 1624 allerdings noch eine Restkrümmung auf, so dass die von verschiedenen Nervenzellen bzw. Nervenfasern stammenden Aktionspotentiale 1622 nicht alle gleichzeitig an den Ausgängen der ersten Stufe 1510 bzw. Eingängen der zweiten Stufe 1512 anliegen.

Auch die zweite Stufe 1512 bewirkt eine weitere Verzögerung, wobei wiederum Signale von Nervenzellen, die für niedrige Frequenzen empfindlich sind, weniger verzögert werden als Signale von Nervenzellen, die für höhere Frequenzen empfindlich sind. Eine dritte grafische Darstellung 1630 zeigt die in der zweiten Stufe 1512 nochmals verzögerten Nervensignale an Ausgängen der zweiten Stufe. Es ist aus der dritten graphischen Darstellung 1630 ersichtlich, dass bei dem vorliegenden Beispiel die Nervensignale an den Ausgängen der zweiten Stufe jeweils so verzögert sind, dass Aktionspotentiale 1632 von mehreren Nervenzellen an den Ausgängen der zweiten Stufe gleichzeitig anliegen. In anderen Worten, eine Trajektorie 1634, die durch die Aktionspotentiale 1632 beschrieben wird, ist zumindest näherungsweise gerade gebogen. Die Aktionspotentiale 1632 treten also gleichzeitig bzw. näherungsweise gleichzeitig (zumindest aber zeitlich überlappend) auf, sodass das gleichzeitige Auftreten durch eine Summation der an den Ausgängen der zweiten Stufe (bzw. Eingängen der dritten Stufe) anliegenden Signale einen deutlichen Peak aufweist, der groß genug ist, um einen vorgegebenen Schwellenwert (z. B. zwei oder drei) zu überschreiten.

In anderen Worten, es kann durch eine geeignete Summiereinrichtung (oder eine andere geeignete Einrichtung) erkannt werden, wann eine gekrümmte Trajektorie gerade gebogen wurde. Die entsprechende Information ermöglicht einen Rückschluss sowohl auf den Anfangszeitpunkt der Trajektorie als auch auf die Form der Trajektorie. Es kann nämlich festgestellt werden, nach Durchlaufen von wie vielen Stufe eine Trajektorie gerade gebogen wurde. Dadurch kann bei Kenntnis der Verzögerungen für die einzelnen Nervensignale in den Stufen der Einrichtung 1500 auch auf eine ursprüngliche Form der Trajektorie zurückgeschlossen werden. Ferner ist die Durchlaufzeit für die Stufen bevorzugterweise bekannt, sodass auch der Zeitpunkt, zu dem eine Trajektorie in die Einrichtung 1500 eingelaufen ist, bestimmt werden kann. Somit kann sowohl eine charakteristische Zeitinformationen der Trajektorien als auch Informationen über Form bzw. Krümmung der Trajektorien ermittelt werden, um zu bestimmen, welche Aktivitätsereignisse zu einer Trajektorie gehören und/oder welche Aktivitätsereignisse nicht zu einer Trajektorie gehören.

Im übrigen wird noch darauf hingewiesen, dass eine vierte grafische Darstellung 1640 zur Verbesserung des Verständnisses noch Ausgangssignale an Ausgängen einer dritten Stufe zeigt. Aktionspotentiale 1642 beschreiben eine Trajektorie 1644, die allerdings durch ein weiteres Verbiegen der Trajektorie wieder gekrümmt ist.

Es wird darauf hingewiesen, dass die Verzögerungen in den Stufen 1510, 1512, 1516 auf verschiedenem Weg erzielt werden können. Die Verzögerungseinrichtungen (z.B. 1530) können beispielsweise getaktet sein, und/oder es kann sich um kontinuierlich oder diskret einstellbare Verzögerungseinrichtungen handeln. Im übrigen ist es auch möglich, dass eine oder mehrere Verzögerungseinrichtungen in einer vorgegebenen Stufe für eine oder mehrere Nervensignale deaktiviert sind, sodass einige Nervensignale durch eine Stufe mit geringst möglicher Verzögerung weitergeleitet werden. Im übrigen ist festzuhalten, dass die Einrichtung 1500 insgesamt als eine analoge oder digitale Schaltung implementiert sein kann.

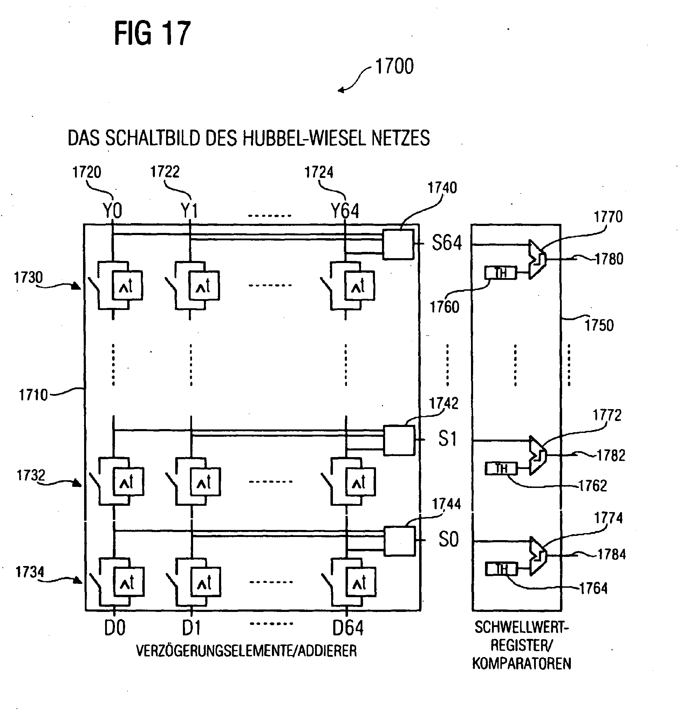

Figur 17 zeigt ein Schaltbild eines beispielhaften Hubel-Wiesel-Netzes zur erfindungsgemäßen Berechnung einer Analysedarstellung eines Audiosignals gemäß dem zweiten Ausführungsbeispiel der vorliegenden Erfindung. Das Schaltbild der Figur 17 ist in seiner Gesamtheit mit 1700 bezeichnet. Ein erster Schaltungsblock 1710 empfängt Eingangssignale 1720, 1722, 1724, die beispielsweise ein Nervenaktivitätsmuster oder ein Anregungsmuster einer Basilarmembran repräsentieren können. Die Eingangssignale 1720, 1722, 1724 werden dann durch eine Mehrzahl von Stufen 1730, 1732, 1734 geleitet. Ein Eingangssignal 1720 durchläuft somit eine Mehrzahl von Stufen 1730, 1732, 1734, wobei ein Eingangssignal 1720 in einer Stufe 1730, 1732, 1734 entweder eine Verzögerungseinrichtung durchläuft oder direkt zu einer darauffolgenden Stufe weitergeleitet wird.

In anderen Worten, die Verzögerungseinrichtungen können auch überbrückt werden.

In anderen Worten, jede Stufe umfasst für jedes Signal eine schaltbare Verzögerungseinrichtung, wobei die Verzögerungseinrichtung in einen Signalpfad, der von einem Eingangssignal durchlaufen wird, eingeschaltet werden kann oder überbrückt werden kann. Signale an den Eingängen jeder Stufe werden abgegriffen und Summierern 1740, 1742, 1744 zugeführt, wobei jeweils die an den Eingängen einer Stufe anliegenden Signale aufsummiert werden. Der erste Schaltungsblock 1710 bildet somit ein Gitter von Verzögerungselementen und Addieren, die in der gezeigten Weise verschaltet sind.

Das Hübel-Wiesel-Netz 1700 weist ferner eine Schwellwerteinrichtung 1750 auf, wobei jeweils ein Wert aus einem Schwellwertregister 1760, 1762, 1764 sowie ein Ausgang eines Summierers 1740, 1743, 1744 einem Komparator 1770, 1772, 1772 zugeführt wird. Ausgangssignale 1780, 1782, 1784 der Komparatoren 1770, 1772, 1774 liefern dabei eine Aussage darüber, ob an den Eingängen einer vorgegebenen Stufe 1730, 1732, 1734 eine Anzahl von Signalen gleichzeitig aktiv sind, wobei eine Mindestanzahl, bei der ein aktives Ausgangssignal 1780, 1782, 1784 ausgegeben wird, durch die Schwellwertregister 1760, 1762, 1764 festgelegt ist. In anderen Worten, durch die Komparatoren 1770, 1772, 1774 kann in Verbindung mit den Summierern 1740, 1742, 1744 und den Schwellwertregistern 1760, 1762, 1764 festgestellt werden, wenn (bzw. nach Durchlaufen wie vieler der Stufen 1730, 1732, 1734) eine Trajektorie, die über die Eingänge 1720, 1722, 1724 des ersten Blocks 1710 eingelesen wurde, gerade gebogen ist.

Die Verzögerungen der einzelnen Stufen 1730, 1732, 1734 können dabei geeignet vorgegeben werden, um eine Erkennung einer möglichst großen Anzahl von Trajektorien (bzw. Trajektorien-Formen) zu ermöglichen.

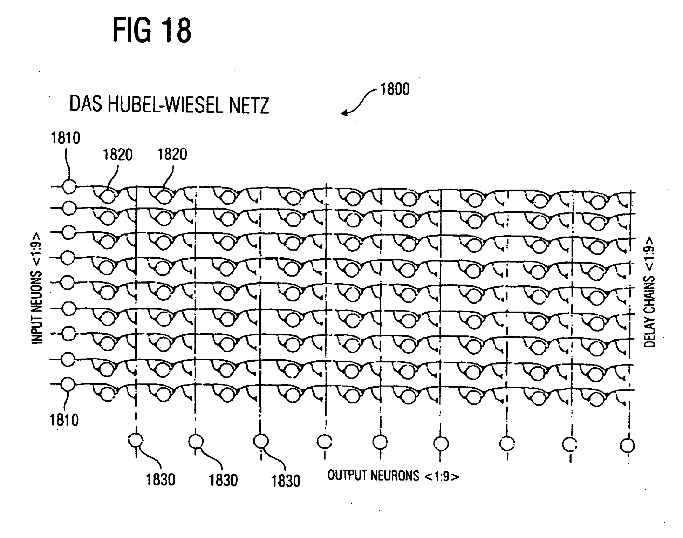

Figur 18 zeigt ein Blockschaltbild eines Hubel-Wiesel-Netzes zur erfindungsgemäßen Identifizierung von Trajektorien in einem Aktivitätsmuster gemäß dem zweiten Ausführungsbeispiel der vorliegenden Erfindung. Das gezeigte Hubel-Wiesel-Netzwerk ist in seiner Gesamtheit mit 1800 bezeichnet.

Eingangsneuronen 1810 sind ausgelegt, um ein Nervenaktivitätsmuster, eine Information über ein Neurotransmitter-Vesikel-Auftreten in einer Mehrzahl von inneren Haarzellen eines Ohrmodells, ein Anregungsmuster einer Basilarmembran eines Ohrmodells oder ein anderweitiges räumlich-zeitliches Aktivitätsmuster in einem Ohrmodell in Form von parallel bereitstehenden Zeitsignalen zu empfangen. Das Nervenaktivitätsmuster (bzw. das Anregungsmuster der Basilarmembran) oder ein anderes räumlich-zeitliches Aktivitätsmuster wird dabei unter optionaler Einbeziehung von Verzögerungsneuronen durch mehrere Stufen des neuronalen Netzes weitergeleitet. Hierbei ist anzumerken, dass die Verzögerungsneuronen 1820 auch überbrückt werden können, sodass in einer Stufe keine Verzögerung eines von einem Eingangsneuron 1810 gelieferten Signals stattfindet. Das neuronale Netz weist ferner Ausgangsneuronen 1830 auf. Die Verschaltung des neuronalen Netzes 1800 kann dabei der Figur 18 entnommen werden. Es wird darauf hingewiesen, dass das gezeigte neuronale Netz in der Lage ist, eine gekrümmte Kurve bzw. Trajektorie in einem Nervenaktivitätsmuster (bzw. Basilarmembran-Anregungsmuster), das über die Eingangsneuronen 1810 das neuronale Netz 1800 eingegeben wird, zu erkennen. Das neuronale Netz ist dabei (nach einem Training) in der Lage, sowohl Zeitpunkt als auch Form einer Trajektorie in einem über die Eingangsneuronen 1810 eingegebenen Nervenaktivitätsmuster zu bestimmen, wobei ein aktives Ausgangsneuron diese Informationen beschreibt. Die Informationen über die Form und Zeit einer Trajektorie sind hierbei dadurch codiert, welches Ausgangsneuron wann aktiviert wird.

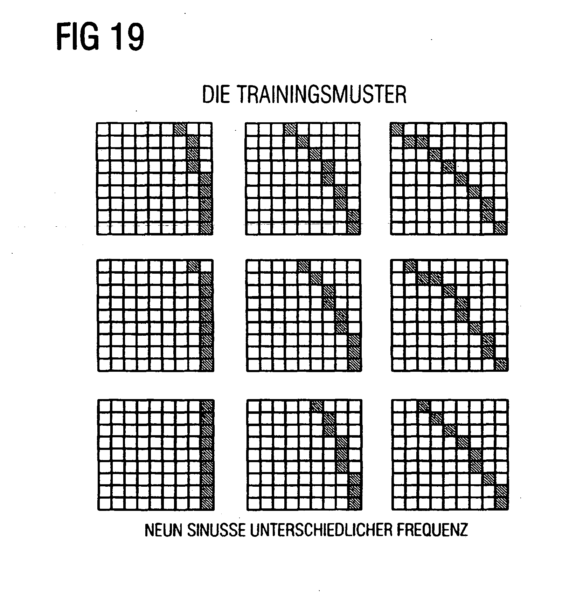

Figur 19 zeigt schließlich eine grafische Darstellung von beispielhaften Trennungsmustern, die zum Trainieren eines neuronalen Netzwerks 1800 verwendet werden können. Nach einem Training ist das neuronale Netzwerk 1800 dann in der Lage, entsprechende gerade oder gekrümmte Verläufe als Trajektorien zu identifizieren.

Somit ist festzuhalten, dass ein Hubel-Wiesel-Netz 1800 sich sehr gut dazu eignet, um Trajektorien in einem Nervenaktivitätsmuster zu erkennen. Hierzu ist lediglich das Nervenaktivitätsmuster an Eingänge 1720, 1722, 1724 des Hubel-Wiesel-Netzes anzulegen. An Ausgängen 1780, 1782, 1784 von Komparatoren 1770, 1772, 1774 stehen dann Signale an, die eine Aussage über Form und zeitliche Lage einer Trajektorie umfassen. Die Ausgangssignale 1780, 1782, 1784 können freilich bei Bedarf noch in eine leichter interpretierbare Form gebracht werden, aus der beispielsweise direkt eine Zeitinformation über eine Trajektorie hervorgeht. Somit kann die gewonnene Information verwendet werden, um zu bestimmen, welche Aktivitätsereignisse bzw. Aktivitätsimpulse zu einer Trajektorie gehören und/oder welche Aktivitätsereignisse bzw. Aktivitätsimpulse nicht zu einer Trajektorie gehören.

Ferner wird darauf hingewiesen, dass das Verarbeiten des Nervenaktivitätsmusters (bzw. allgemein: des Aktivitätsmusters) bevorzugt durch Anwendung einer sogenannten Hough-Transformation erfolgt (Vergleiche US 3,069,654). Eine Hough-Transformation ist nämlich in der Lage, in effektiver Weise aufeinanderfolgende Trajektorien in eine räumlich-zeitlichen Muster zu erkennen. Damit eignet sich die Hough-Transformation hervorragend zum Erkennen von Trajektorien in einem Aktivitätsmuster, das auf einem Audiosignal basiert.

Weiterhin wird darauf hingewiesen, dass auch andere bekannte Verfahren zur Mustererkennung eingesetzt werden können, um Trajektorien in den Nervenaktivitätsmuster zu erkennen. Beispielsweise können besonders vorteilhaft solche Verfahren eingesetzt werden, die eine Erkennung von gebogenen Linien ermöglichen, da erkannt wurde, dass Trajektorien in den Nervenaktivitätsmuster oder in einem der anderen beschriebenen Aktivitätsmuster typischerweise eine hyperbolische Form aufweisen. Hierbei ist zu berücksichtigen, dass das Nervenaktivitätsmuster für eine Mehrzahl von Nerven über der Zeit ein zweidimensionales Muster ergibt, wobei entlang einer ersten Richtung Signale auf mehreren Nervenfasern beispielsweise durch eine Intensitätsverteilung bzw. durch numerische Werte dargestellt werden können, während hingegen in einer zweiten Richtung die zeitliche Entwicklung angetragen ist (oder umgekehrt). Eine typische Darstellungsform für einen zeitlichen Verlauf eines Nervenaktivitätsmusters kann somit beispielsweise ähnlich zu den gezeigten Cochlea-Diagrammen (Cochleogrammen) sein.

Somit ist es möglich, einen beliebigen Mustererkennungsalgorithmus einzusetzen, der in der Lage ist, gekrümmte Linien zu erkennen. Allerdings hat sich gezeigt, dass die Anwendung einer Hough-Transformation besonders vorteilhaft ist, weil eben die Hough-Transformation sich besonders gut für eine Erkennung von gekrümmten Linien eignet. Im übrigen wird darauf hingewiesen, dass bei Durchführen einer Hough-Transformation in einer Anordnung 1700 bzw. 1800 auch bei Vorliegen mehrerer eng benachbarten Trajektorien ein gutes Erkennungsergebnis erzielt werden kann, wenn nur die Schwellenwerte bzw. Ansprechempfindlichkeiten der Ausgangsneuronen (bzw. der Komparatoren) passend eingestellt sind.

Ferner ist es möglich, im Rahmen einer Hough-Transformation (oder einer anderen Mustererkennungsoperation) auch eine Länge einer Trajektorie zu erkennen, sodass neben den Informationen über Zeitpunkt und Form der Trajektorie noch eine dritte Information für eine anschließende Ausfilterung von Aktivierungsereignissen, die nicht zu einer Sprachverständlichkeit eines Lauts beitragen, zur Verfügung steht.

Im übrigen wird darauf hingewiesen, dass auch ein zweidimensionaler Mustervergleich über der zeitlichen Darstellung des Nervenaktivitätsmuster durchgeführt werden kann, um eine Folge von Zeitinformationen, die eine zeitliche Lage von aufeinanderfolgenden Trajektorien beschreiben, zu gewinnen.

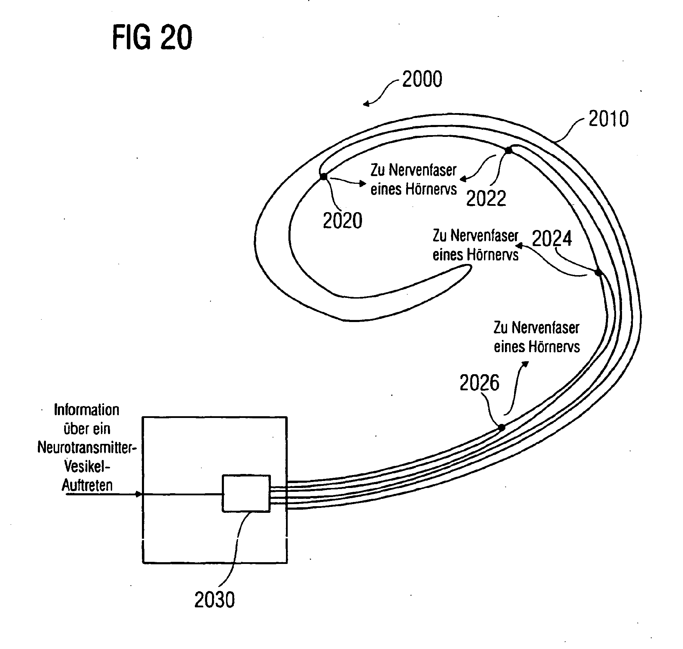

Fig. 20 zeigt eine schematische Darstellung eines Cochlea-Implantats der Verwendung in Verbindung mit der vorliegenden Erfindung. Die schematische Darstellung der Fig. 20 ist in ihrer Gesamtheit mit 2000 bezeichnet. Das in der schematischen Darstellung 2000 gezeigte Cochlea-Implantat umfasst einen Elektrodenträger 2010. Der Elektrodenträger 2010 ist ausgelegt, um in ein menschliches Ohr eingesetzt zu werden. Der Elektrodenträger 2010 umfasst Elektroden 2020, 2022, 2024, 2026, die ausgelegt sind, um verschiedene Nervenfasern eines (menschlichen oder tierischen) Hörnerv anzuregen. In anderen Worten, die Elektroden 2020, 2022, 2024, 2026 sind ausgelegt, um verschiedene Hörnerven anzuregen, die zu verschiedenen inneren Haarzellen gehören, die an verschiedenen Orten entlang der Cochlear angeordnet sind. Das Cochlea-Implantat 2000 umfasst ferner eine Elektroden-Ansteuereinrichtung 2030 die mit den Elektroden 2020, 2022, 2024, 2026 gekoppelt ist, und die ausgelegt ist, um die Elektroden 2020, 2022, 2024, 2026 mit einem beispielsweise elektrischen Ansteuersignal zu beaufschlagen. Die Elektroden-Ansteuereinrichtung 2030 ist im Übrigen ausgelegt, um eine Information über ein Neurotransmitter-Vesikel-Auftreten zu empfangen, und um basierend auf dem empfangenen Neurotransmitter-Vesikel-Auftreten Ansteuersignale zur Anregung von verschiedenen Nervenfasern des Hörnerven zu erzeugen.

Die Elektroden-Ansteuereinrichtung 2030 kann ferner aber auch ausgelegt sein, um ein anderes Aktivitätsmuster, das in einem Ohrmodell auftritt, zu empfangen, beispielsweise ein Nervenaktivitätsmuster von Nervenfasern, die mit der Mehrzahl von inneren Haarzellen eines Gehörmodells gekoppelt sind. Ferner kann das Cochlea-Implantat 2000 ausgelegt sein, um die Nervenfasern des menschlichen oder tierischen Hörnervs in verschiedener Weise anzuregen, beispielsweise elektrisch, chemisch oder mechanisch. Ganz allgemein lässt sich somit festhalten, dass das Cochlea-Implantat 2000 die Aufgabe erfüllt, eine beispielsweise in der erfindungsgemäßen Weise bereinigte Information über ein Aktivitätsmuster zu erhalten bzw. zu empfangen und basierend auf der bereinigten Information bzw. dem bereinigten Aktivitätsmuster Nervenfasern eines Hörnerven anzuregen.

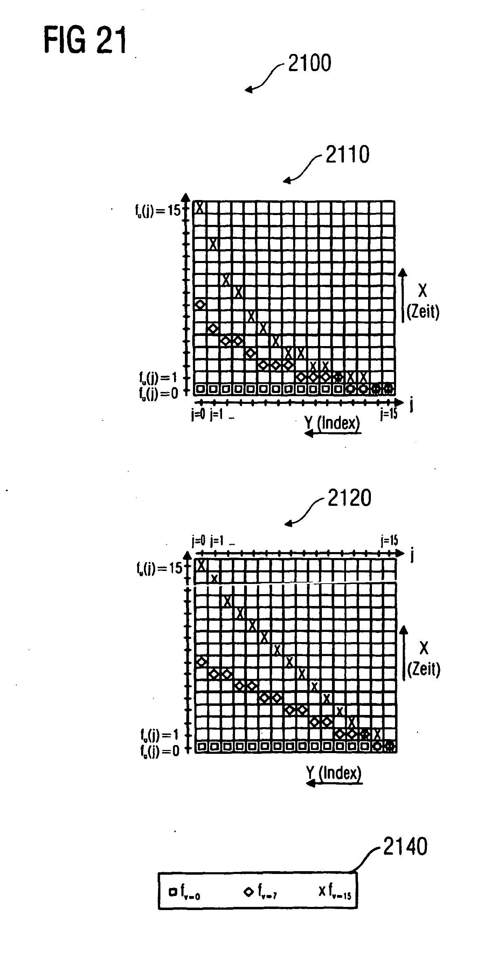

Fig. 21 zeigt eine grafische Darstellung von möglichen Trajektorien, die beispielsweise in einer zweidimensionalen Darstellung eines Aktivitätsmusters, wie sie anhand der Fig. 13 und 14 beschrieben wurde, durch eine Anordnung 1500, 1700 oder 1800 erkannt werden können. Die grafische Darstellung der Fig. 21 ist in ihrer Gesamtheit mit 2100 bezeichnet. Die möglichen Trajektorien sind in der grafischen Darstellung der Fig. 21 als ein Muster von Bildpunkten gezeigt, wobei in einer ersten Richtung x Aktivitätsereignisse als Funktion der Zeit dargestellt sind, und wobei in einer zweiten Richtung y die Aktivitätsereignisse für verschiedene innere Haarzellen für verschiedene Nervenfasern dargestellt sind.

Die grafische Darstellung 2100 der Fig. 21 zeigt 2 grafische Darstellungen 2110, 2120, die Trajektorien mit verschiedener Krümmung beschreiben. Die Lage der Aktivitätsimpulse in den grafischen Darstellungen 2110, 2120 wird für verschiedene Kurvenindizes $\nu$ ($\nu = 1,...,n_{p-1}$) und für verschiedene Krümmungen $f_{min}$ durch den folgenden Zusammenhang beschrieben:

$$f_\nu(j) = \frac{f_{min} \cdot \nu \cdot (n_p - 1 - j)}{(j + f_{min}) \cdot (n_p - 1)} .$$

Dabei ist $n_p$ die Größe einer quadratischen grafischen Darstellung, in der die jeweiligen Trajektorien dargestellt sind. In anderen Worten, $n_p$ gibt die Anzahl der bei einer Berechnung einer Trajektorie berücksichtigten inneren Haarzellen an. $j$ ist ein Index der inneren Haarzellen, der angibt, in welcher inneren Haarzelle ein Aktivitätsereignis zu berechnen ist. $\nu$ ist ein Index der betrachteten Kurve, wobei gilt:

$$\nu = 0, 1, ..., n_{p-1}$$

Ferner gilt:

$$j = 0, 1, ..., n_{p-1}$$

$f_{min}$ ist ferner eine freie Variable, die verwendet wird, um eine durchschnittliche Krümmung der Trajektorien zu beschreiben.

$f_v$ (j) beschreibt somit die zeitliche Lage eines Aktivitätsimpulses in bzw. an einer inneren Haarzelle mit dem Index j. Durch den Laufindex v werden verschiedene Trajektorien einer Schar von möglichen Trajektorien beschrieben.

Die grafischen Darstellungen 2110, 2120 zeigen beispielsweise jeweils drei Trajektorien der möglichen Schar von beispielsweise 16 Trajektorien, nämlich die Trajektorien mit den Scharindizes v = 0, v = 7 und v = 15 (vgl. Legende 2140).

Die vorliegenden Erfindung basiert somit auf einer Anordnung, bei der Neurotransmitter-Vesikel 1:1 als elektrische Impulse interpretiert werden, so dass jedes Neurotransmitter-Vesikel einen elektrischen Impuls triggert, der an das Cochlea-Implantat geleitet wird. Aus der Neurobiologie ist bekannt, dass ein Neurotransmitter-Vesikel nur dann ein Aktionspotenzial an nachgeschalteten Spiralganglienzellen auslösen kann, wenn sich die entsprechende Spiralganglienzelle nicht in einer Refraktärzeit befindet. Hat kurz zuvor ein früheres Neurotransmitter-Vesikel ein Aktionspotenzial (in der Spiralganglienzelle) generiert, bestimmt die Refraktärzeit die Zeit, bis wieder ein Ruhepotenzial erreicht ist. Die Auslösung von Aktionspotenzialen kann beispielsweise nach den sog. Hodgkin-Huxley-Gleichungen modelliert werden, die in der Veröffentlichung "Computing with Spiking Neurons" (Berechnen mit Puls-erzeugenden Neuronen) von W. Mass (In: Maass, W., Bishop, C. (Hrsg.): Pulsed Neuro Networks. Cambridge; MIT Press, 1998) auf den Seiten 397-404 beschrieben ist. Weitere Details können auch der bereits erwähnten Veröffentlichung "Schallanalyse: Neuronale Repräsentation des Hörvorgangs als Basis" von G. Szepannek, F. Klefenz und C. Weihs entnommen werden.

Jedes Aktionspotenzial kann eindeutig auf ein auslösendes Neurotransmitter-Vesikel zugeführt und zugeordnet werden. Hier setzt eine erste Rückfilterung an. Nur die Neurotransmitter-Vesikel, die ein Aktionspotential generieren, werden gespeichert und selektiv an das Cochlea-Implantat geleitet. Die Neurotransmitter-Vesikel-Verteilung besteht bei quasi-stationären Anregungslauten wie Vokalen aus Bündeln von Verzögerungstrajektorien. Die Verzögerungstrajektorien werden nach Auftrittszeitpunkt und Krümmungsform in einem neuronalen Hubel-Wiesel Vorwärts-Weiterleitungs-Zeitgebungsnetz (Hubel-Wiesel Feed Forward Timing Neural Net) detektiert. Wird das Vorliegen einer Verzögerungstrajektorie (Delay-Trajektorie) festgestellt, so werden alle Neurotransmitter-Vesikel markiert, die zu der Verzögerungstrajektorie gehören. Diese werden selektiv an das Cochlea-Implantat geleitet. Die anderen Neurotransmitter-Vesikel werden gelöscht.

Es wird ferner darauf hingewiesen, dass es im Übrigen bei einem weiteren Ausführungsbeispiel vorteilhaft ist, Vesikel der Verzögerungstrajektorien zu unterdrücken, also diejenigen Vesikel herauszufiltern (zu eliminieren), die Teil von Verzögerungstrajektorien sind, und nur die übrigen Vesikel, die nicht Teil von Verzögerungstrajektorien sind, an das Cochlea-Implantat weiterzuleiten. Damit können beispielsweise bestimmt störende Ereignisse aus dem Audiosignal (wie z.B. "Klicks") entfernt werden, da diese durch eine charakteristische Trajektorie gekennzeichnet sind.

Außerdem hat es sich als vorteilhaft erwiesen, alternativ dazu eine Filterung des Aktivitätsmusters über der Zeit in einer Mehrzahl von beispielsweise n Bändern vorzunehmen.

Ferner hat sich gezeigt, dass die Erkennung von Trajektorien, wie sie anhand der Fig. 15 bis 19 beschrieben wurde, besonders vorteilhaft in einem durch die Hardware-Beschreibungssprache VHDL definierte Geistiges-Eigentum-Kern (Intellectual Property Core) realisiert werden kann. Eine solche Realisierung basiert darauf, ein Hubel-Wiesel-Netzwerk, wie es beispielsweise anhand der Figuren 17 und 18 gezeigt ist, als eine schaltungstechnische Implementierung als getaktete Logikschaltung zu bilden.

Die vorliegende Erfindung bringt somit den Vorteil mit sich, dass die Sprachverständigkeit eines Audiosignals für Patienten mit einem Cochlea-Implantat wesentlich erhöht werden kann, indem bei der Erzeugung von Ansteuersignalen für ein Cochlea-Implantat Aktivitätsimpulse aus einem Aktivitätsmuster in bzw. an inneren Haarzellen eines Gehörmodells herausgefiltert werden, die für eine Sprachverständlichkeit nicht erheblich sind.

Ein Ausführungsbeispiel gemäß der Offenbarung schafft ein Verfahren 100 zum Erzeugen eines Ansteuersignals für ein Cochlea-Implantat, basierend auf einem Audiosignal, mit folgenden Schritten: Berechnen 110 einer ersten Information $A_1(t)$, $A_2(t)$, $A_n(t)$, die ein Aktivitätsmuster über der Zeit (t) an einer Mehrzahl von inneren Haarzellen IHZ1, IHZ2, IHZ3, IHZn eines Gehörmodells beschreibt, das sich aufgrund des Audiosignals 112 ergibt; Herausfiltern 120 von durch die erste Information beschriebenen Aktivitätsereignissen 1030,1032, 1034,1036,1040; AP7, AP14 basierend auf einer Erkennung eines charakteristischen Musters in dem Aktivitätsmuster, um aus der ersten Information eine bereinigte Information $A_{1,ber}(t)$, $A_{2,ber}(t)$, $A_{n,ber}(t)$ durch Eliminieren von Aktivitätsereignissen, die zu einem charakteristischen Muster gehören, oder durch Eliminieren von Aktivitätsereignissen, die nicht zu einem charakteristischen Muster gehören, zu erhalten, wobei die Erkennung eines charakteristischen Musters ein Erkennen eines linienförmigen Verlaufs in einer Darstellung des Aktivitätsmusters über der Zeit und über einen Index der inneren Haarzellen als charakteristisches Muster umfasst; und Verwenden der bereinigten Information als Ansteuersignal für ein Cochlea-Implantat, oder Ableiten eines Ansteuersignals für ein Cochlea-Implantat von der bereinigten Information.

Gemäß einem Aspekt der Offenbarung umfasst das Verfahren 100 ein Übertragen des Ansteuersignals zu dem Cochlea-Implantat.

Gemäß einem Aspekt der Offenbarung umfasst das Verfahren 100 ein Beaufschlagen von Elektroden des Cochlea-Implantats mit dem Ansteuersignal.

Gemäß einem Aspekt der Offenbarung beschreibt bei dem Verfahren 100 die erste Information $A_1(t)$, $A_2(t)$, $A_n(t)$ über das Aktivitätsmuster über der Zeit zeitliche Verläufe 410 einer Anzahl an freigesetzten Neurotransmitter-Vesikeln für eine Mehrzahl von inneren Haarzellen IHZ1, IHZ2, IHZ3, IHZn, und das Herausfiltern 120 umfasst ein Entfernen von Neurotransmitter-Vesikel-Auftreten 1030, 1032, 1034, 1036, 1040, 1046, die nicht zu einer Erzeugung eines Aktionspotentials 472, AP1, AP2,AP3, AP4, AP5, AP6, AP7, AP8, AP9, AP10, AP11, AP12, AP13, AP14 beitragen oder ein Aktionspotential generieren, aus der ersten Information.

Gemäß einem Aspekt der Offenbarung umfasst ein Entfernen von Neurotransmitter-Vesikel-Auftreten 1030, 1032, 1034, 1036, 1040, 1046 bei dem Verfahren 100 ein zu Null-Setzen einer Anzahl an freigesetzten Neurotransmitter-Vesikeln in einem bestimmten Zeitintervall für eine bestimmte Haarzelle IHZ1, IHZ2, IHZ3, IHZn, wenn in dem bestimmten Zeitintervall in der bestimmten Haarzelle freigesetzte Neurotransmitter-Vesikel nicht zu einer Erzeugung eines Aktionspotentials auf einer mit der bestimmten Haarzelle gekoppelten Nervenfaser NF1, NF2, NF3, NF4, NF5, NF6, NFn beitragen oder ein Aktionspotential auf der mit der bestimmten Haarzelle gekoppelten Nervenfaser generieren.

Gemäß einem Aspekt der Offenbarung umfasst das Herausfiltern 120 bei dem Verfahren 100 ein Entfernen von Neurotransmitter-Vesikel-Auftreten 1030,1032, 1034,1036 für Haarzellen IHZ1, IHZ2, IHZ3, IHZn, die sich aufgrund einer zeitlich zurückliegenden Freisetzung eines Aktionspotentials 472, AP1, AP2,AP3, AP4, AP5, AP6, AP7, AP8, AP9, AP10, AP11, AP12, AP13, AP14 in einem Refraktär-Zeitraum befinden.

Gemäß einem Aspekt der Offenbarung umfasst das Herausfiltern 120 bei dem Verfahren 100 ein Bestimmen, welche der durch die erste Information beschriebenen Aktivitätsereignisse 1020,1022,1024, 1026,1030, 1032,1034,1036,1040,1046, 1050, 1052, 1054, 1056; AP1, AP2, AP3, AP4, Ap5, Ap6, AP67, AP8, AP9, AP10, AP11, AP12, AP13, AP14 nicht zu einer Trajektorie 1028, 01058; 1130, 1140 gehören, und ein Ableiten der bereinigten Information $A_{1,ber}(t)$, $A_{2,ber}(t)$, $A_{n,ber}(t)$ über das Aktivitätsmuster, wobei die bereinigte Information aus der ersten Information durch Eliminieren von Aktivitätsereignissen, die nicht zu einer Trajektorie gehören, abgeleitet wird.

Gemäß einem Aspekt der Offenbarung umfasst das Herausfiltern 120 bei dem Verfahren 100 ein Bestimmen, welche der durch die erste Information beschriebenen Aktivitätsereignisse 1020, 1022, 1024, 1026, 1030, 1032, 1034, 1036, 1040, 1046, 1050, 1052 , 1054,1056; AP1, AP2, AP3, AP4, Ap5, Ap6, AP67, AP8, AP9, AP10, AP11, AP12, AP13, AP14 zu einer Trajektorie 1028,1058;1130,1140 gehören, und ein Ableiten der bereinigten Information $A_{1,ber}(t)$, $A_{2,ber}(t)$, $A_{n,ber}(t)$ über das Aktivitätsmuster, wobei die bereinigte Information aus der ersten Information durch Eliminieren von Aktivitätsereignissen, die zu einer Trajektorie gehören, abgeleitet wird.

Gemäß einem Aspekt der Offenbarung beschreibt bei dem Verfahren 100 die erste Information $A_1(t)$, $A_2(t)$, $A_n(t)$ zeitliche Verläufe 410,1010,1012,1014, 1016 einer Anzahl an freigesetzten Neurotransmitter-Vesikeln für eine Mehrzahl von inneren Haarzellen IHZ1, IHZ2, IHZ3, IHZn, wobei jeder inneren Haarzelle aus der Mehrzahl von inneren Haarzellen ein zeitlicher Verlauf zugeordnet ist.

Gemäß einem Aspekt der Offenbarung beschreibt bei dem Verfahren 100 die erste Information $A_1(t)$, $A_2(t)$, $A_n(t)$ eine Mehrzahl von zeitlichen Spannungsverläufen 1126 an einer Mehrzahl von Nervenfasern NF1, NF2, NF3, NFn, die mit verschiedenen inneren Haarzellen IHZ1, IHZ2, IHZ3, IHZn gekoppelt sind, wobei jeder inneren Haarzelle aus der Mehrzahl von inneren Haarzellen ein zeitlicher Spannungsverlauf auf einer zugehörigen Nervenfaser zugeordnet ist.

Gemäß einem Aspekt der Offenbarung beschreibt bei dem Verfahren 100 eine Trajektorie 1028,1058;1130,1140 ein Auftreten von Aktivitätsereignissen an einer Mehrzahl von inneren Haarzellen IHZ1, IHZ2, IHZ3, IHZn aufgrund des gleichen Ereignisses in dem Audiosignal 112.

Gemäß einem Aspekt der Offenbarung besteht bei dem Verfahren 100 eine bestimmte Trajektorie 1028,1058;1130,1140 aus einer Mehrzahl von Aktivitätsereignissen 1020,1022,1024,1026,1050,1052,1054, 1056; AP1, AP2, AP3, AP4, AP5, AP6, AP8, AP9, AP10, AP11, AP12, AP13 an eine Mehrzahl von inneren Haarzellen IHZ1, IHZ2, IHZ3, IHZn, und die zu einer Trajektorie gehörigen Aktivitätsereignisse nähern in einer zweidimensionalen graphischen Darstellung 1220;1300; 1400 eine gerade oder gekrümmte Linie 1410,1412, 1414,1416 an, wobei in der zweidimensionalen graphischen Darstellung an einer ersten Achse die Zeit t aufgetragen ist, und wobei an einer zweiten Achse ein Index i der Haarzellen angetragen ist, zu denen die zeitlichen Verläufe gehören, und wobei die Aktivitätsereignisse in der zweidimensionalen Darstellung gekennzeichnet sind.

Gemäß einem Aspekt der Offenbarung umfasst bei dem Verfahren 100 das Bestimmen, welche der durch die erste Information $A_1(t)$, $A_2(t)$, $A_n(t)$ beschriebenen Aktivitätsereignisse 1030,1032,1034,1036,1040; AP7, AP14 zu einer Trajektorie 1028,1058;1130,1140 gehören oder nicht zu einer Trajektorie gehören, ein Durchführen einer Mustererkennung, wobei das Durchführen der Mustererkennung folgende Schritte aufweist: Empfangen der ersten Information über das Aktivitätsmuster über der Zeit; Interpretieren des Aktivitätsmusters über der Zeit für eine Mehrzahl von Haarzellen als eine zweidimensionale Darstellung 1022,1300,1400, wobei eine erste Richtung x der zweidimensionalen Darstellung die Zeit t beschreibt, wobei eine zweite Richtung y der zweidimensionalen Darstellung einen Index i der Haarzellen beschreibt, und wobei die in der zweidimensionalen Darstellung enthaltene Information ein Auftreten von Aktivitätsereignissen in Abhängigkeit von der Zeit und von dem Index der inneren Haarzellen beschreibt; und Identifizieren eines Kurvenverlaufs in der zweidimensionalen Darstellung als eine Trajektorie, falls der Kurvenverlauf hinsichtlich eines vorgegebenen Ähnlichkeitsmaßes mindestens eine vorgegebene Ähnlichkeit mit einem Vergleichs-Kurvenverlauf aufweist.

Gemäß einem Aspekt der Offenbarung weist bei dem Verfahren 100 das Durchführen der Mustererkennung folgende Schritte auf: Empfangen einer Information aus der ersten Information über Aktivitätsereignisse 1030,1032,1034,1036,1040; AP7, AP14 an verschiedenen inneren Haarzellen IHZ1, IHZ2, IHZ3, IHZn zu einem ersten Zeitpunkt oder für ein erstes Zeitintervall; Interpretieren der Information für den ersten Zeitpunkt oder für das erste Zeitintervall als eine erste Zeile einer zweidimensionalen Darstellung der ersten Information; Empfangen einer Information aus der ersten Information über Aktivitätsereignisse an verschiedenen inneren Haarzellen für einen zweiten Zeitpunkt oder für ein zweites Zeitintervall; und Interpretieren der Information für den zweiten Zeitpunkt oder für das zweite Zeitintervall als eine zweite Zeile einer zweidimensionalen Darstellung der ersten Information.

Gemäß einem Aspekt der Offenbarung umfasst bei dem Verfahren 100 das Identifizieren eines Kurvenverlaufs in der zweidimensionalen Darstellung 1220; 1300;1400 ein Vergleichen der zweidimensionalen Darstellung oder eines Ausschnitts aus der zweidimensionalen Darstellung mit einem Vergleichsmuster.

Gemäß einem Aspekt der Offenbarung umfasst bei dem Verfahren 100 das Identifizieren eines Kurvenverlaufs in der zweidimensionalen Darstellung 1220; 1300;1400 ein Identifizieren eines geraden oder hyperbelartig gekrümmten Kurvenverlaufs.

Gemäß einem Aspekt der Offenbarung ist bei dem Verfahren 100 der Vergleichs-Kurvenverlauf eine gerade oder hyperbelförmige Kurve.

Gemäß einem Aspekt der Erfindung umfasst bei dem Verfahren 100 das Identifizieren eines Kurvenverlaufs 1028, 1058; 1130, 1140; 1410, 1412, 1414, 1416 in der zweidimensionalen Darstellung 1220;1300;1400 als eine Trajektorie ein schrittweises Verzerren der zweidimensionalen Darstellung, um eine verzerrte zweidimensionale Darstellung des Aktivitätsmusters über der Zeit zu erhalten, sowie ein Bestimmen, ob in der verzerrten zweidimensionalen Darstellung eine näherungsweise gerade Linie enthalten ist, wobei eine in der verzerrten zweidimensionalen Darstellung erkannte näherungsweise gerade Linie als eine Trajektorie identifiziert wird.

Gemäß einem Aspekt der Offenbarung erfolgt bei dem Verfahren 100 das schrittweise Verzerren derart, dass eine in der zweidimensionalen Darstellung 1220; 1300;1400 enthaltene gekrümmte Linie durch das schrittweise Verzerren schrittweise gerade gebogen wird.

Gemäß einem Aspekt der Offenbarung umfasst bei dem Verfahren 100 das Identifizieren eines Kurvenverlaufs 1028,1058;1130,1140;1410,1412,1414,1416 in der zweidimensionalen Darstellung 1220;1300;1400 als eine Trajektorie ein Anwenden einer Hough-Transformation, die für eine Erkennung von geraden oder gebogenen Kurvensegmenten konfiguriert ist, auf die zweidimensionale Darstellung, wobei eine Trajektorie basierend auf einem Ergebnis der Hough-Transformation erkannt wird.

Gemäß einem Aspekt der Offenbarung umfasst bei dem Verfahren 100 das Identifizieren eines Kurvenverlaufs 1028,1058;1130,1140;1410,1412,1414,1416 in der zweidimensionalen Darstellung 1220;1300;1400 als eine Trajektorie ein Eingeben der zweidimensionalen Darstellung in ein neuronales Netz, wobei das neuronale Netz ausgelegt ist, um einen Kurvenverlauf in der zweidimensionalen Darstellung zu erkennen, der eine Trajektorie bildet.

Gemäß einem Aspekt der Offenbarung umfasst bei dem Verfahren 100 das Bestimmen, welche Aktivitätsereignisse 1030,1032,1034,1036,1040; AP7, AP14 zu einer Trajektorie gehören oder nicht zu einer Trajektorie gehören, ein Bestimmen von Aktivitätsereignissen, die zu einer Trajektorie gehören, wobei eine Trajektorie nur dann erkannt wird, wenn eine Anzahl von zusammengehörigen Aktivitätsereignissen, die durch das gleiche Ereignis in dem Audiosignal bedingt sind, größer eine vorgegebene Mindestanzahl ist.

Gemäß einem Aspekt der Offenbarung umfasst bei dem Verfahren 100 ein Ereignis in dem Audiosignal 112 einen Beginn eines Vokals, eines Konsonanten, eines Lauts oder einer Klicks.

Gemäß einem Aspekt der Offenbarung wird bei dem Verfahren 100 eine Trajektorie 1028,1058; 1130,1140;1410,1412,1414,1416 durch eine Mehrzahl von Aktivitätsimpulsen 1030,1032,1034,1036,1040; AP7,AP14 an einer Mehrzahl von inneren Haarzellen IHZ1, IHZ2, IHZ3, IHZn, die eine Wanderwelle 670 auf einer Basilarmembran 620 des Ohrmodells beschreiben, gebildet.

Gemäß einem Aspekt der Offenbarung umfasst bei dem Verfahren 100 eine Trajektorie Aktivitätsereignisse 1030,1032,1034,1036,1040; AP7,AP14 an einer Mehrzahl von inneren Haarzellen IHZ1, IHZ2, IHZ3, IHZn, die durch eine Anregung der Mehrzahl von inneren Haarzellen durch eine einzige Wanderwelle 670 auf einer Basilarmembran 620 des Ohrmodells ausgelöst werden.

Gemäß einem Aspekt der Offenbarung umfasst bei dem Verfahren 100 das Ableiten der bereinigten Information $A_{1,ber}(t)$, $A_{2,ber}(t)$, $A_{n,ber}(t)$ ein Erkennen einer Trajektorie 1028,1058;1130,1140;1410,1412,1414, 1416 in der ersten Information, ein Markieren von Aktivitätsereignissen 1020,1022,1024,1026,1050,1052, 1054,1056; AP1, AP2, AP3, AP4, AP5, AP6, AP8, AP9, AP10, AP11, AP12, AP13, die zu der Trajektorie gehören, sowie ein Eliminieren von nicht-markierten Aktivitätsereignissen 1040,1046; AP7, AP14.

Gemäß einem Aspekt der Offenbarung umfasst bei dem Verfahren 100 das Ableiten der bereinigten Information $A_{1,ber}(t)$, $A_{2,ber}(t)$, $A_{n,ber}(t)$ ein Markieren von Aktivitätsereignissen, derer Abstand von einer erkannten Trajektorie 1028,1058;1130,1140;1410,1412,1414, 1416 kleiner als ein vorgegebener maximaler Abstand ist.

Gemäß einem Aspekt der Offenbarung umfasst bei dem Verfahren 100 das Berechnen 110 der ersten Information $A_1(t)$, $A_2(t)$, $A_n(t)$ über das Aktivitätsmuster über der Zeit ein Berechnen des Aktivitätsmusters über der Zeit an einer Mehrzahl von inneren Haarzellen IHZ1, IHZ2, IHZ3, IHZn eines Gehörmodells, wobei die inneren Haarzellen an verschiedenen Orten entlang der Cochlea angeordnet sind.

Gemäß einem Aspekt der Offenbarung weisen bei dem Verfahren 100 die inneren Haarzellen IHZ1, IHZ2, IHZ3, IHZn maximale Empfindlichkeiten bei verschiedenen in dem Audiosignal 112 enthaltenen Frequenzen auf.

Gemäß einem Aspekt der Offenbarung ist bei dem Verfahren 100 die bereinigte Information $A_{1,ber}(t)$, $A_{2,ber}(t)$, $A_{n,ber}(t)$ eine bereinigte Information über ein Neurotransmitter-Vesikel-Auftreten, und das Verwenden der bereinigten Information umfasst ein Übertragen der bereinigten Information an das Cochlea-Implantat.

Gemäß einem Aspekt der Offenbarung ist bei dem Verfahren 100 die bereinigte Information $A_{1,ber}(t)$, $A_{2,ber}(t)$, $A_{n,ber}(t)$ ein bereinigtes Nervenaktivitätsmuster, und das Verwenden der bereinigten Information umfasst ein Übertragen der bereinigten Information an das Cochlea-Implantat.

Ein Ausführungsbeispiel gemäß der Erfindung schafft ein Computerprogramm zur Durchführung des Verfahrens 100, wenn das Computerprogramm auf einem Computer abläuft.

Ein Ausführungsbeispiel gemäß der Erfindung schafft eine Vorrichtung zum Erzeugen eines Ansteuersignals für ein Cochlea-Implantat basierend auf einem Audiosignal, mit folgenden Merkmalen: einer Einrichtung zum Berechnen einer ersten Information $A_1(t)$, $A_2(t)$, $A_n$ (t) über ein Aktivitätsmuster 1030,1032,1034,1036,1040; AP7, AP14 über der Zeit an einer Mehrzahl von inneren Haarzellen IHZ1, IHZ2, IHZ3, IHZn eines Gehörmodells, das sich aufgrund des Audiosignals ergibt; einer Einrichtung zum Herausfiltern von durch die erste Information beschriebenen Aktivitätsereignissen 1030,1032,1034, 1036,1040; AP7, AP14 basierend auf einer Erkennung eines charakteristischen Musters in dem Aktivitätsmuster, um aus der ersten Information eine bereinigte Information $A_{1,ber}(t)$, $A_{2,ber}(t)$, $A_{n,ber}(t)$ durch Eliminieren von Aktivitätsereignissen, die zu einem charakteristischen Muster gehören, oder durch Eliminieren von Aktivitätsereignissen, die nicht zu einem charakteristischen Muster gehören, zu erhalten, wobei die Erkennung eines charakteristischen Musters ein Erkennen eines linienförmigen Verlaufs in einer Darstellung des Aktivitätsmusters über der Zeit und über einen Index der inneren Haarzellen als charakteristisches Muster umfasst; und einer Einrichtung zum Ansteuern des Cochlea-Implantats, die ausgelegt ist, um die bereinigte Information oder eine von der bereinigten Information abgeleitete Information als Ansteuersignal für das Cochlea-Implantat zu verwenden.

Gemäß einem Aspekt der Erfindung ist bei der Vorrichtung die Einrichtung zum Ansteuern des Cochlea-Implantats ausgelegt, um das Ansteuersignal an das Cochlea-Implantat zu übertragen.

Gemäß einem Aspekt der Erfindung ist bei der Vorrichtung die Einrichtung zum Ansteuern des Cochlea-Implantats ausgelegt, um Elektroden des Cochlea-Implantats mit dem Ansteuersignal zu beaufschlagen.

Gemäß einem Aspekt der Erfindung weist die Vorrichtung folgende Merkmale auf: eine Einrichtung zum Bestimmen, welche der durch die erste Information $A_1(t)$, $A_2(t)$, $A_n(t)$ beschriebenen Aktivitätsereignisse 1030, 1032, 1034, 1036, 1040, 1046; 472, AP1, AP2, AP3, AP4, AP5, AP6, AP7, AP8, AP9, AP10, AP11, AP12, AP13, AP14 nicht zu einer Trajektorie gehören, und eine Einrichtung zum Ableiten der bereinigten Information $A_{1,ber}(t)$, $A_{2,ber}(t)$, $A_{n,ber}(t)$ über das Aktivitätsmuster durch Eliminieren von Aktivitätsereignissen, die nicht zu einer Trajektorie gehören, auf der ersten Information.

Gemäß einem Aspekt der Erfindung beschreibt bei der Vorrichtung die erste Information zeitliche Verläufe 910,1010,1012,1014,1016 eine Anzahl an freigesetzten Neurotransmitter-Vesikeln für eine Mehrzahl von inneren Haarzellen IHZ1, IHZ2, IHZ3, IHZn, wobei jeder inneren Haarzelle aus der Mehrzahl von inneren Haarzellen ein zeitlicher Verlauf zugeordnet ist.

Gemäß einem Aspekt der Erfindung beschreibt bei der Vorrichtung die erste Information $A_1(t)$, $A_2(t)$, $A_n$ (t) eine Mehrzahl von zeitlichen Spannungsverläufen 1126 an einer Mehrzahl von Nervenfasern NF1, NF2, NF3, NFn, die mit verschiedenen inneren Haarzellen IHZ1, IHZ2, IHZ3, IHZn gekoppelt sind, wobei jeder inneren Haarzelle aus der Mehrzahl von inneren Haarzellen ein zeitlicher Spannungsverlauf auf einer zugehörigen Nervenfaser zugeordnet ist.

Gemäß einem Aspekt der Erfindung umfasst bei der Vorrichtung die Einrichtung zum Bestimmen, welche der durch die erste Information $A_1(t)$, $A_2(t)$, $A_n(t)$ beschriebenen Aktivitätsereignisse nicht zu einer Trajektorie gehören, eine Kurvenerkennungseinrichtung 1500; 1700; 1800, die ausgelegt ist, um das Aktivitätsmuster über der Zeit an der Mehrzahl von Haarzellen in Form von parallelen Signalen 1520,1522,1524;1720,1722, 1724 parallel zu empfangen, wobei jedes Signal einer Haarzelle zugeordnet ist, und um die Signale unterschiedlich schnell durch eine Mehrzahl von hintereinander geschalteten Stufen 1510, 1512, 1514;1730,1732, 1734 weiterzuleiten, wobei mindestens eine vorbestimmte Stufe der Mehrzahl von hintereinandergeschalteten Stufen eine Schwellwerterkennungseinrichtung 1770, 1772, 1774 aufweist, die ausgelegt ist, um zu erkennen, wenn mindestens eine vorgegebene Anzahl an Signalen in der vorbestimmten Stufe gleichzeitig aktiv sind, wobei das Vorliegen von mindestens der vorgegeben Anzahl an gleichzeitig aktiven Signalen in oder vorbestimmten Stufe ein Vorliegen einer Trajektorie 1028,1058;1130, 1140;1410,1412,1414,1416 in dem Aktivitätsmuster anzeigt.

Gemäß einem Aspekt der Erfindung ist bei der Vorrichtung mindestens eine Stufe 1510,1512,1514; 1730,1732,1734 ausgelegt, um mehrere Signale 1520,1522,1524;1720,1722,1724 bei einer Weiterleitung durch die Stufe unerschiedlich stark zu verzögern.

Gemäß einem Aspekt der Erfindung umfasst bei der Vorrichtung die Einrichtung zum Herausfiltern eine Einrichtung zur Durchführung einer parallelen Hough-Transformation, die ausgelegt ist, um das Vorliegen einer Trajektorie in dem Aktivitätsmuster anzuzeigen, wobei eine Trajektorie ein charakteristisches Muster in dem Aktivitätsmuster bildet.

**Patentansprüche**

1. Computerprogramm zur Durchführung eines Verfahrens (100) zum Erzeugen eines Ansteuersignals für ein Cochlea-Implantat, basierend auf einem Audiosignal, wenn das Computerprogramm auf einem Computer abläuft,

   wobei das Verfahren (100) zum Erzeugen des Ansteuersignals folgende Schritte aufweist:

   Berechnen (110) einer ersten Information ($A_1$(t), $A_2$(t), $A_n$(t)), die ein Aktivitätsmuster über der Zeit (t) an einer Mehrzahl von inneren Haarzellen (IHZ1, IHZ2, IHZ3, IHZn) eines Gehörmodells beschreibt, das sich aufgrund des Audiosignals (112) ergibt;

   Herausfiltern (120) von durch die erste Information beschriebenen Aktivitätsereignissen (1030, 1032,1034,1036,1040; AP7, AP14) basierend auf einer Erkennung eines charakteristischen Musters in dem Aktivitätsmuster, um aus der ersten Information eine bereinigte Information ($A_{1,ber}$(t), $A_{2,ber}$(t), $A_{n,ber}$(t)) durch Eliminieren von Aktivitätsereignissen, die zu einem charakteristischen Muster gehören, oder durch Eliminieren von Aktivitätsereignissen, die nicht zu einem charakteristischen Muster gehören, zu erhalten,

   wobei die Erkennung eines charakteristischen Musters ein Erkennen eines linienförmigen Verlaufs in einer Darstellung des Aktivitätsmusters über der Zeit und über einen Index der inneren Haarzellen als charakteristisches Muster umfasst; und

   Verwenden der bereinigten Information als Ansteuersignal für ein Cochlea-Implantat, oder Ableiten eines Ansteuersignals für ein Cochlea-Implantat von der bereinigten Information.

2. Computerprogramm gemäß Anspruch 1, wobei die erste Information ($A_1$(t), $A_2$(t), $A_n$(t)) über das Aktivitätsmuster über der Zeit zeitliche Verläufe (410) einer Anzahl an freigesetzten Neurotransmitter-Vesikeln für eine Mehrzahl von inneren Haarzellen (IHZ1, IHZ2, IHZ3, IHZn) beschreibt, und

   bei dem das Herausfiltern (120) ein Entfernen von Neurotransmitter-Vesikel-Auftreten (1030,1032, 1034,1036,1040,1046), die nicht zu einer Erzeugung eines Aktionspotentials (472, AP1, AP2,AP3, AP4, AP5, AP6, AP7, AP8, AP9, AP10, AP11, AP12, AP13, AP14) beitragen oder ein Aktionspotential generieren, aus der ersten Information umfasst.

3. Computerprogramm gemäß Anspruch 2, wobei ein Entfernen von Neurotransmitter-Vesikel-Auftreten (1030,1032,1034,1036,1040,1046) ein zu Null-Setzen einer Anzahl an freigesetzten Neurotransmitter-Vesikeln in einem bestimmten Zeitintervall für eine bestimmte Haarzelle (IHZ1, IHZ2, IHZ3, IHZn) umfasst, wenn in dem bestimmten Zeitintervall in der bestimmten Haarzelle freigesetzte Neurotransmitter-Vesikel nicht zu einer Erzeugung eines Aktionspotentials auf einer mit der bestimmten Haarzelle gekoppelten Nervenfaser (NF1, NF2, NF3, NF4, NF5, NF6, NFn) beitragen oder ein Aktionspotential auf der mit der bestimmten Haarzelle gekoppelten Nervenfaser generieren.

4. Computerprogramm gemäß Anspruch 2 oder 3, wobei das Herausfiltern (120) ein Entfernen von Neurotransmitter-Vesikel-Auftreten (1030,1032,1034, 1036) für Haarzellen (IHZ1, IHZ2, IHZ3, IHZn) umfasst, die sich aufgrund einer zeitlich zurückliegenden Freisetzung eines Aktionspotentials (472, AP1, AP2, AP3, AP4, AP5, AP6, AP7, AP8, AP9, AP10, AP11, AP12, AP13, AP14) in einem Refraktär-Zeitraum befinden.

5. Computerprogramm gemäß Anspruch 1, wobei das Herausfiltern (120) ein Bestimmen, welche der durch die erste Information beschriebenen Aktivitätsereignisse (1020,1022,1024,1026,1030,1032,1034, 1036,1040,1046,10 50,1052,1054,1056; AP1, AP2, AP3, AP4, Ap5, Ap6, AP67, AP8, AP9, AP10, AP11, AP12, AP13, AP14) nicht zu einer Trajektorie (1028,1058;1130,1140) gehören, und ein Ableiten der bereinigten Information ($A_{1,ber}$(t), $A_{2,ber}$(t), $A_{n,ber}$(t)) über das Aktivitätsmuster umfasst, wobei die bereinigte Information aus der ersten Information durch Eliminieren von Aktivitätsereignissen, die nicht zu einer Trajektorie gehören, abgeleitet wird.

6. Computerprogramm gemäß Anspruch 1, wobei das Herausfiltern (120) ein Bestimmen, welche der durch die erste Information beschriebenen Aktivitätsereignisse (1020,1022,1024,1026,1030,1032,1034, 1036,1040,1046,10 50,1052,1054,1056; AP1, AP2, AP3, AP4, Ap5, Ap6, AP67, AP8, AP9, AP10, AP11, AP12, AP13, AP14) zu einer Trajektorie (1028,1058; 1130,1140) gehören, und ein Ableiten der bereinigten Information ($A_{1,ber}(t)$, $A_{2,ber}(t)$, $A_{n,ber}(t)$) über das Aktivitätsmuster umfasst, wobei die bereinigte Information aus der ersten Information durch Eliminieren von Aktivitätsereignissen, die zu einer Trajektorie gehören, abgeleitet wird.

7. Computerprogramm gemäß Anspruch 5 oder 6, wobei die erste Information ($A_1(t)$, $A_2(t)$, $A_n(t)$) zeitliche Verläufe (410,1010,1012,1014,1016) einer Anzahl an freigesetzten Neurotransmitter-Vesikeln für eine Mehrzahl von inneren Haarzellen (IHZ1, IHZ2, IHZ3, IHZn) beschreibt, wobei jeder inneren Haarzelle aus der Mehrzahl von inneren Haarzellen ein zeitlicher Verlauf zugeordnet ist; oder
wobei die erste Information ($A_1(t)$, $A_2(t)$, $A_n(t)$) eine Mehrzahl von zeitlichen Spannungsverläufen (1126) an einer Mehrzahl von Nervenfasern (NF1, NF2, NF3, NFn), die mit verschiedenen inneren Haarzellen (IHZ1, IHZ2, IHZ3, IHZn) gekoppelt sind, beschreibt, wobei jeder inneren Haarzelle aus der Mehrzahl von inneren Haarzellen ein zeitlicher Spannungsverlauf auf einer zugehörigen Nervenfaser zugeordnet ist.

8. Computerprogramm gemäß einem der Ansprüche 5 bis 7, wobei das Bestimmen, welche der durch die erste Information ($A_1(t)$, $A_2(t)$, $A_n(t)$) beschriebenen Aktivitätsereignisse (1030,1032,1034,1036,1040; AP7, AP14) zu einer Trajektorie (1028,1058; 1130,1140) gehören oder nicht zu einer Trajektorie gehören, ein Durchführen einer Mustererkennung umfasst, wobei das Durchführen der Mustererkennung folgende Schritte aufweist:

Empfangen der ersten Information über das Aktivitätsmuster über der Zeit;
Interpretieren des Aktivitätsmusters über der Zeit für eine Mehrzahl von Haarzellen als eine zweidimensionale Darstellung (1022,1300, 1400), wobei eine erste Richtung (x) der zweidimensionalen Darstellung die Zeit (t) beschreibt, wobei eine zweite Richtung (y) der zweidimensionalen Darstellung einen Index (i) der Haarzellen beschreibt, und wobei die in der zweidimensionalen Darstellung enthaltene Information ein Auftreten von Aktivitätsereignissen in Abhängigkeit von der Zeit und von dem Index der inneren Haarzellen beschreibt; und
Identifizieren eines Kurvenverlaufs in der zweidimensionalen Darstellung als eine Trajektorie, falls der Kurvenverlauf hinsichtlich eines vorgegebenen Ähnlichkeitsmaßes mindestens eine vorgegebene Ähnlichkeit mit einem Vergleichs-Kurvenverlauf aufweist.

9. Computerprogramm gemäß Anspruch 8, bei dem das Identifizieren eines Kurvenverlaufs (1028, 1058; 1130, 1140; 1410, 1412, 1414, 1416) in der zweidimensionalen Darstellung (1220;1300;1400) als eine Trajektorie ein schrittweises Verzerren der zweidimensionalen Darstellung, um eine verzerrte zweidimensionale Darstellung des Aktivitätsmusters über der Zeit zu erhalten, sowie ein Bestimmen, ob in der verzerrten zweidimensionalen Darstellung eine näherungsweise gerade Linie enthalten ist, umfasst, wobei eine in der verzerrten zweidimensionalen Darstellung erkannte näherungsweise gerade Linie als eine Trajektorie identifiziert wird.

10. Computerprogramm gemäß Anspruch 9, bei dem schrittweise Verzerren derart erfolgt, dass eine in der zweidimensionalen Darstellung (1220;1300; 1400) enthaltene gekrümmte Linie durch das schrittweise Verzerren schrittweise gerade gebogen wird.

11. Computerprogramm gemäß einem der Ansprüche 8 bis 10, bei dem das Identifizieren eines Kurvenverlaufs (1028,1058;1130,1140;1410,1412,1414, 1416) in der zweidimensionalen Darstellung (1220; 1300;1400) als eine Trajektorie ein Anwenden einer Hough-Transformation, die für eine Erkennung von geraden oder gebogenen Kurvensegmenten konfiguriert ist, auf die zweidimensionale Darstellung umfasst, wobei eine Trajektorie basierend auf einem Ergebnis der Hough-Transformation erkannt wird.

12. Vorrichtung zum Erzeugen eines Ansteuersignals für ein Cochlea-Implantat basierend auf einem Audiosignal, mit folgenden Merkmalen:

einer Einrichtung zum Berechnen einer ersten Information ($A_1(t)$, $A_2(t)$, $A_n(t)$) über ein Aktivitätsmuster (1030,1032,1034,1036,1040; AP7, AP14) über der Zeit an einer Mehrzahl von inneren Haarzellen (IHZ1, IHZ2, IHZ3, IHZn) eines Gehörmodells, das sich aufgrund des Audiosignals ergibt;
einer Einrichtung zum Herausfiltern von durch die erste Information beschriebenen Aktivitätsereignissen (1030,1032,1034,1036,1040; AP7, AP14) basierend auf einer Erkennung eines charakteristischen Musters in dem Aktivitätsmuster, um aus der ersten Information eine bereinigte Information ($A_{1,ber}(t)$, $A_{2,ber}(t)$, $A_{n,ber}(t)$) durch Eliminieren von Aktivitätsereignissen, die zu einem charakteristischen Muster gehören, oder durch Eliminieren von Aktivitätsereignissen, die nicht zu einem charakteristischen Muster gehören, zu erhalten,

wobei die Erkennung eines charakteristischen Musters ein Erkennen eines linienförmigen Verlaufs in einer Darstellung des Aktivitätsmusters über der Zeit und über einen Index der inneren Haarzellen als charakteristisches Muster umfasst; und

einer Einrichtung zum Ansteuern des Cochlea-Implantats, die ausgelegt ist, um die bereinigte Information oder eine von der bereinigten Information abgeleitete Information als Ansteuersignal für das Cochlea-Implantat zu verwenden.

13. Vorrichtung gemäß Anspruch 12, wobei die Einrichtung zum Ansteuern des Cochlea-Implantats ausgelegt ist, um das Ansteuersignal an das Cochlea-Implantat zu übertragen.

14. Vorrichtung gemäß Anspruch 12, wobei die Einrichtung zum Ansteuern des Cochlea-Implantats ausgelegt ist, um Elektroden des Cochlea-Implantats mit dem Ansteuersignal zu beaufschlagen.

15. Vorrichtung gemäß einem der Ansprüche 12 bis 14, mit folgenden Merkmalen:

einer Einrichtung zum Bestimmen, welche der durch die erste Information ($A_1(t)$, $A_2(t)$, $A_n(t)$) beschriebenen Aktivitätsereignisse (1030, 1032,1034,1036,1040,1046; 472, AP1, AP2, AP3, AP4, AP5, AP6, AP7, AP8, AP9, AP10, AP11, AP12, AP13, AP14) nicht zu einer Trajektorie gehören, und

einer Einrichtung zum Ableiten der bereinigten Information ($A_{1,ber}(t)$, $A_{2,ber}(t)$, $A_{n,ber}(t)$) über das Aktivitätsmuster durch Eliminieren von Aktivitätsereignissen, die nicht zu einer Trajektorie gehören, auf der ersten Information.

16. Vorrichtung gemäß einem der Ansprüche 12 bis 15, bei der die erste Information zeitliche Verläufe (410,1010,1012,1014,1016) eine Anzahl an freigesetzten Neurotransmitter-Vesikeln für eine Mehrzahl von inneren Haarzellen (IHZ1, IHZ2, IHZ3, IHZn) beschreibt, wobei jeder inneren Haarzelle aus der Mehrzahl von inneren Haarzellen ein zeitlicher Verlauf zugeordnet ist.

17. Vorrichtung gemäß einem der Ansprüche 12 bis 15, bei der die erste Information ($A_1(t)$, $A_2(t)$, $A_n(t)$) eine Mehrzahl von zeitlichen Spannungsverläufen (1126) an einer Mehrzahl von Nervenfasern (NF1, NF2, NF3, NFn), die mit verschiedenen inneren Haarzellen (IHZ1, IHZ2, IHZ3, IHZn) gekoppelt sind, beschreibt, wobei jeder inneren Haarzelle aus der Mehrzahl von inneren Haarzellen ein zeitlicher Spannungsverlauf auf einer zugehörigen Nervenfaser zugeordnet ist.

18. Vorrichtung gemäß einem der Ansprüche 12 bis 17, bei der die Einrichtung zum Bestimmen, welche der durch die erste Information ($A_1(t)$, $A_2(t)$, $A_n(t)$) beschriebenen Aktivitätsereignisse nicht zu einer Trajektorie gehören, eine Kurvenerkennungseinrichtung (1500; 1700; 1800) umfasst, die ausgelegt ist, um das Aktivitätsmuster über der Zeit an der Mehrzahl von Haarzellen in Form von parallelen Signalen (1520,1522,1524;1720,1722,1724) parallel zu empfangen, wobei jedes Signal einer Haarzelle zugeordnet ist, und um die Signale unterschiedlich schnell durch eine Mehrzahl von hintereinander geschalteten Stufen (1510,1512,1514;1730,1732,1734) weiterzuleiten,

wobei mindestens eine vorbestimmte Stufe der Mehrzahl von hintereinandergeschalteten Stufen eine Schwellwerterkennungseinrichtung (1770,1772, 1774) aufweist, die ausgelegt ist, um zu erkennen, wenn mindestens eine vorgegebene Anzahl an Signalen in der vorbestimmten Stufe gleichzeitig aktiv sind,

wobei das Vorliegen von mindestens der vorgegeben Anzahl an gleichzeitig aktiven Signalen in der vorbestimmten Stufe ein Vorliegen einer Trajektorie (1028,1058;1130,1140;1410,1412,1414,1416) in dem Aktivitätsmuster anzeigt.

19. Vorrichtung gemäß Anspruch 18, bei der mindestens eine Stufe (1510,1512,1514;1730,1732,1734) ausgelegt ist, um mehrere Signale (1520,1522, 1524;1720,1722,1724) bei einer Weiterleitung durch die Stufe unterschiedlich stark zu verzögern.

20. Vorrichtung gemäß einem der Ansprüche 12 bis 19, bei der die Einrichtung zum Herausfiltern eine Einrichtung zur Durchführung einer parallelen Hough-Transformation umfasst, die ausgelegt ist, um das Vorliegen einer Trajektorie in dem Aktivitätsmuster anzuzeigen, wobei eine Trajektorie ein charakteristisches Muster in dem Aktivitätsmuster bildet.

**Claims**

1. Computer program for performing a method (100) of generating a control signal for a cochlear implant, based on an audio signal, when the computer program runs on a computer, the method (100) of generating a control signal comprising:

calculating (110) first information ($A_1(t)$, $A_2(t)$, $A_n(t)$) describing an activity pattern over time (t) at a plurality of inner hair cells (IHC1, IHC2, IHC3, IHCn) of an auditory model, which is yielded based on the audio signal (112);
filtering out (120) activity events (1030, 1032, 1034, 1036, 1040; AP7, AP14) described by the first information based on a recognition of a characteristic pattern in the activity pattern, for obtaining, from the first information, cleared information ($A_{1,cl}(t)$, $A_{2,cl}(t)$, $A_{n,cl}(t)$) by eliminating activity events pertaining to a characteristic pattern or by eliminating activity events not pertaining to a characteristic pattern,

wherein the recognition of a characteristic pattern includes recognizing a line-shaped curve in a representation of the activity pattern over time and over an index of the inner hair cells as a characteristic pattern; and
using the cleared information as a control signal for a cochlear implant, or deriving a control signal for a cochlear implant from the cleared information.

2. Computer program of claim 1, wherein the first information ($A_1(t)$, $A_2(t)$, $A_n(t)$) describes, via the activity pattern, over time, temporal devolutions (410) of a number of released neurotransmitter vesicles for a plurality of inner hair cells (IHC1, IHC2, IHC3, IHCn), and
wherein the filtering out (120) includes removing neurotransmitter vesicle occurrences (1030, 1032, 1034, 1036, 1040, 1046) not contributing to a generation of an action potential (472, AP1, AP2, AP3, AP4, AP5, AP6, AP7, AP8, AP9, AP10, AP11, AP12, AP13, AP14) or generating an action potential, from the first information.

3. Computer program of claim 2, wherein a removing of neurotransmitter vesicle occurrences (1030, 1032, 1034, 1036, 1040, 1046) includes setting to zero a number of released neurotransmitter vesicles in a certain time interval for a certain hair cell (IHC1, IHC2, IHC3, IHCn), when neurotransmitter vesicles released in the certain hair cell in the certain time interval do not contribute to a generation of an action potential on a nerve fiber (NF1, NF2, NF3, NF4, NF5, NF6, NFn) coupled to the certain hair cell or generate an action potential on the nerve fiber coupled to the certain hair cell.

4. Computer program of claims 2 or 3, wherein the filtering out (120) includes removing neurotransmitter vesicle occurrences (1030, 1032, 1034, 1036) for hair cells (IHC1, IHC2, IHC3, IHCn), which, due to a temporally previous release of an action potential (472, AP1, AP2, AP3, AP4, AP5, AP6, AP7, AP8, AP9, AP10, AP11, AP12, AP13, AP14) are in a refractory period.

5. Computer program of claim 1, wherein the filtering out (120) includes determining, which of the activity events (1020, 1022, 1024, 1026, 1030, 1032, 1034, 1036, 1040, 1046, 1050, 1052, 1054, 1056; AP1, AP2, AP3, AP4, AP5, AP6, AP7, AP8, AP9, AP10, AP11, AP12, AP13, AP14) described by the first information do not pertain to a trajectory (1028, 1058; 1130, 1140), and deriving the cleared information ($A_{1,cl}(t)$, $A_{2,cl}(t)$, $A_{n,cl}(t)$) on the activity pattern, wherein the cleared information is derived from the first information by eliminating activity events not pertaining to a trajectory.

6. Computer program of claim 1, wherein the filtering out (1020) includes determining, which of the activity events (1020, 1022, 1024, 1026, 1030, 1032, 1034, 1036, 1040, 1046, 1050, 1052, 1054, 1056; AP1, AP2, AP3, AP4, AP5, AP6, AP7, AP8, AP9, AP10, AP11, AP12, AP13, AP14) described by the first information pertain to a trajectory (1028, 1058; 1130; 1140), and deriving the cleared information ($A_{1,cl}(t)$, $A_{2,cl}(t)$, $A_{n,cl}(t)$) on the activity pattern, wherein the cleared information is derived from the first information by eliminating activity events pertaining to a trajectory.

7. Computer program of claims 5 or 6, wherein the first information ($A_1(t)$, $A_2(t)$, $A_n(t)$) describes temporal devolutions (410, 1010, 1012, 1014, 1016) of a number of released neurotransmitter vesicles for a plurality of inner hair cells (IHC1, IHC2, IHC3, IHCn), wherein each inner hair cell of the plurality of inner hair cells has allocated thereto a temporal devolution; or
wherein the first information ($A_1(t)$, $A_2(t)$, $A_n(t)$) describes a plurality of temporal voltage shapes (1126) at a plurality of nerve fibers ((NF1, NF2, NF3, NFn) coupled to different inner hair cells (IHC1, IHC2, IHC3, IHCn), wherein each inner hair cell of the plurality of inner hair cells has allocated thereto a temporal voltage shape on an associated nerve fiber.

8. Computer program of one of claims 5 to 7, wherein the determining, which of the activity events (1030, 1032, 1034, 1036, 1040; AP7, AP14) described by the first information ($A_1(t)$, $A_2(t)$, $A_n(t)$) pertain to a trajectory (1028, 1058; 1130, 1140) or do not pertain to a trajectory, includes performing a pattern recognition, wherein the performing of the pattern recognition comprises:

receiving the first information on the activity pattern over time;
interpreting the activity pattern over time for a plurality of hair cells as a two-dimensional representation (1022, 1300, 1400), wherein a first direction (x) of the two-dimensional representation describes the time (t), wherein a second direction (y) of the two-dimensional representation describes an index (i) of the hair cells, and wherein the information contained in the two-dimensional representation describes an occurrence of activity events as a function of the time and of the index of the inner hair cells; and

identifying a curve shape in the two-dimensional representation as a trajectory, if the curve shape exhibits, with respect to a given measure of similarity, at least one given similarity to a comparative curve shape.

9. Computer program of claim 8, wherein the identifying of a curve shape (1028, 1058; 1130, 1140; 1410, 1412, 1414, 1416) in the two-dimensional representation (1220; 1300; 1400) as a trajectory includes a step-by-step distorting of the two-dimensional representation so as to obtain a distorted two-dimensional representation of the activity pattern over time, as well as determining if an approximately straight line is contained in the distorted two-dimensional representation, wherein an approximately straight line recognized in the distorted two-dimensional representation is identified as a trajectory.

10. Computer program of claim 9, wherein the step-by-step distorting takes place such that a curved line contained in the two-dimensional representation (1220; 1300; 1400) is straightened out in a step-by-step manner by the step-by-step distorting.

11. Computer program of one of claims 8 to 10, wherein the identifying of a curve shape (1028, 1058; 1130, 1140; 1410, 1412, 1414, 1416) in the two-dimensional representation (1220; 1300; 1400) as a trajectory includes applying a Hough transform, which is configured for a recognition of straight or curved graph segments, to the two-dimensional representation, wherein a trajectory is recognized based on a result of the Hough transform.

12. Device for generating a control signal for a cochlear implant based on an audio signal, comprising:

means for calculating a first information ($A_1(t)$, $A_2(t)$, $A_n(t)$) on an activity pattern (1030, 1032, 1034, 1036, 1040; AP7, AP14) over time at a plurality of inner hair cells (IHC1, IHC2, IHC3, IHCn) of an auditory model, which is yielded due to the audio signal;
means for filtering out activity events (1030, 1032, 1034, 1036, 1040; AP7, AP14) described by the first information based on a recognition of a characteristic pattern in the activity pattern in order to obtain, from the first information, cleared information ($A_{1,cl}(t)$, $A_{2,cl}(t)$, $A_{n,cl}(t)$), by eliminating activity events pertaining to a characteristic pattern, or by eliminating activity events not pertaining to a characteristic pattern,

wherein the recognition of a characteristic pattern includes recognizing a line-shaped curve in a representation of the activity pattern over time and over an index of the inner hair cells as a characteristic pattern; and
means for controlling the cochlear implant, which is configured to use the cleared information or information derived from the cleared information as a control signal for the cochlear implant.

13. Device of claim 12, wherein the means for controlling the cochlear implant is configured to transmit the control signal to the cochlear implant.

14. Device of claim 12, wherein the means for controlling the cochlear implant is configured to impart electrodes of the cochlear implant with the control signal.

15. Device of one of claims 12 to 14, comprising:

means for determining, which of the activity events (1030, 1032, 1034, 1036, 1040, 1046; 472, AP1, AP2, AP3, AP4, AP5, AP6, AP7, AP8, AP9, AP10, AP11, AP12, AP13, AP14) described by the first information ($A_1(t)$, $A_2(t)$, $A_n(t)$), do not pertain to a trajectory, and
means for deriving the cleared information ($A_{1,cl}(t)$, $A_{2,cl}(t)$, $A_{n,cl}(t)$) on the activity pattern by eliminating activity events not pertaining to a trajectory, on the first information.

16. Device of one of claims 12 to 15, wherein the first information describes temporal devolutions (410, 1010, 1012, 1014, 1016) of a number of released neurotransmitter vesicles for a plurality of inner hair cells (IHC1, IHC2, IHC3, IHCn), wherein each inner hair cell of the plurality of inner hair cells has allocated thereto a temporal devolution.

17. Device of one of claims 12 to 15, wherein the first information ($A_1(t)$, $A_2(t)$, $A_n(t)$) describes a plurality of temporal voltage shapes (1126) at a plurality of nerve fibers ((NF1, NF2, NF3, NFn) coupled to different inner hair cells (IHC1, IHC2, IHC3, IHCn), wherein each inner hair cell of the plurality of inner hair cells has allocated thereto a temporal voltage shape on an associated nerve fiber.

18. Device of one of claims 12 to 17, wherein the means for determining, which of the activity events described by the first information ($A_1(t)$, $A_2(t)$, $A_n(t)$) do not pertain to a trajectory, include curve recognition means (1500; 1700; 1800) configured to receive the activity pattern over time at the plurality of hair cells in the form of parallel signals (1520, 1522, 1524; 1720, 1722, 1724) in a parallel manner, wherein each signal is allocated to a hair cell, and to forward the signals through a plurality of stages (1510, 1512, 1514; 1730, 1732, 1734) connected one after the other at different speeds, wherein at least one predetermined stage of the plurality of stages connected one after the other comprises threshold-value recognition means (1770, 1772, 1774) configured to recognize, when at least a given number of signals are simultaneously active in the predetermined stage,

wherein the presence of at least the given number of simultaneously active signals in the predetermined stage indicates a presence of a trajectory (1028, 1058; 1130, 1140; 1410, 1412, 1414, 1416) in the activity pattern.

**19.** Device of claim 18, wherein at least one stage (1510, 1512, 1514; 1730, 1732, 1734) is configured to delay multiple signals (1520, 1522, 1524; 1720, 1722, 1724) more or less in a forwarding through the stage.

**20.** Device of one of claims 12 to 19, wherein the means for filtering out includes means for performing a parallel Hough transform, which is configured to indicate the presence of a trajectory in the activity pattern, wherein a trajectory forms a characteristic pattern in the activity pattern.

**Revendications**

**1.** Programme d'ordinateur pour réaliser un procédé (100) pour générer un signal d'activation d'un implant cochléaire, sur base d'un signal audio, lorsque le programme d'ordinateur est exécuté sur un ordinateur,

le procédé (100) pour générer le signal d'activation présentant les étapes suivantes consistant à:

calculer (110) une première information ($A_1(t)$, $A_2(t)$, $A_n(t)$) qui décrit un modèle d'activité dans le temps (t) sur une pluralité de cellules sensorielles internes (IHZ1, IHZ2, IHZ3, IHZn) d'un modèle d'ouïe qui est obtenu sur base du signal audio (112);

éliminer par filtration (120) des événements d'activité (1030, 1032, 1034, 1036, 1040; AP7, AP14) décrits par la première information sur base d'une identification d'un modèle caractéristique dans le modèle d'activité, pour obtenir à partir de la première information une information nettoyée ($A_{1,ber}(t)$, $A_{2,ber}(t)$, $A_{n,ber}(t)$) par élimination des événements d'activité appartenant à un modèle caractéristique, ou par élimination des événements d'activité n'appartenant pas à un modèle caractéristique,

l'identification d'un modèle caractéristique comprenant une identification d'une évolution linéaire dans une représentation du modèle d'activité dans le temps et sur un indice des cellules sensorielles internes comme modèle caractéristique; et

utiliser l'information nettoyée comme signal d'activation de l'implant cochléaire, ou dériver un signal d'activation d'un implant cochléaire de l'information nettoyée.

**2.** Programme d'ordinateur selon la revendication 1, dans lequel la première information ($A_1(t)$, $A_2(t)$, $A_n(t)$) sur le modèle d'activité dans le temps décrit des évolutions dans le temps (410) d'un nombre de vésicules de neurotransmission dégagées pour une pluralité de cellules sensorielles internes (IHZ1, IHZ2, IHZ3, IHZn), et

dans lequel l'élimination par filtration (120) comprend une élimination de la première information de production de vésicules de neurotransmission (1030, 1032, 1034, 1036, 1040, 1046) qui ne contribuent pas à une génération d'un potentiel d'action (472, AP1, AP2, AP3, AP4, AP5, AP6, AP7, AP8, AP9, AP10, AP11, AP12, AP 13, AP 14) ou ne génèrent pas de potentiel d'action.

**3.** Programme d'ordinateur selon la revendication 2, dans lequel une élimination de production de vésicules de neurotransmission (1030, 1032, 1034, 1036, 1040, 1046) comprend une remise à zéro d'un nombre de vésicules de neurotransmission dégagées dans un intervalle de temps déterminé pour une cellule sensorielle déterminée (IHZ1, IHZ2, IHZ3, IHZn) lorsque, dans l'intervalle de temps déterminé, les vésicules de neurotransmission dégagées dans la cellule sensorielle déterminée ne contribuent pas à une génération d'un potentiel d'action sur une fibre nerveuse (NF1, NP2, NF3, NF4, NFS, NF6, NFn) couplée à la cellule sensorielle déterminée ou ne génèrent pas de potentiel d'action sur la fibre nerveuse couplée à la cellule sensorielle déterminée.

**4.** Programme d'ordinateur selon la revendication 2 ou 3, dans lequel l'élimination par filtration (120) comprend une élimination de productions de vésicules de neurotransmission (1030, 1032, 1034, 1036) pour les cellules sensorielles (IHZ1, IHZ2, IHZ3, IHZn) qui se trouvent, par suite d'un dégagement retardé dans le temps d'un potentiel d'action (472, AP1, AP2, AP3, AP4, AP5, AP6, AP7, AP8, AP9, AP10, AP 11, AP12, AP13, AP14), dans un espace de temps réfractaire.

**5.** Programme d'ordinateur selon la revendication 1, dans lequel l'élimination par filtration (120) comprend une détermination des événements d'activité (1020, 1022, 1024, 1026, 1030, 1032, 1034, 1036, 1040, 1046, 1050, 1052, 1054, 1056; AP1, AP2, AP3, AP4, Ap5, Ap6, AP67, AP8, AP9, AP10, AP 11, AP12, AP 13, AP 14) décrits par la première information qui n'appartiennent pas à une trajectoire (1028, 1058; 1130, 1140) et une dérivation de l'information nettoyée ($A_{1,ber}(t)$, $A_{2,ber}(t)$, $A_{n,ber}(t)$) sur le modèle d'activité, l'information nettoyée étant dérivée de la première information par élimination d'événements d'activité n'appartenant pas à une trajectoire.

6. Programme d'ordinateur selon la revendication 1, dans lequel l'élimination par filtration (120) comprend une détermination des événements d'activité (1020, 1022, 1024, 1026, 1030, 1032, 1034, 1036, 1040, 1046, 1050, 1052, 1054, 1056; AP1, AP2, AP3, AP4, Ap5, Ap6, AP67, AP8, AP9, AP10, AP 11, AP12, AP13, AP14) décrits par la première information qui appartiennent à une trajectoire (1028, 1058; 1130, 1140), et une dérivation de l'information nettoyée ($A_{1,ber}(t)$, $A_{2,ber}(t)$, $A_{n,ber}(t)$) sur le modèle d'activité, l'information nettoyée étant dérivée de la première information par élimination d'événements d'activité appartenant à une trajectoire.

7. Programme d'ordinateur selon la revendication 5 ou 6, dans lequel la première information ($A_1(t)$, $A_2(t)$, $A_n(t)$) décrit des évolutions dans le temps (410, 1010, 1012, 1014, 1016) d'un nombre de vésicules de neurotransmission dégagées pour une pluralité de cellules sensorielles internes (IHZ1, IHZ2, IHZ3, IHZn), à chaque cellule sensorielle interne parmi la pluralité de cellules sensorielles internes étant associée une évolution dans le temps; ou dans lequel la première information ($A_1(t)$, $A_2(t)$, $A_n(t)$) décrit une pluralité d'évolutions de tension dans le temps (1126) à une pluralité de fibres nerveuses (NF1, NF2, NF3, NFn) qui sont couplées à différentes cellules sensorielles internes (IHZI, IHZ2, IHZ3, IHZn), à chaque cellule sensorielle interne parmi la pluralité de cellules sensorielles internes étant associée une évolution de tension dans le temps sur une fibre nerveuse associée.

8. Programme d'ordinateur selon l'une des revendications 5 à 7, dans lequel la détermination des événements d'activité (1030, 1032, 1034, 1036, 1040; AP7, AP14) décrits par la première information ($A_1(t)$, $A_2(t)$, $A_n(t)$) qui appartiennent à une trajectoire (1028, 1058; 1130, 1140) ou n'appartiennent pas à une trajectoire comprend la réalisation d'une identification de modèle, la réalisation de l'identification de modèle présentant les étapes suivantes consistant à:

recevoir la première information sur le modèle d'activité dans le temps; interpréter le modèle d'activité dans le temps pour une pluralité de cellules sensorielles comme une représentation bidimensionnelle (1022, 1300, 1400), une première direction (x) de la représentation bidimensionnelle décrivant le temps (t), une deuxième direction (y) de la représentation bidimensionnelle décrivant un indice (i) des cellules sensorielles, et l'information contenue dans la représentation bidimensionnelle décrivant une production d'événements d'activité en fonction du temps et de l'indice des cellules sensorielles internes; et identifier une évolution de courbe dans la représentation bidimensionnelle comme trajectoire lorsque l'évolution de courbe présente, en ce qui concerne une mesure de similitude prédéterminée, au moins une similitude prédéterminée avec une évolution de courbe de comparaison.

9. Programme d'ordinateur selon la revendication 8, dans lequel l'identification d'une évolution de courbe (1028, 1058; 1130, 1140; 1410, 1412, 1414, 1416) dans la représentation bidimensionnelle (1220; 1300; 1400) comme trajectoire comprend une distorsion pas à pas de la représentation bidimensionnelle, pour obtenir une représentation bidimensionnelle distorsionnée du modèle d'activité dans le temps, ainsi qu'une détermination de s'il est contenu, dans la représentation bidimensionnelle distorsionnée, une ligne environ droite, une ligne environ droite identifiée dans la représentation bidimensionnelle distorsionnée étant identifiée comme trajectoire.

10. Programme d'ordinateur selon la revendication 9, dans lequel la distorsion pas à pas s'effectue de sorte qu'une ligne courbe contenue dans la représentation bidimensionnelle (1220; 1300; 1400) soit redressée pas à pas par la distorsion pas à pas.

11. Programme d'ordinateur selon l'une des revendications 8 à 10, dans lequel l'identification d'une évolution de courbe (1028, 1058; 1130, 1140; 1410, 1412, 1414, 1416) dans la représentation bidimensionnelle (1220; 1300; 1400) comme trajectoire comprend une application d'une transformation de Hough qui est configurée pour une identification de segments de courbe droits ou courbés dans la représentation bidimensionnelle, une trajectoire étant identifiée sur base d'un résultat de la transformation de Hough.

12. Dispositif pour générer un signal d'activation d'un implant cochléaire sur base d'un signal audio, aux caractéristiques suivantes:

un moyen destiné à calculer une première information ($A_1(t)$, $A_2(t)$, $A_n(t)$) sur un modèle d'activité (1030, 1032, 1034, 1036, 1040; AP7, AP14) dans le temps sur une pluralité de cellules sensorielles internes (IHZ1, IHZ2, IHZ3, IHZn) d'un modèle d'ouïe qui est obtenu sur base du signal audio; un moyen destiné à éliminer par filtration des événements d'activité (1030, 1032, 1034, 1036, 1040; AP7, AP14) décrits par la première information sur base d'une identification d'un modèle caractéristique dans le modèle d'activité, pour obtenir à partir de la première information une information nettoyée ($A_{1,ber}(t)$, $A_{2,ber}(t)$, $A_{n,ber}(t)$) par élimination des événements d'activité appartenant à un modèle caractéristique, ou par élimination de événements d'activité n'appartenant pas à un modèle caractéristique,

l'identification d'un modèle caractéristique comprenant une identification d'une évolution linéaire dans une représentation du modèle d'activité dans le temps et sur un indice des cellules sensorielles internes comme modèle caractéristique; et

un moyen destiné à activer l'implant cochléaire qui est conçu de manière à utiliser l'information nettoyée ou un signal d'activation dérivé de l'information nettoyée comme signal d'activation de l'implant cochléaire.

13. Dispositif selon la revendication 12, dans lequel le moyen destiné à activer l'implant cochléaire est conçu de manière à transmettre le signal d'activation à l'implant cochléaire.

14. Dispositif selon la revendication 12, dans lequel le moyen destiné à activer l'implant cochléaire est conçu de manière à soumettre des électrodes de l'implant cochléaire au signal d'activation.

15. Dispositif selon l'une des revendications 12 à 14, aux caractéristiques suivantes:

un moyen destiné à déterminer les événements d'activité (1030, 1032, 1034, 1036, 1040, 1046; 472, AP1, AP2, AP3, AP4, AP5, AP6, AP7, AP8, AP9, AP10, AP 11, AP12, AP13, AP14) décrits par la première information ($A_1(t)$, $A_2(t)$, $A_n(t)$) qui n'appartiennent pas à une trajectoire, et

un moyen destiné à dériver l'information nettoyée ($A_{1,ber}(t)$, $A_{2,ber}(t)$, $A_{n,ber}(t)$) sur le modèle d'activité par élimination des événements d'activité qui n'appartiennent pas à une trajectoire sur la première information.

16. Dispositif selon l'une des revendications 12 à 15, dans lequel la première information décrit des évolutions dans le temps (410, 1010, 1012, 1014, 1016) d'un nombre de vésicules de neurotransmission dégagées pour une pluralité de cellules sensorielles internes (IHZ1, IHZ2, IHZ3, IHZn), à chaque cellule sensorielle interne parmi la pluralité de cellules sensorielles internes étant associée une évolution dans le temps.

17. Dispositif selon l'une des revendications 12 à 15, dans lequel la première information ($A_1(t)$, $A_2(t)$, $A_n(t)$) décrit une pluralité d'évolutions de tension dans le temps (1126) à une pluralité de fibres nerveuses (NF1, NF2, NF3, NFn) qui sont couplées à différentes cellules sensorielles internes (IHZ1, IHZ2, IHZ3, IHZn), à chaque cellule sensorielle interne parmi la pluralité de cellules sensorielles internes étant associée une évolution de tension dans le temps sur une fibre nerveuse associée.

18. Dispositif selon l'une des revendications 12 à 17, dans lequel le moyen destiné à déterminer les événements d'activité décrits par la première information ($A_1(t)$, $A_2(t)$, $A_n(t)$) qui n'appartiennent pas à une trajectoire comprend un moyen d'identification de courbe (1500; 1700; 1800) qui est conçu de manière à recevoir en parallèle le modèle d'activité dans le temps à la pluralité de cellules sensorielles sous forme de signaux parallèles (1520, 1522, 1524; 1720, 1722, 1724), chaque signal étant associé à une cellule sensorielle, et à transmettre les signaux à une vitesse différente par une pluralité d'étages (1510, 1512, 1514; 1730, 1732, 1734) connectés l'un derrière l'autre,

au moins un étage prédéterminé parmi la pluralité d'étages connectés l'un derrière l'autre présentant un moyen d'identification de valeur de seuil (1770, 1772, 1774) qui est conçu de manière à identifier le moment où au moins un nombre prédéterminé de signaux sont simultanément actifs dans l'étage prédéterminé,

la présence d'au moins le nombre prédéterminé de signaux simultanément actifs dans l'étage prédéterminé indiquant une présence d'une trajectoire (1028, 1058; 1130, 1140; 1410, 1412, 1414, 1416) dans le modèle d'activité.

19. Dispositif selon la revendication 18, dans lequel au moins un étage (1510, 1512, 1514; 1730, 1732, 1734) est conçu de manière à retarder plusieurs signaux (1520, 1522, 1524; 1720, 1722, 1724) d'un degré différent lors d'une transmission par l'étage.

20. Dispositif selon l'une des revendications 12 à 19, dans lequel le moyen destiné à éliminer par filtration comprend un moyen pour réaliser une transformation de Hough parallèle qui est conçu de manière à indiquer la présence d'une trajectoire dans le modèle d'activité, une trajectoire constituant un modèle caractéristique dans le modèle d'activité.

## FIG 1

$\nearrow$100

Audiosignal

$\nearrow$112

$\nearrow$110

Berechnen einer Information über ein Aktivitätsmuster über der Zeit an einer Mehrzahl von inneren Hörzellen eines Gehörmodells aufgrund des Audiosignals

IHZ1                    IHZ2                    ... IHZn

$A_1(t)$                    $A_2(t)$                    $A_n(t)$

$\nearrow$120

Herausfiltern von Aktivitätsereignissen basierend auf einer Erkennung eines charakteristischen Musters in dem Aktivitätsmuster, um eine bereinigte Information zu erhalten

$A_{1,be}(t)$                    $A_{2,be}(t)$                    $A_{n,be}(t)$

Verwendung der bereinigten Information als Ansteuersignal für ein Cochlea-Implantat oder Ableiten des Ansteuersignals von der bereinigten Information

# FIG 2

200

Audiosignal —————————— 210

↓

| Mechanisch Schallwandlung im Außen-Ohr | ∼ 214

↓

Anregung des Trommelfells ———————— 218

↓

| Schallübertragung über Gehörknöchelchen | ∼ 222

↓

Anregung des oralen Fensters ——————— 226
zwischen Mittelohr und Cochlea

↓

| Berechnung der hydromechanischen Schwingungsanregung der Cochlea | ∼ 230

↓

Schwingungsanregung der Cochlea —————— 234

↓

| Berechnung der Basilarmembran-Bewegung | ∼ 238

↓

Basilarmembran-Bewegung ——————— 242

↓

| Berechnung einer Auslenkung eines Stereoziliums | ∼ 246

↓

Auslenkung eines Stereoziliums ——————— 252

↓

| Berechnung einer Calcium-Konzentration | ∼ 256

↓

Calcium-Konzentration ———————— 260

↓

| Berechnung einer Transmitter-Freisetzungs-Rate | ∼ 264

↓

Transmitter-Freisetzungs-Rate ——————— 268

↓

| Bestimmung eines Neurotransmitter-Versikel-Auftretens | ∼ 272

↓

Neurotransmitter-Vesikel-Auftreten ————— 276

↓

| Bestimmung eines Nervenaktivitätsmusters durch Bestimmung von den zu den Neurotransmitter-Versikel-Auftreten gehörigen Aktionspotentialen | ∼ 280

↓

Nervenaktivitätsmusters ——————— 284

# FIG 3

300

seperat für eine Mehrzahl von Inneren Haarzellen:

Information über Neurotransmitter-Vesikel-Auftreten

310

Bestimmen, welche Neurotransmitter-Vesikel-Auftreten, die ein Aktionspotential generieren

320

Weiterleiten lediglich der Neurotransmitter-Vesikel-Auftreten,
die ein Aktionspotential generieren

330

erstes bereinigtes Neurotransmitter-Vesikel-Auftreten

340

optional: Übertragen des ersten bereinigten Neurotransmitter-Vesikel-Auftretens
zu dem Cochlea-Implantat

# FIG 4

410

430

450

Neurotransmitter-
Vesikel-Freisetzung
(im Zeitintervall) 414

Potential 434

Aktionspotential 454

470

$t_1$

$t_2$

472

$t_3$

460 absolute
Refraktionszeit

$t_4$

462

452

Neurotransmitter-
Vesikel, die nicht

Ruhepotential

510 relative
Refraktionszeit

412

432

t

t

t

1. Möglichkeit für ein bereinigte
Neurotransmitter-
Vesikel-Freisetzung

2. Möglichkeit für ein bereinigte
Neurotransmitter-
Vesikel-Freisetzung

$t_1$

$t_2$

$t_1$

$t_2$

$t_3$

$t_4$

480

490

# FIG 5

500

erstes bereinigtes Neurotransmitter-Vesikel-Auftreten

IHZ1          IHZn

510

| Bestimmen, welche Neurotransmitter-Vesikel-Auftreten nicht zu einer Trajektorie gehören |
| --- |

520

| Eliminieren von Neurotransmitter-Vesikel-Auftreten, die nicht zu einer Trajektorie gehören |
| --- |

530

zweites bereinigtes Neurotransmitter-Vesikel-Auftreten

540

| optional: Übertragen des zweiten bereinigten Neurotransmitter-Vesiklel-Auftretens zu dem Cochlea-Implantat |
| --- |

FIG 6

# FIG 7

## BERECHNUNG DER VERZÖGERUNG ALS FUNKTION DER FREQUENZ

$d_a = (1000/f_i) + 2 \text{(ms)}$

DURCH EIN SINUSSIGNAL HERVOR-
GERUFENE LATENZZEIT DES HÖRNERVS
• Chinchilla (Ruggero &Rich, 1987)
—Cat (Goldstein et al, 1971)

LATENZZEIT DER AUDITORISCHEN NERVENFASER (ms)

CHARAKTERISTISCHE FREQUENZEN (kHz)

BASILAR MEMBRAN

ABSTAND VON DER BASIS IN mm (SKAL: ZU EINER MENSCHLICHEN COCHLEA)

LATENZZEIT EINER ANREGUNG EINER EINZELNEN FASER DES HÖRNERVS
ALS FUNKTION DER SINUSFÖRMIGEN SIGNALFREQUENZ

# FIG 8

COCHLEAGRAM DES VOKALS i

DETAILS DES COCHLEAGRAMS ZEIGen DIE FEINEN ZEITLICHE UND RÄUMLICHE STRUKTUR
IN DER MODELLIERTEN ANTWORT AUF EINEN VOKAL /i/ (LINKS) UND AUF EINEN
NICHT-HARMONISCHEN TONKOMPLEX VON 700Hz, 900Hz UND 1100Hz (RECHTS)

EP 1 981 582 B1

# FIG 9

VOKAL "A":

CHOCHLEAGRAM

← 910

FREISETZUNGS-
WAHRSCHEINLICHKEIT

← 920

VESIKEL-FREISETZUNG

← 930

EP 1 981 582 B1

# FIG 10a

Anzahl an Neurotransmitter-Vesikel

IHZ1 1010 1012 1014 1016

1000

1020 Trajektorie 1
1022
1024
1026

1030
1032
1034
1036

$t_1$

$t_2$ trägt nicht zur Erzeugung eines Aktionspotentials bei

1028

gehört nicht zu einer Trajektorie

1040 Trajektorie 2

1046

1050
1058
1052
1054
1056

t

# FIG 10b

1060

IHZ1 1070 IHZ2 1072 IHZ3 1074 IHZ4 1076

Trajektorie 1
$t_1$
1020′
1022′
1024′
1026′

Trajektorie 2

1050′
1052′
1054′
1056′

t

# FIG 11

1100

Audiosignal

Berechnen eines Nerven-Aktivitatsmusters — 1110

Cochlea (abgewickelt)

1120

1126

1130

1140

IHZ1  IHZ2  IHZn

AP14  AP11  AP13
AP10  AP12
AP8  AP9  AP7
AP3 AP4  AP6
AP1  AP2  AP5

NF1  NF2  NFn

Bestimmen, welche der Aktionspotentiale
nicht zu einer Trajektiorie gehören

AP7, AP14

Eliminieren der Aktionspotentiale, die nicht zu einer Trajektiorie gehören

NF3  NF4  NF5  NF6  AP13  1140
AP10  AP11  AP12  1130
AP8  NF2  AP9  AP6  1170
AP1  AP2  AP3  AP4  AP5

bereinigtes Nervenaktivitätsmuster

# FIG 12

/1200

FIG 13

1300

## FIG 14

innerhalb
Refraktarzeit,

1420

1424

nicht innerhalb der
Refraktarzeit, aber nicht
einer Trajektorie gehörig

X
(Zeit)

# FIG 15

1500

VON NERVENZELLE 1     VON NERVENZELLE 2     VON NERVENZELLE n

1530    1520         1534     1522         1536     1524

1510    | VERZÖGERUNG 1,1 |    | VERZÖGERUNG 1,2 |    | VERZÖGERUNG 1,n |    $\Sigma_1$

1532

1512    | VERZÖGERUNG 2,1 |    | VERZÖGERUNG 2,2 |    | VERZÖGERUNG 2,n |    $\Sigma_2$

1514    | VERZÖGERUNG i,1 |    | VERZÖGERUNG i,2 |    | VERZÖGERUNG i,n |    $\Sigma_i$

# FIG 16

1600

EINGÄNGE
VON STUFE 1

VON NERVEN    VON       VON       VON          1614
ZELLE 1       NZ 2      NZ 3      NZ 4

1610

1612

Σ = 1

1612

1612

1612

1612

NACH STUFE 1
(AUSGÄNGE DER
1. STUFE
= EINGÄNGE DER
2. STUFE

VON       VON       VON       VON
NZ 1      NZ 2      NZ 3      NZ 4

1624      1620

1622

Σ = 1

1622      1622      1622

1622

NACH STUFE 2
(AUSGÄNGE DER
2. STUFE
= EINGÄNGE DER
3. STUFE

VON       VON       VON       VON
NZ 1      NZ 2      NZ 3      NZ 4

1630

1634  Σ = 4

TRAJEKTORIE
GERADE-
GEBOGEN

1632      1632      1632      1632

NACH STUFE 3

VON       VON       VON       VON
NZ 1      NZ 2      NZ 3      NZ 4

1640

1644

Σ = 1

1642      1642      1642

1642

# FIG 17

1700

## DAS SCHALTBILD DES HUBBEL-WIESEL NETZES

SCHWELLWERT-
REGISTER/
KOMPARATOREN

VERZÖGERUNGSELEMENTE/ADDIERER

# FIG 18

DAS HUBEL-WIESEL NETZ

1800

INPUT NEUONS <1:9>

DELAY CHAINS <1:9>

1810

1820    1820

1810

1830    1830    1830    OUTPUT NEURONS <1:9>

# FIG 19

## DIE TRAININGSMUSTER

NEUN SINUSSE UNTERSCHIEDLICHER FREQUENZ

## FIG 20

2000

2010

Zu Nervenfaser
eines Hörnervs 2022

2020

Zu Nervenfaser 2024
eines Hörnervs

Zu Nervenfaser
eines Hörnervs

2026

Information
über ein
Neurotransmitter-
Vesikel-
Auftreten

2030

# FIG 21

2100

2110

2120

2140

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0199470 A1
- US 5381512 A
- US 5388182 A
- US 4980918 A
- US 20005069162 A1
- US 3069654 A


**In der Beschreibung aufgeführte Nicht-Patentliteratur**

- Neuronale Repräsentation des Hörvorgangs als Basis. **von G. Szepannek ; F. Klefenz ; C. Weihs.** Informatik-Spektrum. Springer-Verlag, Oktober 2005, vol. 28, 389-395
- **von F. Baumgarte.** Ein psychophysiologisches Gehörmodell zur Nachbildung von Wahrnehmungsschwellen für die Audiocodierung. Doktorarbeit an der Universität Hannover, 2000
- Computing with Spiking Neurons. **von W. Mass.** Pulsed Neuro Networks. Cambridge. MIT Press, 1998, 397-404
- **von G. Szepannek ; F. Klefenz ; C. Weihs.** *Schallanalyse: Neuronale Repräsentation des Hörvorgangs als Basis*